(19) Europäisches Patentamt
European Patent Office
Office européen des brevets

(11) **EP 4 520 260 A1**

(12) **EUROPÄISCHE PATENTANMELDUNG**

(43) Veröffentlichungstag:
**12.03.2025   Patentblatt 2025/11**

(21) Anmeldenummer: **23196265.5**

(22) Anmeldetag: **08.09.2023**

(51) Internationale Patentklassifikation (IPC):
*A61B 5/022* (2006.01)     *A61B 5/08* (2006.01)
*A61B 5/113* (2006.01)     *A61B 5/00* (2006.01)
*A61B 5/33* (2021.01)

(52) Gemeinsame Patentklassifikation (CPC):
**A61B 5/02233; A61B 5/02208; A61B 5/0816;**
**A61B 5/113; A61B 5/721;** A61B 5/33

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**KH MA MD TN**

(71) Anmelder: **Redtel, Holger**
**19348 Perleberg (DE)**

(72) Erfinder: **Redtel, Holger**
**19348 Perleberg (DE)**

(74) Vertreter: **Raffay & Fleck**
**Patentanwälte**
**Stephansplatz 2-6**
**20354 Hamburg (DE)**

(54) **BLUTDRUCKMESSVERFAHREN**

(57)     Die Erfindung betrifft ein Verfahren zur Ermittlung atmungskorrigierten Blutdruckwerten sowie eine Vorrichtung mit einer Steuereinheit zum Ausführen der Verfahren. Dabei werden in einer einzigen Messung mindestens zwei Blutdruckwerte zur Verfügung gestellt, auf Basis derer der Einfluss der Atmung und somit die Atmungskorrektur bestimmt wird. Ferner ist es mit dem Verfahren auch möglich kontinuierliche Blutdruckwerte aus Einzelwertmessungen zu bestimmen, selbst bei einer Verwendung einer schmalen Manschette.

Figur 3

**EP 4 520 260 A1**

**Beschreibung**

Die Erfindung betrifft Verfahren zur Ermittlung von Blutdruckwerten sowie eine Vorrichtung mit einer Steuereinheit zum Ausführen der Verfahren.

Der Blutdruck und der zeitliche Verlauf des Blutdrucks ist ein wichtiger Parameter, insbesondere hinsichtlich des medizinischen Zustandes einer Person. Aufgrund des Verlaufs der Blutdruckwerte und aus dem Verlauf der Blutdruckwerte als Funktion der Zeit können bestimmte Parameter errechnet bzw. abgeleitet werden. Hierbei wird die zeitliche Variation des Blutdrucks während mehrerer Herzzyklen erfasst, um hieraus bestimmte Kennwerte abzuleiten, wie beispielsweise den systolischen Blutdruckwert und den diastolischen Blutdruckwert. Hierbei ist es wichtig, die Erfassung des Wertes des Blutdrucks durch eine Blutdruckmessung möglichst akkurat und präzise zu gestalten.

Die Blutdruckmessung ist eine grundlegende Untersuchungsmethode in der Medizin und spielt eine entscheidende Rolle bei der Diagnose und Überwachung von Bluthochdruck, auch als Hypertonie bekannt. Die regelmäßige Überwachung des Blutdrucks ist von großer Bedeutung für die Aufrechterhaltung der körperlichen Gesundheit. Ein hoher Blutdruck kann auf ernsthafte gesundheitliche Probleme wie Herz-Kreislauf-Erkrankungen, Schlaganfälle und Nierenversagen hinweisen. Daher ist es wichtig, den Blutdruck regelmäßig zu überwachen und bei Bedarf geeignete Maßnahmen zu ergreifen. Die Blutdruckmessung kann auch dazu beitragen, verschiedene Krankheiten zu erkennen und zu behandeln. Dazu gehören beispielsweise Bluthochdruck, Diabetes, Nieren- oder Schilddrüsenerkrankungen.

Im Stand der Technik sind verschiedene Methoden zur Messung des Blutdrucks beschrieben. Die am Häufigsten verwendete Methode ist die nicht-invasive Methode nach Riva Rocci, die auf der Auswertung von Druckmessungen am Arm basiert. Diese Methode gilt heute als Goldstandard und wird aufgrund ihrer Genauigkeit und Zuverlässigkeit häufig in klinischen und häuslichen Umgebungen eingesetzt.

Die Methode nach Riva Rocci ist eine nicht-invasive Methode zur Messung des Blutdrucks. Sie wurde erstmals im Jahr 1896 von dem italienischen Arzt Scipione Riva Rocci entwickelt. Diese Methode verwendet eine aufblasbare Manschette, die um den Oberarm gelegt wird, um den Blutfluss in der Arterie zu blockieren, und ein Stethoskop, um die Geräusche zu hören, die entstehen, wenn die Manschette langsam entleert wird. Die Messwerte werden dann anhand eines Manometers abgelesen.

Im Laufe der Zeit wurden aus der Riva Rocci Methode weitere Methoden zur Messung des Blutdrucks entwickelt, wie zum Beispiel die automatische Messung mit digitalen Geräten. Dessen ungeachtet bleibt die grundsätzliche Methode nach Riva Rocci aufgrund ihrer Zugänglichkeit und Einfachheit nach wie vor eine wichtige Methode in der medizinischen Praxis.

Eine Messung nach der wie oben beschriebenen Verfahrensweise nach der Riva Rocci Methode dauert in der Regel mehr als 60 Sekunden und kann, insbesondere aufgrund des hohen notwendigen Drucks am Anfang, unangenehm für den Nutzer sein. Daher ist eine dauerhafte bzw. langfristige Überwachung des Blutdrucks nach dem Stand der Technik nur in Ausnahmefällen und mit sehr grober zeitlicher Auflösung (üblicherweise ein Wert alle 10 Minuten) möglich. Eine derartige Messreihe von Einzelmesswerten im Abstand von mehreren Minuten wird heutzutage verwendet und teils als eine "kontinuierliche" Überwachung bezeichnet. Diese Bezeichnung ist jedoch irreführend, da gerade keine kontinuierliche Überwachung stattfindet und die Intervalle und somit die zeitliche Auflösung so groß sind, dass die dynamischen Veränderungen des Blutdrucks weit unterhalb dieser zeitlichen Auflösung stattfinden. Daher kann eine Auffälligkeit nicht gesichert gefunden werden, es verbleibt vielmehr dem Zufall, ob eine Auffälligkeit detektiert wird. Eine Auffälligkeit kann nämlich nur dann aufgefunden werden, wenn das Zeitintervall der Messung zufällig so gewählt wurde, dass ein Messpunkt gerade zufällig zu einem auffälligen Ereignis stattfindet. Hinzu kommt, dass gerade bei derartigen Ereignissen eine Vielzahl der nach dem Stand der Technik messenden Manschettenmessgeräten fehlerhaft misst und darüber hinaus auch bei einer erfolgreichen Messung verfahrensbedingt keine aussagefähigen Messwerte ausgegeben kann.

Im Folgenden wird für die vorliegenden Erfindung zwar der Begriff "kontinuierlich" verwendet, jedoch nicht notwendigerweise auch mit der aus dem Stand der Technik Bedeutung einer langen Intervallmessung. Vielmehr sind zumindest die einzelnen Messintervalle kleiner und zudem können teils im gleichen Zeitraum neben dem Blutdruck auch weitere Parameter bestimmt werden, um bspw. bei einer Überwachung aussagefähigen Werte aus den Messwerten zu erhalten. Im Idealfall können Blutdruckwerte für jeden, innerhalb eines Messzeitraums vorhandenen, Herzpuls bestimmt werden, wodurch eine tatsächliche kontinuierliche Überwachung bereitgestellt wird.

Um die Probleme der Ungenauigkeiten der Messung des Blutdrucks und der kontinuierlichen Überwachung zu überwinden, wurden im Stand der Technik andere Methoden entwickelt, die den Blutdruck indirekt über andere Vitaldaten bestimmen können. Beispielsweise ist seit vielen Jahren bekannt, dass die Pulswellenlaufzeit ein Abbild des Blutdrucks ist. Theoretisch ist es mit derartigen Methoden möglich den Blutdruck für jeden Herzschlag zu bestimmen. Damit aus der Pulswellenlaufzeit oder auch aus anderen Vitalparameter der Blutdruck abgeleitet werden kann (die Messwerte der Pulswellenlaufzeit oder der anderen Vitalparameter sich sinnvoll in den Blutdruckwerte abbilden lassen), muss vor einer solchen kontinuierlichen indirekten Blutdruckmessung, die also indirekt und über den Umweg anderer Vitalparameter erfolgt, eine Kalibration zwischen Vitalwert und einer anderen den Blutdruck messenden Methode durchgeführt werden.

Für die kontinuierliche Abbildung ist jedoch auch die Analyse von ins Gewebe eingestrahltem Licht interessant.

Diese Verfahren, die unter dem Oberbegriff Photoplethysmographie (PPG) zusammengefasst werden, zeigen mit dem Herzpuls Variationen im Ausgangssignal, deren Analyse prinzipiell auch die Bestimmung des Blutdrucks ermöglicht. Der Vorteil dieser Verfahren ist die Verwendung von nur einer Messstelle am Körper und der damit einhergehenden einfachen Bedienung. Jedoch beruht auch hierbei die Blutdruckbestimmung auf einer qualitativ guten Kalibration.

Die Genauigkeit der heute bekannten Methoden (bspw. digitale Oberarmmanschette oder anderen Manschetten), die auf Riva Rocci basieren, ist jedoch nicht ausreichend, um eine einfache und genaue Kalibration durchzuführen. Wird eine Kalibration in Ruhe durchgeführt, so führt die Messungenauigkeit der Riva Rocci Methode dazu, dass im besten Falle nur eine Tendenzmessung möglich ist. Eine bessere, jedoch für den Nutzer komplizierte Kalibration mit den heute bekannten Methoden nach Riva Rocci ist möglich, wenn in Ruhe und bei Belastung des Nutzers kalibriert wird. Dieses Vorgehen hat neben den Fehlerquellen durch Bewegung des Nutzers in Belastung auch den Nachteil, dass der Nutzer aktiv einen Kalibrationsplan ausführen muss und belastet wird.

Wird bei Normaldruck kalibriert und danach soll ein hoher Blutdruck vermessen werden (z.B. beim Sport), so vervielfachen sich die Fehler, die bei der Kalibration eingebracht wurden. Daher ist es entscheidend für eine kontinuierliche und nicht belastende Messung des Blutdrucks auf dem Niveau einer invasiven Blutdruckmessung, dass die Kalibration mit hochpräzisen und möglichst fehlerfreien Messwerten, die auch geringste Blutdruckveränderungen zeigen, durchgeführt wird. Für eine Kalibration ist eine Änderung im Blutdruck notwendig. Ist die Messqualität der Messung zur Kalibration sehr hoch, kann diese Änderung eine natürliche Änderung sein, z.B. die Blutdruckänderung aufgrund der Atmung oder die Blutdruckveränderung aufgrund der turnusmäßigen Arterienanpassung in den Extremitäten.

Heute ist auf dem Markt kein System vorhanden, was kontinuierlich und nicht invasiv den Blutdruck so messen kann, dass eine Diagnose oder Warnung von Erkrankungen möglich wäre. Heutige Verfahren, die eine solche Funktionalität versprechen, mangelt es an Genauigkeit und der Fähigkeit Extremveränderungen des Blutdrucks richtig zu erkennen. Die Gefahr besteht, dass gesunde Menschen als krank eingestuft werden, wodurch diese Menschen sich unnötig Sorgen machen und Gesundheitsmaßnahmen in Anspruch nehmen, wodurch diese Maßnahmen für die Allgemeinheit unnötig verknappt werden. Dies ist jedoch zur umgekehrten Problematik verhältnismäßig akzeptabel. Werden kranke Menschen durch heutige Systeme als gesund eingestuft, so kann dies die Lebensqualität mindern oder sogar lebensbedrohlich für diese Menschen sein. Kontinuierlich messende Systeme versprechen, dass Krankheiten frühzeitig erkannt werden. Wird jedoch eine Krankheit nicht erkannt, so wird der Nutzer in Sicherheit gewogen, bis es möglicherweise zu spät für eine optimale Behandlung ist.

Die vorliegende Erfindung soll diese Lücke schließen, sodass die Methode zur nicht invasiven und insbesondere kontinuierlichen Blutdruckmessung nutzerfreundlich und fehlerfreier kalibriert werden können, sodass medizinische Diagnosen basierend auf den Messwerten ermöglicht werden. Aber das Verfahren stellt auch an sich einen Mehrwert dar. Das Verfahren bestimmt nicht nur den Blutdruck nach heutiger Definition, sondern bezieht auch die dynamischen Einflüsse auf den Blutdruck durch die Atmung mit ein, sodass das Bild des Blutdrucks breiter dargestellt werden kann. So kann die Blutdruckschwankung aufgrund der Atmung neben Systole und Diastole als weiterer Parameter in die Definition des Blutdruckwertetupels miteingehen.

Ein Messwert des Blutdrucks einer Person wird grundsätzlich nicht nur durch den Herzschlag bzw. Herzzyklus beeinflusst. Vielmehr wird der Druck des Blutes in den Arterien durch den Körper aufgrund unterschiedlichster Phänomene reguliert. Diese Regulierung führt zur Schwankung des Blutdrucks und dessen Wert. Typisch sind folgende Ursachen und Phänomene für die Schwankung:

- Herzschlag
- Atmung
- Arterienanpassung in den Extremitäten
- Körperliche Anstrengungen
- Psychische Anstrengungen
- Flüssigkeitshaushalt
- Nahrungsaufnahme
- Schlafzyklus
- Menstruationszyklus
- Monatszyklus
- Arterienzustand
- Lebensalter

Die Blutdruckschwankung als Reaktion des Körpers auf diese Phänomene wird in Ordnungen eingeteilt. Die Ordnung stellt die Wirkung dar und das Phänomen die Ursache.

In der Literatur wird oft diese Differenzierung vermischt. Die Ordnung bezieht sich auf eine Änderungsfrequenz im Blutdruck und das Phänomen auf eine Frequenz in der Ursache. Beispielsweise folgt aus der Atmung die Blutdruckschwankung II. Ordnung. Aber die Phase und die Amplitude des Phänomens Atmung sind nicht notwendigerweise korreliert zur Phase und Amplitude der Blutdruckschwankung II. Ordnung. Mittels der vorliegenden Erfindung kann ein von den durch die Atmung verursachten Blutdruckschwankung unabhängiger Blutdruckwert bestimmen werden. Damit können Phasenverschiebung und abnormale Amplitudenverhältnisse bestimmt werden. Was aus dem vom normal abweichenden Werten erfasst werden kann ist außerhalb dieser Patentschrift und wird in nachfolgenden Veröffentlichungen beschrieben.

In der Literatur (z.B. H. Hinghofer-Szalkay: Praktische Physiologie, 3. Aufl. Blackwell Berlin 1994) werden folgende Ordnungen unterschieden:

- Blutdruckschwankung I. Ordnung: Blutdruckschwankungen deren Ursache sich durch das Phänomen des Herzschlags ergeben. Die Form der Schwankung hängt vom Herzen und den Arterien ab. Der systolische Druck wird maßgeblich von der Auswurfleistung des linken Herzens und der Dehnbarkeit des Arteriensystems beeinflusst. Der diastolische Druck wird maßgeblich von der Pulsfrequenz und dem peripherem Strömungswiderstand beeinflusst.

- Blutdruckschwankung II. Ordnung: Blutdruckschwankungen deren Ursache durch das Phänomen der Atmung verursacht werden.

- Blutdruckschwankung III. Ordnung: Blutdruckschwankungen deren Ursache durch das Phänomen der Anpassung der Arterien in den Extremitäten verursacht werden. Diese Schwankungen werden auch Traube-Hering-Meyer-Wellen genannt.

Die Phänomene treten mit unterschiedlichen Zeitlängen auf. Daher kann eine Blutdruckverlaufsmessung mit z.B Fourieranalyse auf den Einfluss der einzelnen Phänomene hin untersucht werden. Es wird möglich den Einfluss auf den Blutdruck eines Phänomens für sich zu erkennen. Bekannt ist dies für die Atmung, es ist bekannt, dass die Atmung bis zu 15 mmHg in der Systole und bis zu 5 mmHg in der Diastole den Blutdruck ausgehend vom ruhenden Körper verändern kann. Diese Differenzierung trifft heutzutage jedoch an ihre Grenzen. Möglich ist heutzutage jedoch nur die Differenzierung von zwei gleichzeitig vorhandenen Phänomenen. Eines dieser Phänomene ist immer der Herzschlag, was zu den bekannten Blutdruckwerten Systole und Diastole führt und eines der anderen Phänomene, was heutzutage durch einen Druckunterschied zu den Blutdruckwerten Systole und Diastole benannt wird.

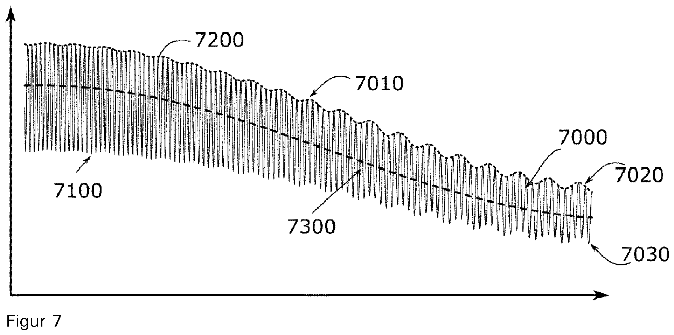

Die Auswirkungen der einzelnen Phänomene auf den Blutdruck können sich gegeneinander aufheben oder verstärken. Beispiel: Unter Belastung kann die Auswirkung der Atmung bei vor allem älteren Menschen nicht mehr erkannt werden. Dies kann dadurch erklärt werden, dass die Steifigkeit der Arterien (Phänomen: Arterienzustand) hoch ist und dass unter hohem Druck eine weitere Erhöhung durch die Atmung unterdrückt wird. Für die Phänomene Herzschlag, Atmung und Belastung ist in Abbildung 7 schematisch die Druckverlaufskurve gezeigt. In dieser Abbildung wird skizziert, wie sich die einzelnen Phänomene auswirken und gegeneinander beeinflussen. In der Abbildung 7 ist schematisch ein Druckverlauf 7000 des Blutes in einer Arterie einer Person als Funktion der Zeit gezeigt, wobei sich der Belastungszustand der Person über die Zeit hinweg ändert. In dem dargestellten Beispiel ist eine Erholungsphase dargestellt, weshalb der Druck in einer globalen Betrachtung absinkt (angedeutet mit der Linie 7300). Auf dem generellen Trend des Absinkens ist jedoch auch ein regionaler Trend erkennbar, am Anfang weniger, gegen Ende, wenn die Person sozusagen erholter ist, stärker. Dieser regionale Trend ist mit der Linie 7200 angedeutet und kann dem Phänomen des Einflusses der Atmung auf den Blutdruck zugeordnet werden. Zuletzt, in der Betrachtung der lokalen Änderungen des Blutdruckverlaufs ist dann noch eine weitere Änderung zu erkennen, deren Stärke (Amplitude der Schwingungskurve) anfangs am höchsten, gegen Ende, wenn die Person erholter ist, geringer, ist. Diese Änderungen entsprechen dabei den Änderungen aufgrund des Herzpulses des Herzens der Person und ist mit der Linie 7100 indiziert. Aus der Abbildung sind somit die Phänomene im Einzelnen, aber auch deren Zusammenwirken ersichtlich.

Fragestellungen, die drei Phänomene betrachten, können messtechnisch aufgrund fehlender Auflösung (oder wenn man die invasive Messung verwenden würde, aufgrund der schwierigen Verwendung), nicht beantwortet werden. Eine solche Fragestellung ist: Welchen Einfluss hat die Atmung auf die Blutdruckwerte Systole und Diastole, wenngleich eine körperliche Anstrengung vorliegt. Für eine Antwort müssen die Einflüsse der Phänomene Herzschlag, Atmung und körperliche Anstrengung auf den Druck in den Arterien getrennt betrachtet werden.

Die vorliegende Erfindung kann von einem Nutzer so verwendet werden, dass nur die zwei Phänomene Herzschlag und Atmung eine Änderung des Blutdrucks bewirken. Einfluss anderer Phänomene mögen zwar vorliegen, deren Einfluss auf die Werte der Messung sollten jedoch aber nur über eine längere Zeitspanne vorhanden sein, sodass der Einfluss über den Messzeitraum als konstant angesehen werden kann. Das Verfahren verwendet insbesondere die Änderung des Blutdrucks aufgrund der Atmung, um die Blutdruckveränderung aufgrund des Herzschlags zu erkennen und dann auch zu späteren Zeitpunkten zu verfolgen und dadurch die Veränderungen durch irgendwelche anderen Phänomene zu erkennen.

Die Atmung verändert die Herzaktionen. Bei Einatmung steigt die Herzrate leicht an und die Arterien versteifen. Wird ausgeatmet sinkt der Puls und auch die Arterienversteifung nimmt ab. Die führt zu einer regelmäßigen Blutdruckanpassung.

Ausgelöst wird dies durch eine Aktivierung des sympathischen Nervensystems bei Einatmung und des parasympathischen Nervensystems während der Ausatmung. Bei normaler Atmung und bei gesunden Menschen bedeutet dies eine Veränderung von 2-3 mmHg für die Systole und 1-2 mmHg für die Diastole. Starke Atmung oder Erkrankungen können jedoch dazu führen, dass diese Veränderung während der Atmung ausgeprägter ist. Neben der Atmung hat die körperliche Belastung, der Gesundheitszustand, die Medikation, der körperliche Zustand und das Alter einen Einfluss auf die Blutdruckanpassung. Ein gesunder junger erwachsener Mensch zeigt bei der Einatmung eine Blutdruckabsenkung bei gleichzeitiger Pulserhöhung und umgekehrt bei der Ausatmung. Mit körperlicher Belastung steigt sowohl Blutdruck als auch Puls. Schon das Beispiel am gesunden Menschen zeigt, dass die Anpassung des Blutdrucks komplex ist. Neben diesen körperlichen Einflüssen hat der Ort bzw. die Art der Messung einen Einfluss auf das Ergebnis der Messung. Eine Manschettenmessung bildet den peripheren Blutdruck in der betroffenen Extremität ab. Die Einflüsse auf den peripheren Blutdruck sind wiederum vielfältig, bspw. kann der Blutfluss durch Erkrankungen wie Arteriosklerose oder beeinträchtigte Windkesselfunktion beeinträchtigt werden. Ein anderes Beispiel ist, dass bei sportlicher Aktivität der zentrale Blutdruck steigt und gleichzeitig steigt der Blutdruck im Arm nicht derart, wenn dieser nicht belastet ist (z.B. beim Fahrradfahren). In der Regel gilt, wenn die Ursache der Daten nur peripher begründet ist, so ist die Blutdruckmessung auch eine Blutdruckmessung für den peripheren Druck. Beispiele hierfür sind Manschettenmessung, Lichtwellenmessung, akustische Messungen und Temperaturmessungen. Wenn Messmethoden auch direkte Aktionen des Herzens aufzeichnen, z.B. bei der Verwendung eines EKGs, so wird zumindest zum Teil auch der zentrale Blutdruck abgebildet, ein Beispiel hierfür ist die Blutdruckbestimmung basierend auf der Pulswellenlaufzeit.

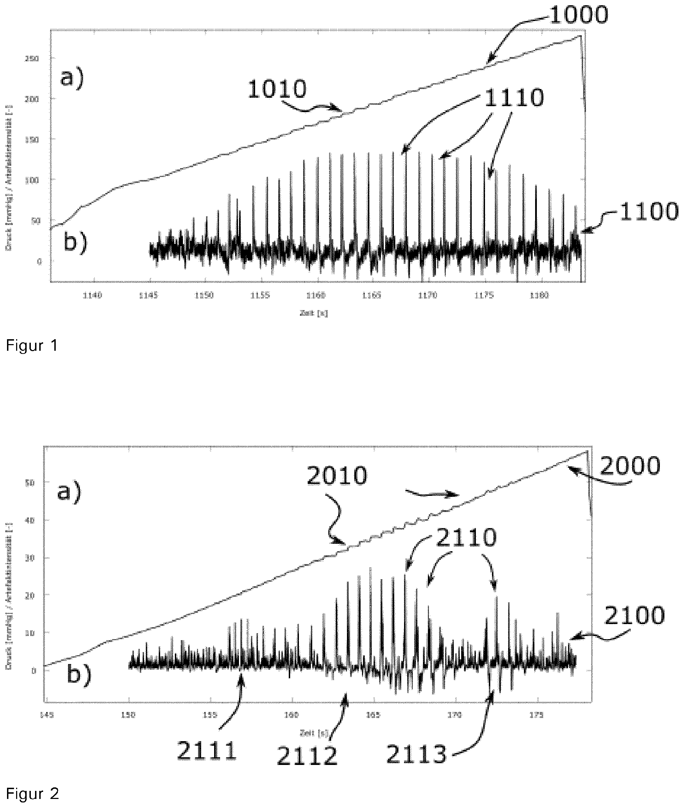

Bei einer Messung nach dem Stand der Technik (vgl. Figur 1) wird der Druck in der Manschette mit einer konstanten Rate erhöht. Der Luftdruck, der in der Manschette vorliegt, wird von dem Puls in den Arterien, die unterhalb der Manschette liegen, beeinflusst. Bei geringen Drücken kann das Blut ungestört fließen, dies bedeutet, dass die Spannung der Muskeln und des Gewebes höher ist als der Druck in der Manschette. Daher ist der Puls oder eine Druckänderung in dem Verlauf der Druckkurve nicht zu erkennen. Steigt der Luftdruck in der Manschette so kann diese Spannung, zunächst während der Diastole, im Herzpuls nicht aufrechterhalten werden. Während der Diastole im Herzpuls kommt es zu Artefakten, also zu Abweichungen, zu niedrigeren Messwerten des Luftdrucks mit dem Herzpuls. Steigt der Druck in der Manschette weiter, kann der Blutfluss auch bei Phasen zwischen Diastole und Systole im Herzpuls nicht mehr störungsfrei aufrechterhalten werden. Bei noch höheren Luftdrücken ist der Blutfluss zu jeder Phase gestört, also auch während der Systole. Die Manschette wird mit dem Puls verformt, der Herzpuls ist somit im Luftdruck abgebildet. Der Luftdruck ist nun genau zwischen dem Wert von Diastole und Systole. Wird der Luftdruck weiter erhöht, so wird die Arterie mehr und mehr abgequetscht bis kein Blutfluss mehr möglich ist, da die Arterie kollabiert ist. Die Intensität der Artefakte aufgrund des Herzpulses steigen zunächst mit dem Luftdruck und fallen danach wieder ab. Die Kurve der Intensität der Artefakte aufgrund der Herzpulses zeigt also genau einen Bogen.

Selbstredend entspricht das Verhalten des Blutdrucks einer realen Person jedoch in der Regel gerade nicht dem idealisierten Verlauf, den die Verfahren nach dem Stand der Technik jedoch annehmen und verwenden. Ein solcher nicht idealisierter Verlauf ist in der Figur 2 gezeigt. So verändert sich der Blutdruck und damit die Luftdruckkurve 2000 in der Manschette durch die Atmung und die Kurve der Intensität 2100 der Artefakte 2010, 2110 aufgrund des Herzpulses zeigt mehrere Bögen 2111, 2112, 2113. In den Verfahren aus dem Stand der Technik wird nun bspw. nur ein Bogen z.B. 2112 aus dieser Kurve verwendet. Dies führt zu einer Überschätzung der Diastole und/oder Unterschätzung der Systole. In den Verfahren aus dem Stand der Technik werden teils bspw. auch alle Bögen gemittelt verwendet und als ein Bogen interpretiert, was zu einer Unterschätzung der Diastole und Überschätzung der Systole führt.

Das Problem an den Verfahren aus dem Stand der Technik liegt also darin, dass für die Messungen bestimmten Annahmen getroffen werden und insbesondere zusätzliche Effekte, die den Blutdruckwert beeinflussen, ignoriert oder aufgrund der großen Intervalle der Messzeiträume verfahrensbedingt willkürlich gemittelt werden. Im Besonderen liegt das Problem bei den vorbekannten Messverfahren darin, dass aus den Einzelwertmessungen bestimmten Werten oder Mittelwerte von bestimmten Werten der Einzelwertmessungen Blutdruckeinzelwerte zugeordnet werden, wobei bei den Einzelwertmessungen keine weiteren Informationen berücksichtigen werden und daher lediglich ein Einzelwert des unkorrigierten, insbesondere nicht weitere Effekte, wie bspw. die Atmung, berücksichtigenden, Blutdruckwert ausgeben wird. Insbesondere ist daher das Problem gegeben, dass aus Luftdruckmessungen keine kontinuierlichen Blutdruckwerte bestimmbar sind bzw. dass die Effekte der Atmung auf die Blutdruckwertmessung ignoriert bzw. willkürlich gemittelt werden, sodass die dabei ermittelten Blutdruckwerte von dem jeweiligen Effekt der Atmung abhängen.

Die vorliegende Erfindung schafft eine Lösung für das vorgenannte Problem, indem es eine bessere Auswertung von Einzelwertmessungen, insbesondere Luftdruckeinzelwertmessungen, bereitstellt. Die Einzelwertmessungen sind beispielsweise solche, die einen Druckwert auf die Haut oberhalb von Arterien darstellen. Heutzutage bevorzugt und auch in dieser Offenbarung der Erfindung vorstellbar, werden diese Druckwerte mit einer Manschette erzeugt und mit dem Luftdruck innerhalb der Manschette quantifiziert. Gleichwohl sind auch weitere Möglichkeiten vorhanden und mit der vorliegenden Erfindung umsetzbar, eine derartige Druckbeaufschlagung zu erzeugen und zu quantifizieren. Eine Möglichkeit ist die Verwendung von Drucksensoren auf keramischer Basis, den Piezoeffekt ausnutzend, oder auf Polymerbasis, durch Messung eines veränderlichen elektrischen Widerstands, welche mit einer mechanischen Vorrichtung an die Haut gedrückt werden. Ungeachtet dessen, wie die Druckbeaufschlagung erzeugt wird und wie diese quantifiziert wird, können die Werte der Einzelwertmessungen derartiger Vorrichtungen für das erfindungsgemäße Verfahren und System verwendet werden.

Insbesondere wird das Problem durch das Verfahren nach Anspruch 1 sowie eine Messvorrichtung, insbesondere in Form einer Manschette, nach Anspruch 13 gelöst. Weitere vorteilhafte Ausgestaltungen sind in den weiteren, abhängigen Ansprüchen sowie in den folgenden Ausführungen ausgeführt.

Dabei ist es möglich mit dem erfindungsgemäßen Verfahren einen atmungskorrigierten Blutdruckwert einer, insbesondere atmenden, Person zu ermitteln. Ein solcher atmungskorrigierten Blutdruckwert gibt einen Blutdruckwert in Abhängigkeit eines bestimmten Atemzustands der Atmung der Person an und ist somit ein atmungskorrigierter Blutdruckwert im Sinne dieser Offenbarung. Ein solcher Atemzustand kann bspw. eine maximale Einatmung oder eine maximale Ausatmung darstellen. Um ein genaueres Bild des Blutdrucks in Abhängigkeit der Atmung bereitzustellen, wird daher vorgeschlagen, dass mindestens zwei atmungskorrigierte Blutdruckwerte ermittelt werden, die möglichst zwei gegensätzliche Extremzustände in dem Atemzustand einer Atemphase der Person abbilden.

Die Erfindung betrifft nun ein Verfahren zum Ermitteln von mindestens zwei atmungskorrigierten ersten Blutdruckwerten einer Person. Ein solcher erster Blutdruckwert kann insbesondere ein systolischer Blutdruckwert sein. Möglich ist es jedoch auch, dass der erste Blutdruckwert ein diastolischer Blutdruckwert ist. Vorstellbar ist jedoch auch, dass neben den mindestens zwei atmungskorrigierten ersten Blutdruckwerten, nämlich systolischen Blutdruckwerten, auch mindestens zwei atmungskorrigierte zweite Blutdruckwerte, nämlich diastolische Blutdruckwerte, ermittelt werden.

Möglich ist es auch eine Vielzahl atmungskorrigierter erster Blutdruckwerte zu ermittelten. Dabei müssen die Werte der Vielzahl nicht mehr jeweils den oben beschriebenen zwei gegensätzliche Extremzustände zugeordnet werden, sofern die Vielzahl auf derartigen Werten basiert. Zum besseren Verständnis wird die Vielzahl der atmungskorrigierten ersten Blutdruckwerte als erste Vielzahl bezeichnet. Auch hier kann der erste Blutdruckwert der ersten Vielzahl ein systolischer Blutdruckwert oder aber ein diastolischer Blutdruckwert sein. Möglich ist es auch sowohl die erste Vielzahl der ersten Blutdruckwerte, nämlich die systolischen Blutdruckwerte, und eine weitere Vielzahl von atmungskorrigierten zweiten Blutdruckwerten, nämlich diastolischen Blutdruckwerten, zu ermitteln. Diese weitere Vielzahl der atmungskorrigierten zweiten Blutdruckwerten, nämlich diastolischen Blutdruckwerten, wird zum besseren Verständnis als zweite Vielzahl bezeichnet.

Dabei erfolgt die Ermittlung der mindestens zwei atmungskorrigierten ersten (oder eben auch der weiteren, wie der zweiten) Blutdruckwerte wie nachfolgenden beschrieben. Dabei ist unter dem Merkmal ,atmungskorrigiert' insbesondere eine Ermittlung von mindestens zwei Blutdruckwerten zu verstehen, bei welchen Blutdruckwerte verwendet werden, die innerhalb ein und desselben Phasenabschnitts einer Atmung ermittelt werden können. Es werden also absichtlich zwei Werte verwendet, die sich innerhalb derselben Atemphase befinden, wodurch sich die vorliegende Erfindung grundsätzlich von den Herangehensweisen der Verfahren aus dem Stand der Technik unterscheidet, bei der derartige Werte nicht einzeln betrachtet und verwendet werden, sondern vielmehr durch beispielsweise einen Algorithmus basierend auf dem gleitenden Mittelwert unberücksichtigt bleiben. Problematisch an dieser Herangehensweise aus dem Stand der Technik ist dabei, dass die Information des Einflusses der Atmung auf den Blutdruck verloren geht und - je nach Auswahl des Bezugsrahmens des Algorithmus - ein falscher Absolutwert ausgegeben wird.

Ausgangspunkt des erfindungsgemäßen Verfahrens ist eine zeitaufgelöste Erfassung von Einzelwerten eines Wertes, der dem Blutdruckwert zugeordnet werden kann. Hierfür werden mehrere Einzelwertmessungen innerhalb eines Messzeitraums durchgeführt. Diese können in kurzem Abstand mittels einer ersten Vorrichtung auf Basis eines ersten Sensors der ersten Vorrichtung durchgeführt werden. Mittels weiterer Verfahrensschritte können dann aus diesen Einzelwertmessungen atmungskorrigierte Blutdruckwerte, und insbesondere kontinuierliche Blutdruckwerte, ermittelt werden.

Dabei können die Einzelwertmessungen in der Regel auf einigen vorbekannten Vorrichtungen ausgeführt werden. Es bedarf demnach nicht notwendigerweise neuer Vorrichtung, die vorbekannten Vorrichtung müssen lediglich diesbezüglich erweitert und eingerichtet werden, die erfindungsgemäßen Verfahren auszuführen. Dies kann im Idealfall durch ein Softwareupdate umgesetzt werden.

Dabei kann in dem erfindungsgemäßen Verfahren insbesondere, wie auch teils aus dem Stand der Technik bekannt, innerhalb des Messzeitraums die, insbesondere erste, Vorrichtung eine Druckbeaufschlagung durchführen. Eine derartige Druckbeaufschlagung erfolgt in der Regel auf einen Teil der Person, der als Messbereich verwendet wird, in der Regel auf einen Teil der Haut der Person. In der Regel ist die Vorrichtung dabei so konfiguriert, dass diese die Druckbeaufschlagung variiert. Hierfür wird in der Regel eine Druckbeaufschlagung anhand einer über die Zeit des Messzeitraums vorbestimmte Druckbeaufschlagungsänderungsrate durchgeführt. So kann beispielsweise die Druckbeaufschlagung auf die Haut innerhalb des Messzeitraums ansteigen, dies gemäß einer vorbestimmte Druckbeaufschlagungsänderungsrate. Im weiteren Verlauf und gemäß der vorbestimmten Druckbeaufschlagungsänderungsrate, kann dieser Anstieg größer oder kleiner werden, oder es kann sogar die Druckbeaufschlagung wieder zurückgenommen werden. Möglich ist es aber auch anfangs, ausgehend von einer schon vorhandenen hohen Druckbeaufschlagung, die Druckbeaufschlagung zu reduzieren, dies anhand der vorbestimmten Druckbeaufschlagungsänderungsrate.

Jedenfalls weisen die Werte der Einzelmessungen diese variierende Druckbeaufschlagung zumindest teilweise auf.

Vorteilhafterweise variiert somit beispielsweise innerhalb des Messzeitraums ein auf einen als Messbereich verwendeter Teil der Person ausgeübter Druck, insbesondere steigt dieser an oder ab, insbesondere von einem Minimalwert des Drucks hin zu einem Maximalwert des Drucks oder von einem Maximalwert des Drucks zu einem Minimalwert des Drucks. Vorteilhafterweise erfolgt die Erfassung der Einzelwertmessungen während der Variation des Drucks. Vorteilhafterweise wird der auf den Messbereich ausgeübte Druck und deren Variation durch die erste Vorrichtung, der auch der erste Sensor zugeordnet ist, generiert.

Im einfachsten Fall handelt es sich bei den Werten der Einzelwertmessung um Druckwerte. Insbesondere kann es sich bei den Druckwerten um Luftdruckwerte handeln, wobei bevorzugt für die Einzelwertmessung eine Manschette, insbesondere eine Luftdruckmanschette, verwendet wird. Insbesondere kann es sich bei dem Druckwert um einen auf Basis einer Messung einer, insbesondere durch den Piezoeffekt verursachten, elektrischen Spannung oder des elektrischen Widerstandes ermittelten Druckwert handeln. Insbesondere weist der erste Sensor einen Drucksensor, insbesondere ein Luftdrucksensor, oder einen Sensor zur Messung der elektrischen Spannung oder des elektrischen Widerstandes auf. Insbesondere ist die erste Vorrichtung eine, insbesondere automatisierte, Druckmanschette.

Unter zeitaufgelöst ist insbesondere eine Messung zu verstehen, bei der sowohl der Messwert, in diesem Fall der Wert, der dem Blutdruckwert zugeordnet wird, und der Zeitpunkt der Erfassung des Messwerts erfasst wird.

Erfindungsgemäß wird/werden in einem weiteren Verfahrensschritt aus den Einzelwertmessungen mindestens ein nicht-atmungskorrigierter erster Blutdruckwert der Person bestimmt. Auch hier ist unter erster Blutdruckwert beispielsweise ein systolischer Blutdruckwert oder ein diastolischer Blutdruckwert zu verstehen. Sofern sowohl atmungskorrigierte erste, nämlich systolische, Blutdruckwerte als auch atmungskorrigierte zweite, nämlich diastolische, Blutdruckwerte ermittelt werden, dann ist vorgesehen, dass neben der Bestimmung des mindestens einen nicht atmungskorrigierten ersten, systolischen, Blutdruckwerte aus den Einzelwertmessungen mindestens ein nicht-atmungskorrigierter zweiter, diastolischer, Blutdruckwert der Person bestimmt wird. Diese Bestimmung des/der nicht-atmungskorrigierten Blutdruckwerts/e erfolgt insbesondere nach dem aus dem Stand der Technik bekannten Methoden, insbesondere nach der Methode gemäß Riva-Rocci und Korotkoff. Dabei wird der Zeitpunkt, der dem Zeitpunkt des Wertes der zeitaufgelösten Einzelwertmessungen entspricht und der dem Zeitpunkt des bestimmten nicht-atmungskorrigierten Blutdruckwertes entspricht, je als ein Referenzzeitpunkt bestimmt. Für den nicht-atmungskorrigierter erster Blutdruckwert wird demnach dieser Zeitpunkt als ein erster Referenzzeitpunkt bestimmt. In analoger Weise kann daher für den nicht-atmungskorrigierter zweiten Blutdruckwert demnach dessen Zeitpunkt als ein zweiter Referenzzeitpunkt bestimmt werden.

Ferner wird erfindungsgemäß zumindest ein erster Phasenabschnitt einer Atmung, insbesondere ein erster Phasenabschnitt einer Atemphase, der Person innerhalb des Messzeitraums zeitaufgelöst erfasst. Diese Erfassung erfolgt insbesondere in Form einer Erfassung einer Atmungskurve der Person, die Phasenabschnitte der Atmung, insbesondere der einzelnen Atmungsphasen, aufweisen. Die Phasenabschnitte, somit auch der erste Phasenabschnitt, weist dabei einen, insbesondere lokalen, Maximalwert in der Atmungskurve auf. Dies kann beispielsweise ein im Vergleich zu vorangehenden und späteren Werten der Atmungskurve maximaler Wert sein. Ein solcher Maximalwert kann beispielsweise eine innerhalb der Atemphase stattfindenden maximale Einatmung der Person sein. Die Zuordnung eines Maximalwerts zu einer maximalen Einatmung ist jedoch maßgeblich von der Methode abhängig, die zur Bestimmung der Atmungskurve und der Phasenabschnitte verwendet wird. Der Einfachheit wegen wird diese Zuordnung verwendet, auch wenn es sich bei dem Maximalwert aus mathematischer Sicht auch nicht um einen maximalen Wert handeln kann.

Durch die zeitaufgelöste Erfassung sind auch die Zeitpunkte dieser Maximalwerte erfassbar. Der Maximalwert des ersten Phasenabschnitts wird dabei als erster Zeitpunkt, nämlich als erster Zeitpunkt des ersten Phasenabschnitts erfasst. Sofern ein zweiter Phasenabschnitt in analoger Weise erfasst wird, wird dessen Maximalwert ebenfalls als erster Zeitpunkt, nämlich als erster Zeitpunkt des zweiten Phasenabschnitts, erfasst.

Die Phasenabschnitte, somit auch der erster Phasenabschnitt, weisen zudem auch Minimalwerte in der Atmungskurve auf. Dies kann beispielsweise ein im Vergleich zu vorangehenden und späteren Werten der Atmungskurve minimaler Wert sein. Ein solcher Minimalwert kann beispielsweise eine innerhalb der Atemphase stattfindenden maximale Ausatmung der Person sein. Die Zuordnung eines Minimalwerts zu einer maximalen Ausatmung ist jedoch maßgeblich von der Methode abhängig, die zur Bestimmung der Atmungskurve und der Phasenabschnitte verwendet wird. Der Einfachheit wegen wird diese Zuordnung verwendet, auch wenn es sich bei dem Minimalwert aus mathematischer Sicht auch nicht um einen minimalen Wert handeln kann. Insbesondere relevant ist vielmehr, dass der Maximalwert den Gegensatz zum Minimalwert darstellt.

Durch die zeitaufgelöste Erfassung sind auch die Zeitpunkte dieser Minimalwerte erfassbar. Der Minimalerwert des ersten Phasenabschnitts wird dabei als zweiter Zeitpunkt, nämlich als zweiter Zeitpunkt des ersten Phasenabschnitts erfasst. Sofern ein zweiter Phasenabschnitt in analoger Weise erfasst wird, wird dessen Minimalwert ebenfalls als zweiter Zeitpunkt, nämlich als zweiter Zeitpunkt des zweiten Phasenabschnitts, erfasst.

Dabei wird aus den erfassten Phasenabschnitten der erste Phasenabschnitt so gewählt, dass der erste Referenzzeitpunkt innerhalb des den ersten Phasenabschnitt umfassenden Zeitraums fällt. Dabei kann der Zeitraum des ersten Phasenabschnitt insbesondere durch den ersten und den zweiten Zeitpunkt des ersten Phasenabschnitts definiert werden. Gleichwohl muss das Ende oder der Beginn des Zeitraums des ersten Phasenabschnitts nicht notwendigerweise mit dem ersten bzw. zweiten Zeitpunkt des ersten Phasenabschnitts, insbesondere exakt übereinstimmen. Eine solche Nähe, insbesondere Übereinstimmung ist insbesondere abhängig von der Methode, die gewählt wurde, die Phasenabschnitte bzw. die Maximalwerte und Minimalwerte zu erfassen und zu bestimmen.

Sofern auch ein zweiter Referenzzeitpunkt erfasst bzw. bestimmt wurde, ist es vorgesehen, dass ein zweiter Phasenabschnitt in analoger Weise wie auch der erste Phasenabschnitt erfasst und dessen Zeitpunkte bestimmt werden, wobei aus den Phasenabschnitten der zweite Phasenabschnitt so gewählt wird, dass der zweite Referenzzeitpunkt innerhalb des den zweiten Phasenabschnitt umfassenden Zeitraums fällt. hinsichtlich des Zeitraums des zweiten Phasenabschnitts wird auf obiges hinsichtlich des Zeitraums des ersten Phasenabschnitts verwiesen.

Hinsichtlich der zeitaufgelösten Erfassung innerhalb des Messzeitraums wird bevorzugt, dass für die Erfassung der Einzelwerte und für die Erfassung einer Atmung, insbesondere einer Atmungskurve, insbesondere der nachfolgend beschriebenen Atmungskurve, dieselbe Zeitauflösung, insbesondere mit denselben Zeitintervallen und denselben Zeitpunkten, verwendet wird.

Insbesondere enthält jeder Phasenabschnitt genau einen Maximalwert, insbesondere genau eine maximale Einatmung, und genau einen Minimalwert, insbesondere genau eine maximale Ausatmung.

Dabei muss der erste Zeitpunkt bspw. des ersten Phasenabschnitts nicht notwendigerweise zeitlich vor dem zweiten Zeitpunkt des ersten Phasenabschnitts sein. Vielmehr basieren die Nummerierungen der Zeitpunkte den Werten in der Atmungskurve, also der erste Zeitpunkt einem Maximalwert und der zweite Zeitpunkt einem Minimalwert. So kann der Phasenabschnitt zeitlich zuerst einen Maximalwert aufweisen und daraufhin einen Minimalwert. Gleichwohl kann der Phasenabschnitt jedoch auch zuerst einen Minimalwert und dann einen Maximalwert aufweisen. In der Regel weist eine Atmungsphase, je nach Definition einer solchen Atmungsphase entweder zwei Maximalwerte und einen Minimalwert (Atmungsphase wird so definiert, dass diese zuerst eine Einatmung aufweist, dann über eine Ausatmung zur nächsten Einatmung gelangt) oder zwei Minimalwerte und einen Maximalwert (Atmungsphase wird so definiert, dass diese zuerst eine Ausatmung aufweist, dann über eine Einatmung zur nächsten Ausatmung gelangt). Auch ist es vorstellbar, dass der erste Phasenabschnitt zuerst einen Maximalwert aufweist und der zweite Phasenabschnitt zuerst einen Minimalwert, oder umgekehrt. Erfindungsgemäß relevant ist lediglich, dass der jeweilige Referenzzeitpunkt innerhalb der jeweiligen durch die jeweiligen ersten und zweiten Zeitpunkte der jeweiligen Phasenabschnitte definierten Zeiträume der Phasenabschnitte liegt bzw. fällt.

Bevorzugt wird der Zeitraum des ersten Phasenabschnitts auf Basis der Zeitpunkte des ersten Zeitpunkts, insbesondere der maximalen Einatmung, und des zweiten Zeitpunkts, insbesondere der maximalen Ausatmung, abgeleitet. Insbesondere wird der Beginn des ersten Phasenabschnitts auf Basis eines der Zeitpunkte bestehend aus dem ersten Zeitpunkt und zweiten Zeitpunkt und das Ende des ersten Phasenabschnitts auf Basis des anderen Zeitpunkts, also des zweiten Zeitpunkts oder des ersten Zeitpunkts, bestimmt.

Vorteilhafterweise stimmt der erste Zeitpunkt eines Phasenabschnitts auch mit dem einem aus Beginn und Ende des Phasenabschnitts und der zweite Zeitpunkt des Phasenabschnitts mit dem anderen aus Beginn und Ende des Phasenabschnitts überein. Die Erfinder haben jedoch erkannt, dass eine solche Übereinstimmung nicht notwendigerweise gegeben ist. Vielmehr ist oft ein Divergieren zwischen eigentlichem Anfang des Phasenabschnitts und bspw. ersten Zeitpunkt oder zweiten Zeitpunkt feststellbar. Die Ursache hierfür liegt in der Messmethode begründet, mit der die Atmung und somit die Atmungskurve, aus der die Phasenabschnitte bestimmt werden, erfasst werden. Je nach Messmethode sind nämlich die Auswirkungen eines dem Maximalwert eigentlich zugrunde liegenden Effekts, eine maximale Einatmung, erst mit einem zeitlichen Versatz vorhanden oder messbar. Daher kann es notwendig sein, dass sowohl der Beginn als auch das Ende des Phasenabschnittes, um so den Zeitraum des Phasenabschnittes zu bestimmen, in dem der Referenzzeitpunkt liegen soll, jeweils auf Basis sowohl des ersten Zeitpunktes als auch des zweiten Zeitpunktes oder auf Basis eines ersten bzw. zweiten Zeitpunktes eines vorhergehenden oder nachfolgenden Phasenabschnitts zu bestimmen. Hierfür kann es notwendig sein, eine vorbestimmte Wichtung der verwendeten Werte, erster und zweiter Zeitpunkt der beteiligten Phasenabschnitte, zu verwenden. Diese Wichtung hängt dabei beispielsweise von der verwendeten Methode ab.

Für die vorbestimmte Wichtung kann beispielsweise ein Zeitmittelpunkt bestimmt werden.

Für den gewichteten Zeitmittelpunkt wird beispielsweise der zeitliche Abstand zwischen den jeweils verwendeten ersten und zweiten Zeitpunkten, insbesondere der jeweils verwendeten maximalen Ausatmung und maximalen Einatmung, verwendet. Ferner werden Offset-Korrekturen der Bestimmungsmethode des ersten und zweiten Zeitpunkts, insbesondere der maximalen Einatmungen und maximalen Ausatmung, für den gewichteten Zeitmittelpunkt verwendet. Diese können entweder vom Hersteller vorgegeben sein oder experimentell zu bestimmen sein.

Die Erfinder haben nämlich erkannt, dass bei der Erfassung der Atmungskurve und der maximalen Einatmung und der maximalen Ausatmung aufgrund der Atmungslänge und der Messmethode es zu einem zeitlichen Versatz der erfassten Zeitpunkte der maximalen Einatmungen und der maximalen Ausatmungen kommen kann. So kann die Atmungskurve bspw. aus einer EKG Kurve oder mittels eines Beschleunigungssensors bestimmt werden. Sofern das EKG verwendet wird, können die bspw. anhand der RR-Intervalle die Zeitpunkte der maximalen Einatmungen und der maximalen Ausatmungen bestimmt werden. Die RR-Intervalle sind jedoch bspw. sinusförmig und zeigen somit nicht unterschiedliche Atemlängen und insbesondere nicht den Umstand, dass eine Einatmungsphase bspw. länger oder kürzer (in der Regel kürzer) sein kann als eine Ausatmungsphase. Bei einer Messung mittels Beschleunigungssensor liegt die Problematik darin, dass bspw. nach der Ausatmungsphase eine kurze Atempause erfolgt. Gleichwohl kann ein Beschleunigungssensor lediglich Veränderungen wahrnehmen, kann somit den maximalen Zeitpunkt nur mit Versatz erfassen.

Diesen Phänomenen wird durch dem gewichteten Zeitmittelpunkt Rechnung getragen. Möglich ist es diesen durch Schätzung oder durch experimentelle Verfahren zu bestimmen, indem eine Kalibration durchgeführt wird. Hierfür wird die eigentliche Erfassung der Atmungskurve einer absoluten Messmethode der Atmungskurve gegenübergestellt und eine entsprechende Kalibration etabliert.

Es wird also beispielsweise aus den erfassten maximalen Einatmungen und maximalen Ausatmungen ein erster Phasenabschnitt bestimmt, in dem auch der erste Referenzzeitpunktliegt. Es wird zudem also beispielsweise aus den erfassten maximalen Einatmungen und maximalen Ausatmungen ein zweiter Phasenabschnitt bestimmt, in dem auch der zweite Referenzzeitpunktliegt.

Um nun einen atmungskorrigierten Blutdruckwert zu ermitteln und vorteilhaferweise auszugeben, werden zumindest mindestens zwei erste Blutdruckwerte bestimmt, die insbesondere jeweils Blutdruckwerte innerhalb eines Phasenabschnitts der Atmung bei maximaler Ausatmung und bei maximaler Einatmung darstellen.

Erfindungsgemäß wird der in den Einzelwertmessungen dem ersten Zeitpunkt des ersten Phasenabschnitts zugeordnete Blutdruckwert oder einem auf Basis des zum ersten Zeitpunkt des ersten Phasenabschnitts zugeordneten Blutdruckwertes ermittelten Blutdruckwert als der erste der mindestens zwei atmungskorrigierten ersten Blutdruckwerte bestimmt. Insbesondere handelt es sich bei dem ersten der mindestens zwei atmungskorrigierten ersten Blutdruckwerte um einen atmungskorrigierten ersten Blutdruckwert bei maximaler Einatmung. Insbesondere handelt es sich bei dem atmungskorrigierten ersten Blutdruckwert bei maximaler Einatmung um einen atmungskorrigierten ersten, systolischen Blutdruckwert bei maximaler Einatmung.

Erfindungsgemäß wird der in den Einzelwertmessungen dem zweiten Zeitpunkt des ersten Phasenabschnitts zugeordnete Blutdruckwert oder einem auf Basis des zweiten Zeitpunkts des ersten Phasenabschnitts zugeordneten Blutdruckwert ermittelten Blutdruckwert als der zweite der mindestens zwei atmungskorrigierten ersten Blutdruckwerte bestimmt. Insbesondere handelt es sich bei dem zweiten der mindestens zwei atmungskorrigierten ersten Blutdruckwerte um einen atmungskorrigierten ersten Blutdruckwert bei maximaler Ausatmung. Insbesondere handelt es sich bei dem atmungskorrigierten ersten Blutdruckwert bei maximaler Ausatmung um einen atmungskorrigierten ersten, systolischen Blutdruckwert bei maximaler Ausatmung.

Es ist auch möglich die Atmung und somit die Phasenabschnitte mit einer anderen Methode zu ermitteln, insbesondere auf Basis innerhalb des Messzeitraums zeitaufgelösten genommenen von Werten von Einzelwertmessungen eines zweiten Sensors, insbesondere einer zweiten Vorrichtung.

So kann dies beispielsweise mittels einer EKG Messung erfolgen. In diesem Falle erfolgt die Messung der Werte der Einzelwertmessung, die für die Ermittlung des Blutdruckwertes verwendet werden mit einer ersten Vorrichtung, und die Messung für die Phasenabschnitte mit einer zweiten Vorrichtung, nämlich einem EKG.

Die Bestimmung der Atmung und der Phasenabschnitte mittels EKG ist aus dem Stand der Technik bekannt. Dabei wird die Atmung aus der Veränderung des Herz-Intervalls bestimmt. Das Herz-Intervall wird dazu für jeden Herzschlag bestimmt. Grundlage dazu sind Daten die den Herzpuls abbilden. Diese werden heutzutage typischerweise mit einem EKG oder einer PPG aufgezeichnet. Aber auch eine Vielzahl andere Sensoren erlauben eine verwendbare Abbildung des Herzpulses, z.B. Temperaturmessung, Polymerdrucksensoren oder auch Ultraschallsensoren. Diese Daten werden hinsichtlich eines markanten Merkmals innerhalb der Abbildung des Herzpulses untersucht, sodass zu jedem Herzpuls ein solches Merkmal gefunden wird. Der Zeitabstand zwischen zwei aufeinanderfolgenden Zeitpunkten, an denen ein solches Merkmal gefunden wird, ist das Herz-Intervall. Wird als Datengrundlage die Daten eines EKGs verwendet, so ist das markante Merkmal typischerweise die R-Zacke und das Herz-Intervall wird RR-Intervall bezeichnet. Werden die Zeitlängen der Herz-Intervalle zu den Zeitpunkten des zugehörigen Herzpulses in einen Graphen aufgetragen, so sind lokale Minima und Maxima zu erkennen. Die Zeitpunkte der lokalen Minima zeigen Zeitpunkte der maximalen Einatmung an, Zeitpunkte der lokalen Maxima zeigen Zeitpunkte der maximalen Ausatmung an.

Sofern auch mindestens zwei atmungskorrigierte zweite Blutdruckwerte bestimmt werden sollen und ein zweiter Phasenabschnitt und ein zweiter Referenzzeitpunkt bestimmt wurde, wird der in den Einzelwertmessungen dem ersten Zeitpunkt des zweiten Phasenabschnitts zugeordnete Blutdruckwert oder einem auf Basis des zum ersten Zeitpunkt des zweiten Phasenabschnitts zugeordneten Blutdruckwertes ermittelten Blutdruckwert als der erste der mindestens zwei atmungskorrigierten zweiten Blutdruckwerte bestimmt. Insbesondere handelt es sich bei dem ersten der mindestens zwei atmungskorrigierten zweiten Blutdruckwerte um einen atmungskorrigierten zweiten Blutdruckwert bei maximaler Einatmung. Insbesondere handelt es sich bei dem atmungskorrigierten zweiten Blutdruckwert bei maximaler Einatmung um einen atmungskorrigierten zweiten, diastolischen Blutdruckwert bei maximaler Einatmung, und wird der in den Einzelwertmessungen dem zweiten Zeitpunkt des zweiten Phasenabschnitts zugeordnete Blutdruckwert oder einem auf Basis des zweiten Zeitpunkts des zweiten Phasenabschnitts zugeordneten Blutdruckwert ermittelten Blutdruckwert als der zweite der mindestens zwei atmungskorrigierten zweiten Blutdruckwerte bestimmt. Insbesondere handelt es sich bei dem zweiten der mindestens zwei atmungskorrigierten zweiten Blutdruckwerte um einen atmungskorrigierten zweiten Blutdruckwert bei maximaler Ausatmung. Insbesondere handelt es sich bei dem atmungskorrigierten zweiten Blutdruckwert bei maximaler Ausatmung um einen atmungskorrigierten zweiten, diastolischen Blutdruckwert bei maximaler Ausatmung.

Insbesondere ist es vorstellbar, dass der auf Basis des jeweiligen Zeitpunkts des jeweiligen Phasenabschnitts zugeordneten Blutdruckwert ermittelte Blutdruckwert auf Basis eine Druckbeaufschlagungskorrektur ermittelt wird. Für die Druckbeaufschlagungskorrektur werden insbesondere die aufgrund der variierenden Druckbeaufschlagung geänderten Werte der Einzelwertmessungen beispielsweise mittels einer auf der vorbestimmte Druckbeaufschlagungsänderungsrate basierenden lineare Regression oder eines Bandpassfilters um den Faktor der Druckbeaufschlagung korrigiert.

Durch das erfindungsgemäße Verfahren ist es also insbesondere möglich einen Blutdruckwert als Funktion der Atmung zu bestimmen und auszugeben. Dabei ist ein Wert bei maximaler Ausatmung bzw. maximaler Einatmung in der Regel besonders aussagekräftig, da bei diesen beiden Zuständen, die maximalen Effekte, hin zu höheren bzw. niedrigeren Werten des Blutdrucks vorliegen. Vorteilhafterweise werden zumindest der erste und der zweite atmungskorrigierte erste Blutdruckwert ausgegeben. Vorstellbar ist es jedoch auch, zumindest einen auf dem erste und der zweite atmungskorrigierte erste Blutdruckwert basierenden atmungskorrigierten ersten Blutdruckwert auszugeben. Hierfür könnten beispielsweise der erste und der zweite atmungskorrigierte erste Blutdruckwert die Basis für einen, insbesondere faktorisierten, Mittelwert darstellen.

Die erfindungsgemäßen Verfahren sollen, wenn diese alleinig verwendet werden, in erster Linie die Qualität einer Messung im Vergleich zu den heute verwendeten Verfahren beruhend auf Riva Rocci verbessern und neue Messwerte bereitstellen, die die vorbekannten Messverfahren nicht erbringen können. Die erfindungsgemäßen Verfahren können daher entweder mit einem Gerät, welches für das Verfahren optimiert ist, durchgeführt werden, oder es kann in bestehende Systeme integriert werden. Bestehende Systeme müssen Anforderungen an die Pumpleistung und Ventilsteuerung erfüllen und über eine geeignete Berechnungseinheit verfügen. Herkömmliche Oberarm- und Unterarmmanschettensysteme nach dem Stand der Technik sind in der Regel nicht, aufgrund beschränkter Hardware, geeignet das Verfahren durchzuführen, können jedoch in der Regel durch die Verwendung moderner Pumpen und Berechnungseinheiten mit vergleichsweise geringem Aufwand befähigt werden. Heutige Smartwatches, welche über eine Manschettenmessung verfügen, sind in der Regel bereits für das Verfahren befähigt.

Die Verbesserung der Qualität der ermittelten Blutdruckwerte gegenüber dem Stand der Technik beruht auf Folgendem: Werden mehrere Messungen nach dem Stand der Technik nacheinander durchgeführt, so kann beobachtet werden, dass in der Regel unterschiedliche Werte ausgegeben werden, obwohl die Manschette nicht verschoben oder anderweitig verändert wurde und auch die gleiche Messmethode bei gleichen optimalen Verhalten des Nutzers verwendet wurde. Die Gründe hierfür sind vielfältig, werden jedoch bei den vorbekannten Verfahren aufgrund der Verfahrensschritte nicht berücksichtigt und führen vielmehr zu systematischen Abweichungen:

1. Veränderung des Blutdrucks durch Veränderung des Erregungszustands des Nutzers (Weißkittelsyndrom). Bspw. ist ein erhöhter Blutdruck der ersten zu weiteren Messungen zu erwarten, wenn dem Nutzer die Messmethode unbekannt ist. Bei der ersten Messung ist der Respekt vor der Messung höher, was zu Erhöhung des Blutdrucks bei der ersten Messung im Vergleich zu den nachfolgenden Messungen führt.

2. Anpassung des Körpers an die Messung. Durch die erstmalige Belastung des Körpers durch die Manschette passen sich die Arterien an, um dem Druck von außen entgegenzuwirken.

3. Unregelmäßiger Herzschlag kann zu einer Fehlinterpretation des Blutdrucks führen.

4. Einfluss der Atmung auf den Blutdruck und die Wahl des Herzschlages zur Messung.

Nur der 1. Punkt ist eine "echte" Blutdruckveränderung. Unter echte Blutdruckveränderung wird dabei eine Veränderung des Blutdrucks aufgrund einer Änderung des Drucks des Blutes innerhalb des Herzkreislaufsystems verstanden. Die anderen Punkte (2 bis 4) sind hingegen Veränderungen aufgrund von Einflüssen, die keine Veränderung am Blutdruck ausgehend vom Herzen im Ergebnis ergeben sollten, jedoch nach dem Stand der Technik zu einer Veränderung des erfassbaren Ergebnisses führen, da beispielsweise eine Mittelung stattfindet, die unterschiedliche Situationen nach den Gründen 2 bis 4 beinhalten (bspw. unterschiedliche Atemzyklen oder bei der Mittelung beinhaltender unregelmäßiger Herzschlag).

Das hier vorgestellte Erfindung löst diese Problematik u.a. beispielsweise dadurch auf, indem nicht eine Mittelung der Herzaktionen betrachtet wird, sondern vorzugsweise jeder Herzschlag im Messzeitraum, welcher dem Zeitraum der Einzelwertmessung herkömmlicher Manschettenmessmethoden entspricht und auch die Druckveränderungsphase umfasst, an sich einbezogen wird und den Einfluss der Atmung erfasst wird.

Die Besonderheit der Erfindung liegt also unter anderem beispielsweise darin, dass neben den Werten, die den Blutdruckwerten zugeordnet werden, noch weitere Informationen einfließen, die im identischen Messzeitraum einen Effekt auf die Werte, die dem Blutdruckwert zugeordnet werden können, haben können, nämlich beispielsweise die Atmung.

Messverfahren nach dem Stand der Technik weisen auch bei unregelmäßigem Herzschlag (z.B.: Arrhythmien) Messungenauigkeiten auf. Dies liegt darin begründet, dass heutige Systeme von einem im Messzeitraum konstanten Blutdruck ausgehen. Bei Arrhythmien ist dies nicht so, hier kann jeder Herzschlag einen anderen Blutdruck haben. Gesundheitlich bedenklich ist hierbei, wenn Herzpausen zu lang werden oder wenn einzelne Herzschläge sehr hohe Blutdruckwerte aufweisen. Gerade diese vereinzelten Druckspitzen können zum Platzen einzelner Arterien führen, was zu Erkrankungen, wie Gerinnsel im Gehirn, führen können. Eine Aufzeichung mehrerer Herzschläge, wie durch das erfindungsgemäße Verfahren, erlaubt es Arrhythmien zu erkennen und diese auch zu klassifizieren. Arrhythmien sind nicht grundsätzlich als Krankheit einzustufen, eigene Untersuchungen haben gezeigt, dass bei sportlichen jungen Menschen gehäuft Arrhythmien auftreten. Andere Untersuchungen besagen, dass bis zu 11 % der Bevölkerung Arrhythmien aufweisen. Die Einordnung einer Arrhythmie berührt auf Häufigkeit und ob ein Muster und Regelmäßigkeit zu erkennen sind. Je nach Einordnung einer Arrhythmie kann das Verfahren nun den Blutdruck der einzelnen Herzschläge zu einem sinnvollen Ergebnis formulieren und/oder eine entsprechende Warnung über die Arrhythmie ausgeben.

Da insbesondere jeder Herzschlag im Messzeitraum erfasst wird, kann das hier vorgestellte Verfahren weitere Messwerte neben den eigentlichen Werten zum Blutdruck erfassen. Diese Daten umfassen Werte zur Atmung, zum Herzschlag, zur Blutdruckkurve und zu Erkrankungen. Die genaue Aufschlüsselung der Messgrößen wird in einem nachfolgenden Abschnitt genauer dargelegt.

Eine weitere Anwendung kann die hier vorgestellte Erfindung im Zusammenhang mit weiteren Messgeräten darstellen. Diese sind zum einen Geräte zur Herzpulserfassung, wie z.B. EKG oder auf Lichtwellen basierende Vorrichtung, und zum anderen Geräte, die eine kontinuierliche Blutdruckmessung ermöglichen. Mit Hilfe von Geräten zur Herzpulserfassung können weitere medizinische Diagnostika durchgeführt werden. Außerhalb von Extremsituationen, wie Vorhofflimmern, wird in der medizinischen Praxis davon ausgegangen, dass für jeden QRS Komplex im EKG eine Herzaktion folgt. Untersuchungen der Erfinder haben aber gezeigt, dass dies nicht der Fall ist. Bei Arrhythmien können z.B. verkürzte RR-Intervalle auftauchen. Hierbei ist bei einigen Probanden zu erkennen, dass der zweite QRS-Komplex eines solch verkürzen RR-Intervalls nicht oder nur wenig zu einem erkennbaren Puls führt, bei anderen Probanden mit zunächst augenscheinlich ähnlichen Symptomen ist jedoch ein Puls zu messen.

Im Zusammenhang mit Temperaturmessungen kann die Veränderung des Gefäßzustands erkannt werden. Untersuchungen der Erfindung mit zeitlich- und werthochauflösenden Temperaturmessmethoden haben gezeigt, dass z.B. bei sportlicher Aktivität Temperatursprünge zu Belastungs- und Entlastungssituationen auftreten.

Maßgeblich wird das hier vorgestellte Verfahren jedoch im Zusammenhang mit weiteren Geräten zur kontinuierlichen Blutdruckmessungen eine Anwendung finden. Diese Geräte basieren darauf, dass andere Vitaldaten erfasst werden und in einen Wert für den Blutdruck mit Hilfe einer Kalibration umgerechnet werden. Auch heute gibt es bereits Systeme, die den Blutdruck kontinuierlich messen können. Diese Systeme sind jedoch sehr ungenau und/oder benötigen eine komplizierte Interaktion mit dem Nutzer zur Kalibration. Beispielsweise ist die Ermittlung von kontinuierlichen Blutdruckwerten aus der Pulswellenlaufzeit seit vielen Jahren bekannt. Für eine Kalibration sind zwei Blutdruckmessungen notwendig, eine bei normalem Blutdruck und eine bei erhöhtem Blutdruck, die aufgrund des zu verändernden Blutdrucks bei der zu vermessenden Person zeitlich voneinander getrennt erfasst werden müssen. Bei einigen heutigen Systemen wird der erhöhte Blutdruck z.B. durch sportliche Aktivität erzeugt. Um eine "gute" Kalibration durchzuführen, wird aufgrund der beschriebenen Messungenauigkeiten und Messeinflüsse eine hohe Blutdruckveränderung benötigt. Dies stellt den Nutzer vor eine hohe Belastung und führt zu Messfehlern, da eine Messung bei oder kurz nach einer hohen körperlichen Belastung aufgrund von Bewegung und erhöhtem Herzschlag fehlerhaft ist.

Eine Kalibration ist notwendig, weil die Zuordnung von Vitaldatum zum Blutdruck für jeden Menschen unterschiedlich ist und von Faktoren, wie Gesundheitszustand, Körpergewicht und Flüssigkeitshaushalt abhängen. Somit ist eine Veränderung der Zuordnung im Laufe weniger Tage vorstellbar und eine Kalibration müsste mindestens täglich wiederholt werden. Dies zeigt, dass heutige Systeme nicht alltagstauglich oder ungenau sind.

Die hier vorgestellte Erfindung umgeht diese Problematiken. Das erfindungsgemäß Verfahren ermittelt den Blutdruck vorzugsweise für jeden Herzschlag innerhalb der Messphase, welche dem Zeitraum der Einzelwertmessung herkömmlicher Manschettenmessmethoden entspricht und auch die Druckveränderungsphase umfasst. Im Folgenden wird dieser Zeitraum auch Messzeitraum genannt. Die Erfinder haben erkannt, dass eine solche Messphase dabei einen oder mehrere Atemzüge umfasst. Die Erfinder haben daher erkannt, dass aufgrund der Atmung schon eine Blutdruckveränderung hervorgerufen wird und somit die Voraussetzung für eine Kalibration mit einem hohen und einen niedrigen Blutdruckwert bereits durch eine Messung unter normal Belastung gegeben sein könnte. Das erfindungsgemäße Verfahren ermittelt z.B. den höchsten und den niedrigsten Blutdruckwert innerhalb der Atmung, des Atemzyklus, zusammen mit den Zeitpunkten der Herzschläge aus denen diese Werte hervorgehen. Dies stellt den ersten Teil der Erfindung dar. Der andere Teil wird vom Gerät zur kontinuierlichen Blutdruckmessung bereitgestellt. Bei einem Gerät beruhend auf der Pulswellenlaufzeit ist dies die Pulswellenlaufzeit zu den gleichen Zeitpunkten. Das Ergebnis der Kalibration in diesem Fall ist ein umgekehrter linearer Zusammenhang zwischen Pulswellenlaufzeit und Blutdruck, sodass für jeden weiteren Wert der Pulswellenlaufzeit ein Wert des Blutdrucks ermittelt werden kann. Heutige derartige System zur kontinuierlichen Überwachung des Blutdrucks, wie z.B. CNAP der Firma Dräger, können derzeit lediglich eine Tendenzmessung durchführen. Mit dem Verfahren könnten diese Systeme auch absolut Werte bestimmen. Das Verfahren stellt somit auch einen Mehrwert im klinischen Bereich dar.

In einer weiteren Anwendung können mehr als ein Gerät, welche mit dem Verfahren betrieben werden, an unterschiedlichen Extremitäten verwendet werden. Durch den Vergleich der Abbildung der Pulswellen zwischen den Extremitäten, bspw. den beiden Beinen bzw. Armen, kann festgestellt werden, ob der Blutfluss in einer der Extremität, z.B. durch Arteriosklerose, gestört ist. Neben diesen Asymmetrien im Blutkreislauf, die bei einseitigen Messungen möglicherweise nicht erkannt werden würden, können weitere Parameter, wie zum Beispiel die Steifigkeit der Arterien, die Elastizität der Blutgefäße, die Herzfrequenzvariabilität und andere wichtige kardiovaskuläre Parameter gemessen werden. Weitere Parameter, wie z.B. die Pulswellenlaufzeit oder der Ort einer Gefäßerkrankung, können erfasst werden, wenn mehr als ein Gerät an einer Extremität angebracht wird oder die Position eines Gerätes variiert wird. Ähnliche Produkte diesbezüglich, welche durch die einfachere Handhabung und die Erfassung anderer Parameter (z.B. bezüglich der Atmung) übertroffen werden können, sind z.B. CardioScreen der Firma medis oder PhysioFlow der Firma Manatec Biomedica.

Im Gegensatz zu dem Stand der Technik, bei dem der Einfluss der Atmung als Störfaktor betrachtet wird und durch Mittelung zu systematischen willkürlichen Abweichungen und Fehlwerten führt, soll das erfindungsgemäße Verfahren den Einfluss der Atmung erkennen, sodass bei einer Ausgabe der Messwerte der Einfluss der Atmung dargestellt werden kann. Der Stand der Technik erlaubt es mit einer Manschette ein Messwerttuple bestehend aus je einen Wert für Systole, Diastole und Puls für eine Messung zu bestimmen. Ferner können weiter Messgrößen wie Pulsdruck und RR-Intervall abgeleitet werden, wobei diese auch nur einmalig pro Messung bestimmt werden können. Eine Messung mit einer Manschette nach dem Stand der Technik benötigt je nach Bautyp und vorliegendem Blutdruck 30-90 Sekunden, vgl. Fig. 1. Aus diesem Grund stammen die Werte für Systole und Diastole in der Regel von unterschiedlichen Herzschlägen, da die Messzeitpunkte der einzelnen Messwerte typischerweise 10-30 Sekunden auseinander liegen. Darüber hinaus führt die Blutdruckschwankung aufgrund der Atmung je nach Hersteller zu unterschiedlichen, gleichwohl systembedingten willkürlichen Abweichungen und Fehlwerten. Allen Messgeräten nach dem Stand der Technik ist gemein, dass der Einfluss der Atmung als Störung interpretiert wird und ein den Effekt der Atmung berücksichtigender Wert nicht im Messwerttuple enthalten ist.

Die Erfindung ermöglicht es durch die Erkennung bzw. Verwendung der Atmung die Messung des Blutdrucks zudem vorzugsweise zum einem genauer zu machen und zum anderen mehr Informationen über das Herzkreislaufsystem bereit zu stellen.

Vorzugsweise können zudem die folgenden Messgrößen aufgrund der Erfindung erhoben werden, insbesondere aus den Werten einer Manschettenmessung, also aus Luftdruckwerten der Manschettenmessung:

1. Messgrößen, die vergleichbar zum Stand der Technik der Manschettenmessungen sind, diese jedoch durch eine bessere Definierung des Zustands des Benutzers zum Messpunkt übertreffen:

- Diastole über die Atmung gemittelt;
- Systole über die Atmung gemittelt;
- Puls / Herzschläge pro Minute über die Atmung gemittelt;
- Erkennung des Vorhandenseins von Arrhythmien;

2. Messgrößen, die nicht durch den Stand der Technik der Manschettenmessungen gemessen werden können:

- Kennwerte der Atmung:

    • Atemfrequenz / Atemzüge pro Minute;
    • Zeit der Einatmung, bspw. der erste Zeitpunkt eines Phasenabschnitts;
    • Zeit der Ausatmung bspw. der zweite Zeitpunkt eines Phasenabschnitts;
    • Synchronität zum Herzpuls;

- kontinuierliche Werte während der Druckveränderungsphase für die Messgrößen Diastole und Systole:

    • Die Bestimmung von mehr als einen Messwert für Diastole und Systole für jeden Herzschlag parallel zur Messung, aus der gleichen Druckverlaufskurve;

- Zuordnung der einzelnen Messwerte auf die Phase der Atmung;

- Kennwerte zu den Messgrößen Diastole und Systole:

- Position innerhalb der Phase der Atmung
- Wert zu vorgegebener Position innerhalb der Phase der Atmung
- Maximal und minimal Wert innerhalb der Atmung

- Kennwerte zum Herzpuls:

- Die Bestimmung von Messwerten für den Herzpuls für jeden Herzschlag im Zeitraum, in dem die Druckverlaufskurve Variationen aufweist, ist möglich;
- Herzratenvariabilität;
- Die Ausprägung und Einstufung von Arrhythmien;
- Wert zu vorgegebener Position innerhalb der Phase der Atmung;
- Maximal und minimal Wert innerhalb der Atmung;

- Abbildung der Blutdruckverlaufskurve für einen Teil des Messzeitraums:

- Vorhandensein von Reflexionswellen;
- Die Augmentation durch Reflexionswellen;
- Die Veränderung der Augmentation durch Atmung;
- Bei Arrhythmien kann durch die Druckhöhe der von Arrhythmie betroffenen Herzschläge auf die Funktionalität der Herzklappen gefolgert werden;
- Abbildung von Herzflimmern und Vorhofflattern;
- Reaktion des Herzpulses auf Medikation.

Dabei ist es möglich, dass die Werte, die dem Blutdruckwerte zugeordnet werden können, und die Atmungen auf zwei verschiedenen Ausgangsdatensätzen basieren, die von unterschiedlichen Sensoren oder Vorrichtungen, bspw. einer ersten und einer zweiten Vorrichtung, erfasst und/oder ermittelt wurden. So kann die Messung von Einzelwerten der Werte, die dem Blutdruckwert zugeordnet werden, mittels einer Manschette, einer ersten Vorrichtung, durchgeführt werden, womit diese Einzelwerte Teil eines erstes Ausgangsdatensatz ist, und die Messung des Atemzyklus mittels eines anderen Gerätes, einer zweiten Vorrichtung, durchgeführt werden, womit die Werte des Atemzyklus Teil eines zweiten Ausgangsdatensatz ist. Es ist jedoch auch möglich, dies wird sogar bevorzugt, sofern sowohl die Werte, die dem Blutdruckwert zugeordnet werden, und die Werte für den Atemzyklus mittels derselben Vorrichtung, der ersten Vorrichtung, erhoben werden, somit nur ein Ausgangsdatensatz notwendig ist. Wesentlich für beide Ausführungsbeispiele ist jedoch, dass die Datensätze denselben Messzeitraum umfassen und möglichst dieselbe Zeitauflösung, vorzugsweise mit denselben Zeitpunkten.

Grundsätzlich kann der dem Blutdruckwert zugeordnete Wert mithilfe jeder dafür geeigneten Vorrichtung erfasst werden. Ebenso kann die Atmung und die Atemzyklen mithilfe jeder dafür geeigneten Vorrichtung erfasst bzw. ermittelt werden. Vorzugsweise wird jedoch zumindest der dem Blutdruckwert zugeordnete Wert mithilfe einer Manschette erfasst.

Vorteilhafterweise weist der erste Sensor einen Drucksensor, insbesondere einen Luftdrucksensor, oder einen Sensor zur Messung der elektrischen Spannung oder des elektrischen Widerstandes auf. Insbesondere ist die erste Vorrichtung eine, insbesondere automatisierte, Druckmanschette. Vorteilhafterweise ist der Wert der Einzelmessungen ein Druckwert, insbesondere ein Luftdruckwert oder ein auf Basis einer Messung einer, insbesondere durch den Piezoeffekt verursachten, elektrischen Spannung oder des elektrischen Widerstandes ermittelter Druckwert.

Vorteilhafterweise werden die Phasenabschnitte, insbesondere der erste Phasenabschnitt, insbesondere die ersten und zweiten Zeitpunkte der Phasenabschnitte, insbesondere der erste und der zweite Zeitpunkt des ersten Phasenabschnitt, insbesondere die Atmung und/oder die Atmungskurve, mittels eines zweiten Sensors, insbesondere einer zweiten Vorrichtung, bestimmt, wobei der zweite Sensor Teil eines EKG ist oder ein Impedanzsensor, ein Lichtwellen-basierender Sensor, insbesondere als Teil eines PPG- oder SpO2-Messgeräts, ein Mikrophon, ein Beschleunigungssensor, ein Ultraschallsensors oder ein Temperatursensors ist. In diesem Fall werden dann also insbesondere zwei Datensätze mit unterschiedlichen Vorrichtungen erfasst.

Alternativ weist vorteilhafterweise der erste Sensor einen Drucksensor, insbesondere einen Luftdrucksensor, oder einen Sensor zur Messung der elektrischen Spannung oder des elektrischen Widerstandes auf. Insbesondere ist die erste Vorrichtung eine, insbesondere automatisierte, Druckmanschette, und vorteilhafterweise werden dann die die Phasenabschnitte, insbesondere der erste Phasenabschnitt, insbesondere die ersten und zweiten Zeitpunkte der Phasenabschnitte, insbesondere der erste und der zweite Zeitpunkt des ersten Phasenabschnitt, insbesondere die Atmung und/oder die Atmungskurve aus den Werten der mit dem ersten Sensor erfassten Einzelwertmessungen bestimmt. In diesem Fall wird dann also insbesondere ein Datensätze mit demselben Sensor erfasst.

Die nachfolgende Tabelle zeigen mögliche Kombinationen von Sensoren bzw. Kombinationen von Datensätzen und deren Herkunft, um einerseits die Einzelwerte, die dem Blutdruckwert zugeordnet werden können, und andererseits die Atmung bzw. die Atemzyklen zu erfassen, wobei hier auf die bevorzugte Methode der Einzelwertmessung, die dem Blutdruckwert zugeordnet werden können, nämlich der Manschettenmessung abgestellt wird:

| Varianten | Bestimmung der Druck- werte mittels: | Bestimmung der Atmung mittels: |
|---|---|---|
| 1. | Manschette | EKG |
| 2. | Manschette | Lichtwellen basierter Sensor |
| 3. | Manschette | Manschette |
| 4. | Manschette | Beschleunigungssensor |
| 5. | Manschette | Mikrophon |
| 6. | Manschette | Ultraschall |
| 7. | Manschette | Temperatur |
| 8. | Manschette | Impedanzmessung |

Allen oben genannten Varianten gemeinsam ist, dass die Druckwerte aus einer Manschettenmessung hervorgehen, dabei kann die Manschette je nach Ausformung an verschiedenen Positionen am Körper verwendet werden, z.B. am Handgelenk oder am Oberarm. Dazu wird der Druck in der Manschette gemessen und gleichzeitig verändert. Die Ausgangswerte zur Bestimmung der Atmung beruhen auf der Analyse der Veränderungen in den jeweiligen Signalen. Die Sensoren können hierbei in einem Gerät mit der Manschette vereint sein, z.B. in Form eines Armbands, oder können, verbunden mit entsprechenden Datenschnittstellen, an anderen Orten des Körpers bezüglich der Manschette getragen werden. Die genauen Logiken bzw. Verfahrensschritte zur Bestimmung der Atmung aus den einzelnen Ausgangssignalen, sowie zur Bestimmung der Druckwerte und der Abgleich der Druckwerte mit der Atmung werden in einem späteren Abschnitt beschrieben.

Das erfindungsgemäße Verfahren ist dabei aus Sicht des Nutzers insbesondere vergleichbar zu den vorbekannten Messungen und Verfahren mit einer automatisierten Manschette. Die Erfindung kann bei Druckerhöhung und bei Druckreduzierung in der Manschette verwendet werden. Vorzuziehen ist die Verwendung bei Druckerhöhung, da dies eine kürzere Messzeit und damit geringere Belastung für den Nutzer erlaubt. Im einfachsten Falle wird die Druckveränderung, insbesondere linear, durchgeführt, typischerweise mit einer Druckveränderung zwischen 1 und 20 mmHg/s, insbesondere zwischen 1 und 10 mmHg/s, insbesondere von ca. 5 mmHg/s. Wird eine höhere Qualität an die Messergebnisse gefordert, so ist eine geringere Druckveränderungsrate von z.B. 1 mmHg/s erforderlich. Auch kann eine niedrigere Rate verwendet werden, damit Werte für Diastole und Systole über einen längeren Zeitraum kontinuierlich erfasst werden können. Hierbei handelt es sich um eine Abschätzung zwischen Belastung des Nutzers und Qualität der Messwerte. Vorzugsweise ist jedoch die Druckveränderungsrate während der Messung dynamisch anzupassen. Bei der Messung während Druckerhöhung kann vom unbelasteten Zustand aus zunächst eine hohe Druckveränderungsrate gewählt werden (z.B. 10mmHg/s) um den Kontakt von Manschette zur Haut zu finden. Danach wird mit einer geringeren Rate (z.B. 7mmHg/s) der unterste Druck gefunden bei dem bereits eine rhythmische Veränderung der Druckkurve aufgrund des Herzpulses zu erkennen ist, dies kann bei geeigneter Drucksensorwahl bereits unterhalb des Drucks der Diastole sein. Daran anschließend ist die eigentliche Messphase hier wird der Druck je nach Messziel verändert. Insbesondere umfasst diese Phasen den Messzeitraum. Ist das Ziel möglichst schnell nur einen Wert für Diastole und Systole zu erhalten, so kann eine Druckveränderung von z.B. 5mmHg/s gewählt werden. Das andere Ende der Anforderungen an die Druckveränderung ist die Abbildung der Druckverlaufskurve innerhalb der Herzpulse, hierzu ist bspw. eine Druckveränderungsrate von 1 mmHg/s vorzuziehen. Die Messung ist beendet, wenn keine Variationen aufgrund des Herzpulses oder nur noch geringe Variationen in der Druckverlaufskurve zu erkennen sind. Bei der Verwendung der Erfindung bei Druckreduzierung wird zunächst auf einen hohen Druck, mit typischerweise der Maximalkraft der Pumpe jedoch nicht mehr als 15mmHg/s, erhöht z.B. 200mmHg. Je nach technischer Ausstattung kann der Druck mit vorgegebener Druckveränderungsrate verändert werden. Technisch kann dies durch ein variables Ventil, mehrere Ventile für verschiedene Raten oder durch ein (oder mehrere) Ventile bei gleichzeitiger Verwendung der Pumpe bei geringerer Leistung erreicht werden. Typischerweise wird jedoch heutzutage nur ein festes, nicht variables Ventil verwendet, was im typischen Messbereich (40-200 mmHg) nahezu gleichmäßig den Druck reduzieren kann. Wenn die Möglichkeit der variablen Druckreduzierung vorhanden ist, wird nach dem Aufpumpen der Druck zunächst schnell (z.B. mit -7mmHg/s) abgelassen, um den Druck zu finden bei dem bereits Veränderungen in der Druckkurve aufgrund des Herzpulses zu erkennen sind, dies kann bei geeigneter Sensorwahl bereits oberhalb der Systole sein. Ist eine Pulsung jedoch schon bei dem Startdruck zu erkennen muss nach gepumpt werden. Die genaue Methode zur Findung dieses Startdrucks ist durch den Stand der Technik beschrieben und wird hier nur zusammenfassend wiedergegeben. Ist nach der ersten Druckerhöhung auch eine Pulsung zu erkennen, muss der Startdruck erneut erhöht werden, dieser Vorgang wird solange wiederholt bis entweder ein geeigneter Startdruck gefunden wurde oder bis ein Maximaldruck erreicht ist (z.B. 300mmHg). Die Findung des Startdrucks kann weiter optimiert werden, indem die Stärke der Druckvariation aufgrund des Herzpulses beim aktuellen Startpunkt bestimmt wird. Es kann aus empirischen Messungen nun eine geeignete Druckerhöhung des Startdruckpunktes bestimmt werden. Ist der Anfangsdruck der Druckpulsung oberhalb der Systole gefunden, wird die Druckreduzierungsrate auf einen (absolut gesehen) niedrigeren Wert reduziert, je nach Messanforderung, siehe oben bei Druckerhöhung. Ist keine mehr oder nur geringe Pulsung zu erkennen (der Druck befindet sich nun unterhalb der Diastole) kann der Druck mit der maximalen Geschwindigkeit abgelassen werden.

Gleichzeitig, also ebenso innerhalb des Messzeitraums, zur Druckmessung, also der Einzelwertmessungen, wird die Messung der Ausgangswerte zur Bestimmung der Atmung durchgeführt. Werden andere Verfahren zur Atmungsbestimmung verwendet, so können diese entweder in der Manschette selbst integriert sein, sodass eine optimale Positionierung mit dem Anbringen der Manschette erreicht ist. Bei externen Geräten werden diese nach den jeweiligen Gerätevorgaben angebracht. Beiden Gerätetypen ist gemein, dass die einzelnen Sensoren auch dynamisch und automatisiert reguliert werden können.

Hierzu sind die in der obigen Tabelle vorgestellten Variationen vorstellbar, wobei auch noch andere Kombinationen vorstellbar sind.

EKG (Variante 1) bzw. Impedanz (Variante 8) benötigt keine weitere Regulierung. Bei auf Lichtwellen basierenden Varianten (Variante 2), diese umfassen Ausprägungen wie PPG oder SpO2 Messgeräte, kann durch die Regulierung des elektrischen Stroms für die Lichterzeugung in der Helligkeit reguliert werden. Wenn die Lichtwellen-Messung in die Manschette integriert ist, so wird das Signal mit zunehmender Druckbeaufschlagung schwächer und kann dann auch keinen Herzpuls detektieren, wenn der Blutfluss zum Erliegen gekommen ist. Es ist daher sinnvoll die Helligkeit bei höheren Drücken zu erhöhen, um ein gutes Ausgangssignal zu erhalten. Dabei sollte die Helligkeit nicht zu stark oder unstetig verändert werden. Beruht die Erkennung der Atmung alleinig auf der Veränderung des RR-Intervalls ist eine stärkere Veränderung (z.B. von 0.5mA/s bei typischen grünen LEDs) möglich. Beruht die Erkennung der Atmung auch auf der Beurteilung der Pulshöhen, so darf die Veränderung der Lichtstärke nicht derart hoch ausfallen (z.B. 0.1 mA/s). Manschette (Variante 3) erlaubt keine weitere Regulierung, da diese Bereits zur Druckverlaufsmessung verwendet wird. Beschleunigungssensor (Variante 4) bedarf keiner weiteren Regulierung. Mikrophone (Variante 5) kann durch die Regulierung der Empfindlichkeit verändert werden. Wird das Mikrophone unterhalb der Manschette verwendet, so verhält es sich analog zur Regulierung bei der Verwendung von Lichtwellen. Ultraschall (Variante 6) kann bezüglich Ausgangsleistung und Empfangsempfindlichkeit reguliert werden. Auch hier verhält es sich analog zur Regulierung bei der Verwendung von Lichtwellen.

Für die Methoden der Varianten 1, 2, 5, 6, 7 und 8 zur Erkennung der Atmung werden typischerweise drei grundlegende Daten verwendet werden: 1. Veränderung des Herzpulsintervalls, 2. Veränderung der maximalen oder minimalen Messwerte innerhalb des Herzpulses, 3. Anpassung von Parametern, die die Sensorik anpassen. Für alle Methoden (außer 3) kann auch eine mit einem low-pass-Filter gefilterte Messwertkurve verwendet werden. Dabei ist die Filterung so gewählt, dass die Atmung sich aus lokalen Minima und Maxima der Messwertkurve ergibt.

Vorteilhafterweise wird aus den Werten der Einzelwertmessungen mittels einer Regressionsgeraden oder mittels eines Bandpassfilter zur Korrektur der variierenden Druckbeaufschlagung eine druckbeaufschlagungskorrigierte Intensitätskurve bestimmt. Dieses Verfahrens ist teils auch aus dem Stand der Technik bekannt und wird derart verwendet.

Vorteilhafterweise werden Änderungen der Werte der Einzelwertmessungen bestimmt. Diese Änderungen der Werte sind dabei im zeitlichen Verlauf vorliegenden Änderungen innerhalb des Messzeitraums. Diese Änderungen werden beispielsweise aus einer mathematischen Ableitung der Werte der Einzelwertmessungen ermittelt, einer Ableitung über die Zeit, also als Funktion der Zeit. Alternativ, jedoch zumindest, zum jetzigen Zeitpunkt aufgrund von Ungenauigkeiten weniger bevorzugt, können die Änderungen auch mittels einer, insbesondere Bandpass-, Filterung oder einem Abzug einer Ausgleichsgeraden bestimmt werden.

Aus den vorgenannten Änderungen wird dann eine Änderungsverlaufskurve bestimmt und aus dieser können dann die Phasenabschnitte, insbesondere der erste Phasenabschnitt, und jeweils der erste und zweite Zeitpunkt der Phasenabschnitte bestimmt werden. Insbesondere können aus der Änderungsverlaufskurve jeweils der Beginn und das Ende des Phasenabschnitts und/oder der Zeitraum des jeweiligen Phasenabschnitts bestimmt werden.

Vorteilhafterweise werden für die Bestimmung der Phasenabschnitte aus dem vorgenannten Änderungsverlaufskurve mehrere lokale Extrema, nämlich lokale Maxima und lokale Minima, insbesondere in der Änderungsverlaufskurve, ermittelt.

Vorteilhafterweise werden für die Bestimmung der Phasenabschnitte eine erste und eine zweite Ausgleichskurve aus den lokalen Extrema ermittelt. Dabei wird die erste Ausgleichskurve als eine Ausgleichskurve gebildet, die auf den Punkten der lokalen Maxima, insbesondere der, insbesondere aller, im zeitlichen Verlauf benachbarten lokalen

**EP 4 520 260 A1**

Maxima, basiert. Ferner wird die zweite Ausgleichskurve als eine Ausgleichskurve gebildet, die auf den Punkten der lokalen Minima, insbesondere der, insbesondere aller, im zeitlichen Verlauf benachbarten lokalen Minima, basiert. Vorteilhafterweise bilden die erste Ausgleichskurve und die zweite Ausgleichskurve eine Einhüllende um die lokalen Extrema, also die lokalen Minima und lokalen Maxima. Vorteilhafterweise werden mehrere, bspw. alle während des Messzeitraums erfassten, lokale maximale Abstände und mehrere, bspw. alle während des Messzeitraums erfassten, lokale minimale Abstände zwischen der ersten Ausgleichskurve und zweiten Ausgleichskurve bestimmt. Insbesondere wird ein Abstand zu mindestens einem Zeitpunkt, insbesondere zu jedem Zeitpunkt, innerhalb des Messzeitraums durch den Differenzwert einer Einhüllenden der lokalen Maxima und dem Wert der Einhüllenden der lokalen Minima gebildet.

Vorteilhafterweise werden die Phasenabschnitte, insbesondere deren Zeitraum, insbesondere dessen Beginn und Ende, durch den Zeitpunkt zweier im zeitlichen Verlauf aufeinanderfolgender lokaler minimaler oder maximaler Abstände bestimmt. Dabei wird vorteilhafterweise als maximale Einatmung des ersten Phasenabschnitts der lokale maximale Abstand innerhalb des ersten Phasenabschnitts bestimmt. Ferner wird vorteilhafterweise der Zeitpunkt dieses lokalen maximalen Abstands als erster Zeitpunkt bestimmt. Vorteilhafterweise wird als maximale Ausatmung des ersten Phasenabschnitts der lokale minimale Abstand innerhalb des ersten Phasenabschnitts bestimmt. Dabei wird vorteilhafterweise der Zeitpunkt dieses lokalen minimalen Abstands als zweiter Zeitpunkt bestimmt.

Alternativ oder zusätzlich wird für die Bestimmung der Phasenabschnitte aus dem zeitlichen Abstand zwischen zwei im zeitlichen Verlauf benachbarte lokaler Extrema jeweils ein Zeitabstand zwischen den zwei im zeitlichen Verlauf benachbarten lokalen Extrema bestimmt, wobei je ein Phasenabschnitt, insbesondere dessen Beginn und Ende, durch zwei im zeitlichen Verlauf aufeinanderfolgender lokale größte Zeitabstände der Zeitabstände bestimmt wird, und als maximale Einatmung des jeweiligen Phasenabschnitts der lokale größte Zeitabstand innerhalb des jeweiligen Phasenabschnitts und der Zeitpunkt des lokalen größten Zeitabstands innerhalb des Phasenabschnitts als erster Zeitpunkt und als maximale Ausatmung des jeweiligen Phasenabschnitts ein lokaler kleinster Zeitabstand der Zeitabstände innerhalb des jeweiligen Phasenabschnitts und der Zeitpunkt des lokalen kleinsten Zeitabstands innerhalb des Phasenabschnitts als zweiter Zeitpunkt bestimmt werden.

Vorteilhafterweise werden (wie auch weiter oben schon angedeutet) eine erste Vielzahl der mindestens zwei atmungskorrigierten ersten Blutdruckwerte der Person als kontinuierliche erste Blutdruckwerte ermittelt. Dabei können weiter vorzugsweise auch eine dritte Vielzahl von Phasenabschnitten ermittelt. Vorzugsweise kann dann jeder Blutdruckwert der ersten Vielzahl je einem Herzzyklus der Person zugeordnet werden. Insbesondere kann jeder Phasenabschnitt der dritten Vielzahl von Phasenabschnitten mindestens zwei der mindestens zwei atmungskorrigierten ersten Blutdruckwert der ersten Vielzahl aufweisen.

Vorteilhafterweise werden - bei der kontinuierlichen Blutdruckwertbestimmung - Änderungen, insbesondere Steigungen, der Werte der Einzelwertmessungen ermittelt. Diese Änderungen werden dabei aus den, insbesondere die variierende Druckbeaufschlagung aufweisenden, Werten der Einzelwertmessungen und sind dabei Änderungen der Werte der Einzelwertmessungen die von der der variierenden Druckbeaufschlagung unabhängig sind, also beispielsweise den linearen Anstieg der Druckbeaufschlagung nicht beinhalten. Derartige Änderungswerte können insbesondere mittels einer mathematischen nach der Zeit durchgeführte Ableitung der Werte der Einzelwertmessungen ermittelt werden. Aus diesen Änderungswerten kann eine Intensitätskurve ermittelt werden. Diese Intensitätskurve kann dabei identisch zu der oben genannten Änderungsverlaufskurve sein.

Diese Intensitätskurve weist dann mehrere lokale Extrema auf. Insbesondere weist die Intensitätskurve mehrere, insbesondere mindestens zwei, insbesondere mindestens drei, insbesondere mindestens sechs, lokale Minima und mehrere, insbesondere mindestens zwei, insbesondere mindestens drei, insbesondere mindestens sechs, lokale Maxima auf. Unter einem lokalen Minimum und unter einem lokalen Maximum ist insbesondere ein mathematisches lokales Minimum bzw. Maximum zu verstehen. Insbesondere weist ein lokales Maximum einen Einzelwert der Änderungen auf, der im Vergleich zu unmittelbar vorhergehend und nachfolgenden Einzelwerten der größte Einzelwert der Änderungen ist. Insbesondere weist ein lokales Minimum einen Einzelwert der Änderungen auf, der im Vergleich zu unmittelbar vorhergehend und nachfolgenden Einzelwerten der kleinste Einzelwert der Änderungen ist.

Die Intensitätskurve unterscheidet sich dabei von der druckbeaufschlagungskorrigierten Intensitätskurve dadurch, dass bei der Intensitätskurve auf die Änderungen in der Steigung einer Kurve aus den Werten der Einzelwertmessungen betrachtet werden, also im einfachsten Fall die mathematische Ableitung der Kurve betrachtet wird, und bei der druckbeaufschlagungskorrigierten Intensitätskurve die Absolutwerte abzüglich der Druckbeaufschlagung betrachtet wird.

Vorteilhafterweise werden zur Ermittlung der druckbeaufschlagungskorrigierten Intensitätskurve aus den die variierende Druckbeaufschlagung aufweisenden Werte der Einzelwertmessungen Änderungen, insbesondere Steigungen, der Einzelwertmessungen ermittelt, die unabhängig von der variierenden Druckbeaufschlagung sind. Diese Ermittlung kann beispielsweise mittels eines, insbesondere digitalen, Bandpassfilters erfolgen, oder auf Basis einer Regressionsgerade oder einer Ableitung des Verlaufs der Einzelwertmessungen als Funktion der Zeit, insbesondere einer ersten mathematischen Ableitung. Aus den so ermittelten Änderungen wird erfindungsgemäß ein Verlauf der Einzelwerte der Änderungen gebildet und als eine druckbeaufschlagungskorrigierten Intensitätskurve ermittelt. Diese druckbeaufschlagungskorrigierten Intensitätskurve weist dann mehrere lokale Extrema auf. Insbesondere weist die Intensitätskurve mehrere, insbesondere mindestens zwei, insbesondere mindestens drei, insbesondere mindestens sechs, lokale Minima und mehrere, insbesondere mindestens zwei, insbesondere mindestens drei, insbesondere mindestens sechs, lokale Maxima auf. Unter einem lokalen Minimum und unter einem lokalen Maximum ist insbesondere ein mathematisches lokales Minimum bzw. Maximum zu verstehen. Insbesondere weist ein lokales Maximum einen Einzelwert der Änderungen auf, der im Vergleich zu unmittelbar vorhergehend und nachfolgenden Einzelwerten der größte Einzelwert der Änderungen ist. Insbesondere weist ein lokales Minimum einen Einzelwert der Änderungen auf, der im Vergleich zu unmittelbar vorhergehend und nachfolgenden Einzelwerten der kleinste Einzelwert der Änderungen ist.

Sofern eine erste Vorrichtung, insbesondere Manschette verwendet wird, die eine schmale Druckbeaufschlagungsfläche, insbesondere Manschettenbreite, aufweist, insbesondere eine Manschettenbreite von weniger als zehn Zentimeter, insbesondere weniger als sechs Zentimeter, insbesondere weniger als fünf Zentimeter, insbesondere maximal vier Zentimeter, insbesondere drei Zentimeter, und/oder von mindestens 0,1 mm, insbesondere mindestens 1 mm, insbesondere mindestens 1 cm, kann erfindungsgemäß vorgesehen sein, dass basierend auf der Intensitätskurve eine vorrichtungsbreitenkorrigierte, insbesondere manschettenbreitekorrigierte, Intensitätskurve aufweisend die mehreren lokale Extrema ermittelt wird. Bei den lokalen Extrema handelt es sich in der Regel um lokale Maxima und lokale Minima, wobei die Extrema dann zur Bildung der Näherungsgeraden verwendet werden.

Sofern als erste Vorrichtung eine Manschette verwendet wird, stellt die vorrichtungsbreitenkorrigierte Intensitätskurve eine manschettenbreitenkorrigierte Intensitätskurve dar.

Die Bestimmung der vorrichtungsbreitenkorrigierten Intensitätskurve basiert dabei auf den Werten der Einzelwertmessungen, diese stellen insbesondere den Ausgangspunkt dar. Für die Bestimmung der vorrichtungsbreitenkorrigierten Intensitätskurve kann daher einerseits die auf den Werten der Einzelwertmessungen basierende Intensitätskurve verwendet werden, die insbesondere die von der variierenden Druckbeaufschlagung unabhängige Änderungen aufweist. Gleichwohl können auch andere Werte, die auf den Werten der Einzelwertmessungen basieren, verwendet werden. Insbesondere wird daher anderseits für die Bestimmung der vorrichtungsbreitenkorrigierten Intensitätskurve zunächst eine Intensitätskurve dadurch bestimmt, dass diese Intensitätskurve die erste mathematische Ableitung der als extrapolierten Kurve dargestellten Werte der Einzelwertmessungen darstellt und somit die Intensitätskurve mittels der ersten mathematischen Ableitung der Werte der Einzelwertmessungen als Funktion der Zeit ermittelt wird. Die so ermittelte Intensitätskurve weist mehrere lokale Extrema, nämlich lokale Maxima und lokale Minima auf.

Vorteilhafterweise werden zur Ermittlung der vorrichtungsbreitenkorrigierte, insbesondere manschettenbreitekorrigierte, Intensitätskurve eine erste, zweite und dritte untere Annäherungsgerade und eine erste, zweite und dritte obere Annäherungsgerade gebildet.

Vorteilhafterweise werden zur Bildung der vorrichtungsbreitenkorrigierte, insbesondere manschettenbreitekorrigierte, Intensitätskurve Einzelwerte der vorrichtungsbreitenkorrigierte Intensitätskurve durch Multiplikation der Einzelwerte der Intensitätskurve mit einem zeitabhängigen Breitenkorrekturfaktor gebildet, wobei der Breitenkorrekturfaktor insbesondere auf einer normierten Differenz basiert, wobei die Differenz eine Differenz zwischen den zu den jeweiligen Zeitpunkten vorhandenen Einzelwerten der ersten, zweiten und dritten unteren Annäherungsgerade und den zu den jeweiligen Zeitpunkten vorliegenden Einzelwerten der ersten, zweiten und dritten oberen Annäherungsgerade ist und um einen Normierungsparameter normiert wird.

Vorteilhafterweise wird die erste obere Annäherungsgerade zu den in einer ersten Zeitspanne vorliegenden lokalen Maxima der Intensitätskurve gebildet. Vorteilhafterweise wird die zweite obere Annäherungsgerade zu den in einer zweiten Zeitspanne vorliegenden lokalen Maxima der Intensitätskurve gebildet. Vorteilhafterweise wird die dritte obere Annäherungsgerade zu den in einer dritten Zeitspanne vorliegenden lokalen Maxima der Intensitätskurve gebildet. Vorteilhafterweise wird die erste untere Annäherungsgerade zu den in einer vierten Zeitspanne vorliegenden lokalen Minima der Intensitätskurve gebildet. Vorteilhafterweise wird die zweite untere Annäherungsgerade zu den in einer fünften Zeitspanne vorliegenden lokalen Minima der Intensitätskurve gebildet. Vorteilhafterweise wird die dritte untere Annäherungsgerade zu den in einer sechsten Zeitspanne vorliegenden lokalen Minima der Intensitätskurve gebildet. Die unteren Annäherungsgeraden werden also den in unterschiedlichen Zeitspannen vorliegenden lokalen Minima der Intensitätskurve zugeordnet und die oberen Annäherungsgeraden werden also den in unterschiedlichen Zeitspannen vorliegenden lokalen Maxima der Intensitätskurve zugeordnet. Dabei liegen der Beginn und das Ende der ersten, zweiten, dritten, vierten, fünften und sechsten Zeitspanne innerhalb des Messzeitraums.

Dabei liegt der Beginn der ersten Zeitspanne vor dem Beginn und Ende der zweiten Zeitspanne und der dritten Zeitspanne und ferner liegt der Beginn der vierten Zeitspanne vor dem Beginn und Ende der fünften Zeitspanne und sechsten Zeitspanne.

Ferner liegt dabei das Ende der dritten Zeitspanne nach dem Beginn und Ende der ersten Zeitspanne und zweiten Zeitspanne und ferner liegt das Ende der sechsten Zeitspanne nach dem Beginn und Ende der vierten und fünften Zeitspanne.

Vorteilhafterweise stimmt der Beginn der ersten Zeitspanne mit dem Beginn der vierten Zeitspanne überein. Vorteilhafterweise stimmt das Ende der dritten Zeitspanne mit dem Ende der sechsten Zeitspanne überein.

Ferner entspricht dabei vorzugsweise der Beginn der zweiten Zeitspanne dem Ende der ersten Zeitspanne und ferner entsprecht das Ende der zweiten Zeitspanne dem Beginn der dritten Zeitspanne und zudem entspricht der Beginn der fünften Zeitspanne dem Ende der vierten Zeitspanne und zudem entspricht das Ende der fünften Zeitspanne dem Beginn der sechsten Zeitspanne.

Ferner wird dabei das Ende der ersten Zeitspanne und der Beginn der dritten Zeitspanne so gewählt, dass die zweite obere Annäherungsgerade die minimalste Abweichung von den innerhalb der zweiten Zeitspanne vorliegenden lokalen Maxima aufweist, und es werden das Ende der vierten Zeitspanne und der Beginn der sechsten Zeitspanne so gewählt, dass die zweite untere Annäherungsgerade die minimalste Abweichung von den innerhalb der fünften Zeitspanne vorliegenden lokalen Minima aufweist.

Vorteilhafterweise wird die zweite obere Näherungsgerade zu den in der dritten und/oder zweiten Zeitspanne vorliegenden lokalen Maxima der lokalen Extrema der vorrichtungsbreitenkorrigierten Intensitätskurve gebildet.

Vorteilhafterweise wird die erste obere Näherungsgerade zu den in der ersten Zeitspanne vorliegenden lokalen Maxima der lokalen Extrema der vorrichtungsbreitenkorrigierten Intensitätskurve gebildet.

Vorteilhafterweise wird die zweite untere Näherungsgerade zu den in der sechsten und/oder fünften Zeitspanne vorliegenden lokalen Minima der lokalen Extrema der vorrichtungsbreitenkorrigierten Intensitätskurve gebildet.

Vorteilhafterweise wird die erste untere Näherungsgerade zu den in der vierten Zeitspanne vorliegenden lokalen Minima der lokalen Extrema der vorrichtungsbreitenkorrigierten Intensitätskurve gebildet.

Vorteilhafterweise basiert der Normierungsparameter auf der zum Beginn der zweiten Zeitspanne vorliegenden Differenz zwischen den unteren und oberen Annäherungsgeraden und der zum Beginn der fünften Zeitspanne vorliegenden Differenz zwischen den unteren und oberen Annäherungsgeraden. Vorteilhafterweise weist die, insbesondere vorrichtungsbreitenkorrigierte, Intensitätskurve mindestens sechs lokale Maxima mit jeweils einem im Vergleich zu unmittelbar vorhergehend und nachfolgenden Einzelwerten größten Einzelwert der , insbesondere vorrichtungsbreitenkorrigierte, Intensitätskurve und mindestens sechs lokale Minima mit jeweils einem im Vergleich zu unmittelbar vorhergehend und nachfolgenden Einzelwerten größten Einzelwert der vorrichtungsbreitenkorrigierten Intensitätskurve auf.

Sofern die erste Vorrichtung eine ausreichend breite Druckbeaufschlagungsfläche bereitstellt kann mit den Werten der Intensitätskurve der nachfolgende Verfahrensschritt durchgeführt werden, andernfalls sollten die Werte der vorrichtungsbreitenkorrigierte Intensitätskurve, und nicht der nicht vorrichtungsbreitenkorrigierte Intensitätskurve, bei dem nachfolgenden Verfahrensschritt verwendet werden.

Erfindungsgemäß wird in einem weiteren Verfahrensschritt mindestens eine, vorzugsweise mindestens zwei, obere Näherungsgerade und mindestens eine, vorzugsweise mindestens zwei, untere Näherungsgerade gebildet. Die mindestens eine obere Näherungsgerade wird dabei als Näherungsgerade zu den in dem Messzeitraum vorliegenden Maxima der lokalen Extrema der, insbesondere vorrichtungsbreitenkorrigierten, Intensitätskurve gebildet. Insbesondere basiert die Bildung der mindestens einen oberen Näherungsgerade auf einer, insbesondere linearen, Regressionsgeraden. Die mindestens eine untere Näherungsgerade wird dabei als eine Näherungsgerade zu den in dem Messzeitraum vorliegenden Minima der lokalen Extrema der, insbesondere vorrichtungsbreitenkorrigierten, Intensitätskurve gebildet. Insbesondere basiert die Bildung der mindestens einen unteren Näherungsgeraden auf einer, insbesondere linearen, Regressionsgeraden. Vorzugsweise werden mindestens zwei obere und mindestens zwei untere Näherungsgeraden gebildet. Dabei umfasst die erste obere Näherungsgerade lokale Maxima, die zu einem früheren Zeitpunkt und/oder zu niedrigeren Drücken in den den Blutdruckwerten zugeordneten Werten der Einzelmessungen und/oder bei niedrigeren Druckbeaufschlagung vorliegen als die lokalen Maxima, die der zweiten oberen Näherungsgeraden zu Grunde gelegt werden. Ferner umfasst die erste untere Näherungsgerade lokale Minima, die zu einem früheren Zeitpunkt und/oder zu niedrigeren Drücken in den den Blutdruckwerten zugeordneten Werten der Einzelmessungen und/oder bei niedrigeren Druckbeaufschlagung vorliegen als die lokalen Minima, die der zweiten unteren Näherungsgeraden zu Grunde gelegt werden.

In einem weiteren Verfahrensschritt werden zur Ermittlung der ersten Vielzahl der mindestens zwei atmungskorrigierten ersten Blutdruckwerte, also den kontinuierlichen atmungskorrigierten ersten Blutdruckwerten, erfindungsgemäß die Einzelwerte der, insbesondere vorrichtungsbreitenkorrigierten, Intensitätskurve normiert und skaliert. Die Normierung basiert insbesondere auf einer zu den jeweiligen Zeitpunkten der Einzelwerte vorliegenden Differenz zwischen der oberen Näherungsgerade und unteren Näherungsgerade. Insbesondere basiert die Normierung also auf einer Funktion der Zeit, die sich aus der Differenz zwischen der oberen Näherungsgerade und unteren Näherungsgerade als Funktion der Zeit bildet.

Die Skalierung basiert insbesondere auf einer Skalierungskonstante, wobei die Skalierungskonstante insbesondere auf dem Unterschied zwischen dem zum ersten und zweiten Zeitpunkt des ersten Phasenabschnitts vorliegenden normierten Wert der, insbesondere vorrichtungsbreitenkorrigierten, Intensitätskurve sowie dem ersten und zweiten der mindestens zwei atmungskorrigieren ersten Blutdruckwerte basiert. Es werden also insbesondere die normierten Einzelwerte derart skaliert, dass der so skalierte normierte zum ersten Zeitpunkt vorliegende Einzelwert dem zuvor bei der Einzelwertbestimmung zum ersten Zeitpunkt bestimmte systolische Blutdruckwert entspricht. Durch die Skalierung ist es möglich, aus den normierten Einzelwerten, die einer Tendenzmessung entsprechen, Druckwerte zu bestimmen, die insbesondere dem Blutdruckwert der Person zugeordnet werden können.

Hierfür kann beispielsweise die Skalierungskonstante nicht nur auf dem eben genannten Unterschied basieren, sondern auch noch auf einem Mittelwert, gebildet aus den mindestens zwei atmungskorrigierten ersten Blutdruckwerten und den mindestens zwei atmungskorrigierten zweiten Blutdruckwerten, basieren, wobei der atmungskorrigierte erste Blutdruckwert einem systolischen Blutdruckwert und der atmungskorrigierte zweite Blutdruckwerte einem diastolischen Blutdruckwert entspricht. Die Skalierungskonstante umfasst somit beispielsweise die Differenz aus den normierten Werten, die Differenz aus den atmungskorrigierten ersten Blutdruckwerten und dem Mittelwert aus den atmungskorrigierten ersten und zweiten Blutdruckwerten, insbesondere in der Form: (1 / ( erster atmungskorrigierter erster Blutdruckwert *minus* zweiter atmungskorrigierter erster Blutdruckwert)) * ( normierter Einzelwert zum ersten Zeitpunkt *minus* normierter Einzelwert zum zweiten Zeitpunkt ) * 0,25 * ( erster atmungskorrigierter erster Blutdruckwert + zweiter atmungskorrigierter erster Blutdruckwert + erster atmungskorrigierter zweiter Blutdruckwert + zweiter atmungskorrigierter zweiter Blutdruckwert ). Statt einer gleichmäßigen Mittelwertbildung (0,25) kann auch eine andere Wichtung verwendet werden.

Aus diesen normierten und skalierten Einzelwerten ist eine Blutdruckverlaufskurve bestimmbar und wird erfindungsgemäß bestimmt, in der lokale Maxima und lokale Minima vorhanden sind. Erfindungsgemäß werden aus den den lokalen Maxima oder Minima der Blutdruckverlaufskurve zugeordneten oder zuordenbaren Einzelwerten Blutdruckverlaufskurve die erste Vielzahl der atmungskorrigierten ersten Blutdruckwerte, bestimmt.

Vorteilhafterweise werden (wie oben bereits angedeutet) neben den mindestens zwei atmungskorrigierten ersten Blutdruckwerten auch mindestens zwei atmungskorrigierten zweite Blutdruckwerte ermittelt. Möglich ist auch die Ermittlung atmungskorrigierter weiterer Blutdruckwerte. In Bezug zu Blutdruckwerten hat sich jedoch in den Fachkreisen durchgesetzt, für den Blutdruckwert die Kennwerte des systolischen und des diastolischen Blutdruckwerts anzugeben. Daher genügt es in der Regel zwei Arten atmungskorrigierter Blutdruckwerte zu ermitteln, nämlich atmungskorrigierte erste Blutdruckwerte und atmungskorrigierte zweite Blutdruckwerte, wobei dann die ersten Blutdruckwerte systolische Blutdruckwerte und die zweiten Blutdruckwerte diastolische Blutdruckwerte sind. Demgemäß, sofern die vorteilhafte Ermittlung der mindestens zwei atmungskorrigierten zweite Blutdruckwerte ausgeführt werden soll, wird in dem erfindungsgemäßen Verfahren hinsichtlich der zweiten Blutdruckwerte, der diastolischen Blutdruckwerte in analoger Weise wie bei den ersten Blutdruckwerten, den systolischen Blutdruckwerten vorgegangen.

Hierfür wird insbesondere wie folgt vorgegangen:
Aus den Einzelwertmessungen wird ein nicht-atmungskorrigierter zweiter Blutdruckwert der Person bestimmt, wobei als ein zweiter Referenzzeitpunkt der Zeitpunkt des nicht-atmungskorrigierten zweiten Blutdruckwerts innerhalb des Messzeitraums bestimmt wird.

Ferner wird ein zweiter Phasenabschnitt der Atmung aufweisend einen Minimalwert, insbesondere eine maximale Einatmung, in der Atmungskurve zu einem ersten Zeitpunkt des zweiten Phasenabschnitts und einen Maximalwert, insbesondere eine maximale Ausatmung, in der Atmungskurve zu einem zweiten Zeitpunkt des zweiten Phasenabschnitts innerhalb des Messzeitraums zeitaufgelöst erfasst, wobei der zweite Phasenabschnitt so gewählt wird, dass der zweite Referenzzeitpunkt innerhalb des den zweiten Phasenabschnitt umfassenden Zeitraums fällt.

Außerdem werden der in den Einzelwertmessungen dem ersten Zeitpunkt des zweiten Phasenabschnitts zugeordnete Blutdruckwert oder einem zum ersten Zeitpunkt des zweiten Phasenabschnitts ermittelten Blutdruckwert als der erste der mindestens zwei atmungskorrigiertem zweiten Blutdruckwerte, insbesondere der maximalen Einatmung, und der in den Einzelwertmessungen dem zweiten Zeitpunkt des zweiten Phasenabschnitts zugeordnete Blutdruckwert oder einem zum zweiten Zeitpunkt des zweiten Phasenabschnitts ermittelten Blutdruckwert als der zweite der mindestens zwei atmungskorrigierten zweiten Blutdruckwert, insbesondere der maximalen Ausatmung, bestimmt.

Möglich ist es, sofern die vorteilhafte Ermittlung der mindestens zwei atmungskorrigierten zweite Blutdruckwerte ausgeführt, dann vorteilhafterweise auch neben der ersten Vielzahl von den mindestens zwei atmungskorrigierten ersten Blutdruckwerten eine zweite Vielzahl von den mindestens zwei atmungskorrigieren zweiten Blutdruckwerten zu ermitteln. Auch kann in dem erfindungsgemäßen Verfahren hinsichtlich der zweiten Vielzahl in analoger Weise wie bei der Ermittlung der ersten Vielzahl vorgegangen werden.

Hierfür wird insbesondere wie folgt vorgegangen:
Aus den den lokalen Maxima der Blutdruckverlaufskurve zugeordneten Blutdruckwerten werden atmungskorrigierte, kontinuierliche, erste, systolische Blutdruckwerte der ersten Vielzahl der mindestens zwei atmungskorrigierten ersten systolischen Blutdruckwerte und aus den den lokalen Minima der Blutdruckverlaufskurve zugeordneten Blutdruckwerten werden atmungskorrigierte, kontinuierliche, zweite, diastolische Blutdruckwerte der zweiten Vielzahl der mindestens zwei atmungskorrigierten zweiten diastolischen Blutdruckwerte bestimmt.

Sofern eine Oberarmmanschette verwendet wird, weist diese üblicherweise eine Breite von ca. 1/3 der Länge zwischen Schultergelenk und Ellenbogengelenk auf, da dies als ideal angesehen wird. Die Erfinder haben erkannt, dass bei Verwendung einer breiteren Manschette ein niedrigerer Blutdruck bestimmt wird und dass bei einer Verwendung einer zu schmalen Manschette, sich zu hohe Werte ergeben. Der Grund hierfür sind die Ursachen für die Variationen in der Luftdruckkurve aufgrund des Herzschlages, diese Ursachen sind:

1. Diastolischer Druck verstärkt den Gewebedruck nicht ausreichend, um dem Luftdruck der Manschette entgegenzuwirken, eine Variation zwischen den Herzpulsen erscheint.

2. Der systolische Druck erhöht den Luftdruck in der Manschette durch Volumenreduzierung der Manschette, eine Variation zu den Herzpulsen erfolgt.

Dabei überlagern sich diese Ursachen je nach Luftdruck in der Manschette; die Ursache 1 tritt bei geringeren Drücken auf und die Ursache 2 bei höheren Drücken. Als weiterer, dritter Wirkmechanismus wirkt das Kollabieren der Arterien, wodurch bei weiter höher werdenden Druck der Blutfluss eingeschränkt und bei noch höherem Druck ab einem bestimmten Druck ganz erliegt. Die Größe der Manschette beeinflusst wie stark die vorgenannten Ursachen auftreten und/oder auftauchen. Eine breite Manschette, wie nach dem Stand der Technik (ca. 1/3 der Länge zwischen Schultergelenk und Ellenbogengelenk), unterdrückt die Ursache 2. Eine zu breite Manschette, also noch breiter als die oben genannten breite Manschette, führt aber auch dazu, dass eine größere Hautoberfläche abgedeckt ist und somit ein größeres Gewebevolumen abgedrückt wird, der Blutfluss ist bereits bei geringeren Drücken beeinflusst, sodass die Ursache 1 bereits bei geringeren Drücken zum Tragen kommt; in einem solchen Fall wird ein zu geringer Blutdruck bestimmt. Eine zu schmale Manschette (also schmaler als die oben genannten Manschette, die eine Breite von ca. 1/3 der Länge zwischen Schultergelenk und Ellenbogengelenk aufweist) lässt jedoch die Ursache 2 stärker zum Tragen kommen; bei einer solchen schmalen Manschette kann noch eine Pulsung bei höheren Drücken festgestellt werden, gleichwohl wird aufgrund des nicht irrelevanten Hervortretens der Ursache 2 ein erhöhter Blutdruckwert ermittelt, sofern die Blutdruckwertermittlung nach den Vorgehensweisen der Bestimmung des Blutdruckes gemäß den Verfahren aus dem Stand der Technik durchgeführt wird.

Für das erfindungsgemäße Verfahren ist insbesondere eine schmale Manschette, z.B. mit einer Breite von ca. 2-5cm, insbesondere 3-4cm, vorteilhaft, auch wenn normale, breite Manschetten ebenso für das erfindungsgemäß Verfahren geeignet sind, bei derartigen breiten Manschetten die Vorrichtungsbreitenkorrektur jedoch nicht durchgeführt werden muss, bzw. eine solche in der Regel kaum zu einer Korrektur führt.

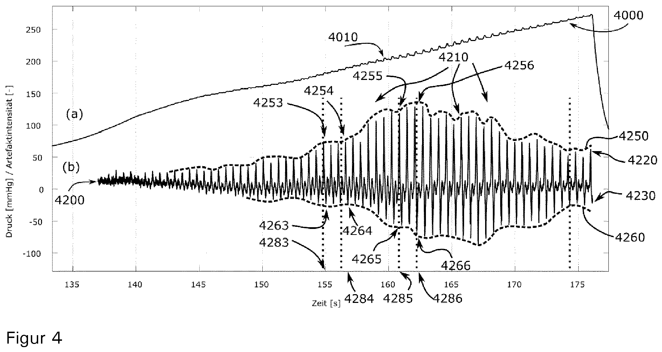

Der Vorteil der Verwendung einer schmalen Manschette, bei der dann insbesondere die erfindungsgemäße Vorrichtungsbreitenkorrektur durchgeführt werden kann, liegt nun darin, dass mit dieser neben den Herzpulsen auch weitere kleinere Variationen besonders gut aufgelöst werden. Eine schmale Manschette dämpft die Variationen nämlich nicht so stark, sodass auch geringe Blutdruckschwankungen, wie z.B. aufgrund der Atmung, in der Intensität der Variationen erkennbar werden. Da in den Verfahren nach dem Stand der Technik der Einfluss der Atmung nicht beachtet und berücksichtigte werden soll, wird genau aus diesem Grund beim Stand der Technik ein breites Armband verwendet, um so diesen Effekt der Ursache 2 möglichst nicht zu haben. Dabei wird die Breite ausgehend vom Armdurchmesser und -länge bestimmt, bei einer Oberarmmanschette sollte die Breite ca. einem Drittel der Länge von Schulter zur Armbeuge entsprechen. Für das erfindungsgemäße Verfahren stellt eine geringe Breite kein größeres Hindernis dar, ist insbesondere von Nutzen, insbesondere wenn die Atmung direkt aus der Luftdruckkurve bestimmt werden soll. In Abbildung 4 ist eine Druckverlaufskurve 4000 gezeigt, die mit einer 3 cm breiten Manschette aufgezeichnet wurde. Es ist zu erkennen, dass die Variationen 4010 bis zum Abbruch bei 270 mmHg zu erkennen sind, eine Bestimmung des Blutdrucks nach dem Stand der Technik würde eine Systole von über 200 mmHg ausgeben (eine Referenzmessung mit einem Gerät nach Stand der Technik ergab einen Blutdruck von 140/100 mmHg). Der Graph 4200 zeigt die erste Ableitung der Luftdruckkurve (skalierte Darstellung). Die beiden Bereiche der Ursachen sind anhand der lokalen Minima zu erkennen. Zum Zeitpunkt 160s werden die lokalen Minima tiefer, dies bedeutet, dass die Ursache 2 zunehmend an Bedeutung für die Variationen erlangt. Der Graph 4200 ist die Berechnungsgrundlage für alle weiteren Schritte, um Zeitpunkte für Druckbestimmungen (die Drücke werden dann auf der Luftdruckkurve 4000 abgelesen zu den jeweiligen Zeitpunkten) und Zeitpunkte für die Atmung zu bestimmen. Außerdem können mit dieser Kurve 4200 die Bereiche der Bedeutung der beiden Ursachen bestimmt werden, wodurch eine Berechnung des Blutdrucks vergleichbar zu der Berechnung nach dem Stand der Technik auch bei schmalen Manschetten ermöglicht wird.

Insbesondere werden einige Verfahrensschritte des erfindungsgemäßen Verfahrens an Verfahrensschritte angelehnt, die auch aus dem Stand der Technik bekannt sind, insbesondere um eine Eignung des erfindungsgemäßen Verfahrens mit bekannten Vorrichtungen zu gewährleisten. Gleichwohl wird das erfindungsgemäß Verfahren um einige wesentliche Verfahrensschritte erweitert, die gerade auf Verwendungen und Bestimmungen von Parametern und Informationen abzielen und diese einbeziehen, die in den Verfahren des Standes der Technik in der Regel ignoriert oder deren Einfluss durch Mittelung oder Auswahl der Manschettenbreite möglichst geringgehalten wurde. Beispielsweise und vereinfacht sind die einzelnen Schritte:

1. Erkennung und Filterung des Effekts durch die Ursache 2 auf die Intensität der Variationen. Dieser Schritt findet sich im Stand der Technik nicht, da hier durch geeignete Auswahl der Vorrichtungsbreite, der Einfluss und Effekt der Ursache 2 versucht wurde, zu minimieren. Nach Erkennung und Filterung ergibt sich eine, insbesondere vorrichtungsbreitekorrigierte, Intensitätskurve, die einer für den Stand der Technik optimalen Intensitätskurve entspricht. Die, insbesondere vorrichtungsbreitekorrigierte, Intensitätskurve entspricht nun einer Intensitätskurve, die der Messkurve, welche mit einer optimal für den Nutzer breiten Manschette aufgenommen wurde wäre.

2. Bestimmung der Blutdruckwerte Diastole und/oder Systole (D1 und S1), ohne Berücksichtigung des Einflusses der Atmung, nach dem Verfahren nach dem Stand der Technik.

3. Die Zeitpunkte an denen D1 und/oder S1 bestimmt wurden, markieren mit ihrem Zeitpunkt die zugehörigen Atemzüge. Es wird nun in der Druckverlaufskurve der Druck zum Zeitpunkt bspw. der Einatmung und bspw. der Ausatmung gefunden. Diese Werte sind dann bspw. die Ergebnisse für den Blutdruck in der Ein- bzw. Ausatmung und geben somit einen Blutdruckwert in Abhängigkeit eines bestimmten Atemzustands der Atmung der Person an, sind somit atmungskorrigierte Blutdruckwerte im Sinne dieser Offenbarung.

Der obige Punkt 1 wird benötigt, wenn eine zu schmale (in Bezug auf den Stand der Technik) Manschette verwendet werden soll, was für eine Bestimmung der Atmung jedoch vorteilhaft ist und für die Abbildung von kontinuierlichen Werten des Blutdrucks in der Regel notwendig ist. Soll jedoch nur der Einfluss der Atmung auf den Blutdruck beurteilt werden, insbesondere also kein kontinuierlicher Blutdruckwert ermittelt werden, so ist dieser Punkt nicht notwendig.

Den Druckvariationen aufgrund des Herzpulses in der Luftdruckkurve kann beispielsweise ein Blutdruck zugeordnet werden. Bei völlig gleichmäßiger Atmung, bei gesundem Herzpuls wäre folgende Herangehensweise denkbar: Die Phase innerhalb der Atmung, an dem sich die Variation befindet, bestimmt linear welcher Wert zwischen den vorher bestimmten Blutdruckwerten für die Einatmung bzw. Ausatmung ausgegeben werden soll. Diese Herangehensweise bringt jedoch keine Mehrinformation und ist auch gefährlich, da Fälle, die nicht der Annahme über gleichmäßige Atmung und den gesunden Herzpuls entsprechen so nicht erkannt werden können. Aber diese Herangehensweise kann zur Überprüfung der nachfolgenden Logik benutzt werden. Wird ein Datensatz aufgezeichnet und sind dabei die oben genannten Annahmen erfüllt, so müssen die Ergebnisse dieser Herangehensweise mit denen der nachfolgenden Logik übereinstimmen.

Im Folgenden wird ein Beispiel für die vorteilhafte Bestimmung der Vielzahl von Blutdruckwerten, den kontinuierlichen Blutdruckwerten, dargestellt: Für die Logik zur Bestimmung von kontinuierlichen Blutdruckwerten können beispielsweise folgende Annahmen gemacht werden:

1. Die Intensität der Variationen auf der Druckverlaufskurve spiegeln den Blutdruck wider. D.h. würde ein Luftdruck in der Manschette (zwischen Diastole und Systole und je nach Manschettenbreite auch darüber) (im Mittel) gehalten werden, so ist eine Veränderung in der Höhe der Variationen linear mit einer Blutdruckveränderung.

2. Für einen Druck oberhalb der Diastole und im Zeitraum in dem die Ursache 2 der Druckvariation zum Tragen kommt ist ein Herzpuls vollständig in der Variation abgebildet.

3. Für den unter 2. genannten Zeitraum gilt weiter: Wenn der Blutdruck gleichbleibend ist und die Manschette nicht zu breit ist, so ist der Höhendurchmesser der Einhüllenden des Intensitätsverlaufs linear abnehmend mit steigendem Luftdruck in der Manschette. Aus 3. geht hervor, dass (unter der Annahme) die Variationen in der Luftdruckkurve auch linear abfallen mit steigendem Druck.

4. Die Werte für die Systole befinden sich in dem unter 2. genannten Zeitraum.

Die Logik geht beispielsweise wie folgt vor:

1. Bestimmung des Zeitraums, in dem die Ursache 2 der Druckvariation hauptsächlich zum Tragen kommt. Dies ist analog zum 1. Punkt bei der Bestimmung der Blutdruckwerte für die Ein- bzw. Ausatmung. Dies erfolgt auf Basis der Intensitätskurve, welche durch die Ableitung der Druckkurve gegeben ist.

2. Es wird die Intensitätskurve nach dem Stand der Technik durch einen geeigneten Bandpass Filter bestimmt.

3. Es wird die Einhüllende um diese Intensitätskurve bestimmt, woraus der Höhendurchmesser für jeden Zeitpunkt bekannt ist.

4. Der zeitliche Verlauf des Höhendurchmessers wird linear genähert.

5. Die Intensitätskurve aus Punkt 2 wird mit dem zeitlichen Verlauf des Höhendurchmessers im Bereich, an dem die Ursache 2 hauptsächlich zum Tragen kommt, normiert. Danach wird die Kurve so skaliert, dass die Intensitätskurve an den Zeitpunkten, an denen die Werte der Systole bestimmt wurden, mit diesen übereinstimmt. Diese Normierung und Skalierung findet in dem Bereich statt in dem die Datenauflösung eine sinnvolle Skalierung zulässt. Bei einem guten Ausgangssignal (hoher Pulsdruck, keine Bewegung) kann dies unter Verwendung heutiger Drucksensoren bis zu einem Höhendurchmesser von einem Zehntel des maximalen Höhendurchmessers erfolgen. Es ergibt sich eine Druckverlaufskurve, die den Blutdruckverlauf in den Arterien unterhalb der Manschette abbildet.

6. Diese Kurve wird auf lokale Minima und Maxima hin untersucht. Die Maxima sind Werte für die Systole, wobei für jeden Herzschlag in diesem Zeitraum ein Wert bereitgestellt werden kann. Die lokalen Minima sind Werte für die Diastole, wobei auch hier für jeden Herzschlag ein Wert bereitgestellt werden kann. Es ist anzumerken, dass die Werte für die Diastole nicht als absolut Werte interpretiert werden sollten, da diese nur indirekt über die Werte der Systole bestimmt wurden. Diese Werte zeigen jedoch die Tendenz und Stärke der Veränderung der Diastole an.

Dieses Ausführungsbeispiel stellt jedoch lediglich ein mögliches Beispiel des Ausführens des erfindungsgemäßen Verfahrens dar.

Vorteilhafterweise umfasst der Messzeitraum mindestens zehn Sekunden und vorteilhafterweise erfolgt die Erfassung der Einzelwertmessungen, die die kontinuierliche Blutdruckmessung ergeben, zu jedem Herzschlag.

Alternativ kann die oben genannten Bestimmung einer Vielzahl von Blutdruckwerten auch eine Bestimmung einer Vielzahl von nicht-atmungskorrigierter Blutdruckwerte oder gar eine Bestimmung einer Vielzahl von nicht-atmungskorrigierten oder atmungskorrigierten relativer Blutdruckwerte sein. Eine solche Bestimmung könnte als Tendenzmessung bezeichnet werden. Hierfür wird auf die Skalierung der normierten Einzelwerte, der insbesondere vorrichtungsbreitenkorrigierten, Intensitätskurve verzichtet oder lediglich auf Basis nicht-atmungskorrigierter Blutdruckwerte skaliert. Der Vorteil einer derartigen Bestimmung ist, dass bspw. eine Bestimmung der Atmung nicht notwendig ist. Gleichwohl ist eine Bestimmung des Verlaufs des Blutdrucks, entweder allein als eine Änderung über die Zeit oder als nicht-atmungskorrigierter Wert möglich. Unter bestimmten Umständen genügen derartige Aussagen ebenso und stellen eine Verbesserung gegenüber bisher bekannten Methoden der kontinuierlichen Erfassung des Blutdrucks dar.

Vorgeschlagen wird daher auch eine Bestimmung von einer Vielzahl von relativen Blutdruckwerten. Dieses Verfahren wird in analoger Weise wie das oben beschriebene erfindungsgemäße Verfahren durchgeführt. Hierfür werden zunächst die mehreren Einzelwertmessungen des dem Blutdruckwert der Person zugeordneten Wertes innerhalb des Messzeitraums zeitaufgelöst mittels des ersten Sensors der ersten Vorrichtung erfasst. Gleichwohl muss, zur Bestimmung der Vielzahl der relativen Blutdruckwerten kein nicht-atmungskorrigierter Blutdruckwert bestimmt werden. Vielmehr können direkt die von der variierenden Druckbeaufschlagung unabhängige Änderungen, insbesondere Steigungen, der Werte der Einzelwertmessungen, insbesondere ermittelt mittels einer mathematischen Ableitung nach der Zeit, aus den, insbesondere die variierende Druckbeaufschlagung aufweisenden, Werten der Einzelwertmessungen ermittelt werden und hieraus dann kann direkt die Intensitätskurve, aufweisend die mehreren lokalen Extrema, ermittelt werden. Auch hier kann nötigenfalls, wie oben beschrieben, eine vorrichtungsbreitenkorrigierte Intensitätskurve ermittelt werden.

Zu den in dem Messzeitraum, insbesondere der dritten und/oder der zweiten Zeitspanne oder ersten Zeitspanne, vorliegenden lokalen Maxima der, insbesondere vorrichtungsbreitenkorrigierten, Intensitätskurve wird dann die mindestens eine, insbesondere mindestens zwei, obere Näherungsgerade gebildet und zu den in dem Messzeitraum, insbesondere der sechsten und/oder der fünften Zeitspanne oder der vierten Zeitspanne, vorliegenden lokalen Minima der, insbesondere vorrichtungsbreitenkorrigierten, Intensitätskurve wird dann die mindestens eine, insbesondere mindestens zwei, untere Näherungsgerade gebildet.

Zur Ermittlung der Vielzahl relativer Blutdruckwerte werden die Einzelwerte der, insbesondere vorrichtungsbreitenkorrigierten, Intensitätskurve auf Basis der zu den jeweiligen Zeitpunkten vorliegenden Differenz zwischen der oberen Näherungsgerade und unteren Näherungsgerade normiert. Es erfolgt jedoch keine Skalierung mit der Skalierungskonstante.

Aus den normierten Einzelwerten wird dann eine relative Blutdruckverlaufskurve bestimmt wird, die lokale Maxima und lokale Minima aufweist. Aus den den lokalen Maxima oder Minima der relativen Blutdruckverlaufskurve zugeordneten Blutdruckwerten die Vielzahl relativen Blutdruckwerte bestimmt werden.

Möglich ist es auch eine Skalierung auf Basis von nicht-atmungskorrigierter Blutdruckwerte durchzuführen. Hierdurch könnte man eine Vielzahl nicht-atmungskorrigierte Blutdruckwerte ermitteln. Hierfür wird bei der Skalierungskonstante nicht ein atmungskorrigierter Blutdruckwert, sondern ein nicht-atmungskorrigierter Blutdruckwert und als Zeitpunkt der jeweilige Referenzzeitpunkt verwendet.

Die Aufgabe wird auch durch eine Vorrichtung, insbesondere eine Manschette, insbesondere Luftdruckmanschette, mit einem Armband, gelöst, wobei die Vorrichtung ein aufblasbares Volumen bereitstellt und eine Steuereinheit aufweist. Dabei ist die Steuereinheit eingerichtet und konfiguriert, das Volumen aufzublasen, insbesondere anhand einer vorbestimmten Druckbeaufschlagungsänderungsrate, und ferner eingerichtet und konfiguriert ist, das erfindungsgemäße Verfahren auszuführen. Insbesondere weist die Vorrichtung auch mindestens ein nichtflüchtiges Speichermedium auf, das insbesondere mit der Steuereinheit verbunden ist und/oder auf dem die Druckbeaufschlagungsänderungsrate und/oder das erfindungsgemäß Verfahren hinterlegt ist. Vorteilhafterweise ist die Vorrichtung auch dazu eingerichtet die Atmungskurve des erfindungsgemäßen Verfahrens zu erfassen und zu bestimmen.

Vorteilhafterweise ist die Vorrichtung eine Manschette, insbesondere Luftdruckmanschette, mit einem Armband, wobei das Armband eine Längserstreckung aufweist, wobei das Armband eingerichtet ist, entlang deren Längserstreckung einen Teil des Körpers zu umschließen, wobei das Armband eine Breitenerstreckung mit einer Breite von weniger als 5 cm, insbesondere 4 cm oder weniger, aufweist.

Vorteilhafterweise ist die Vorrichtung eine Manschette mit einem Armband, wobei die Manschette insbesondere ein Teil einer Armbanduhr, insbesondere einer Smart-Watch, ist.

Weitere vorteilhafte Ausführungsformen ergeben sich exemplarisch aus der nachstehenden Beschreibung der beigefügten schematischen Figuren. Dabei zeigt:

Fig. 1 (a) eine ideale Druckverlaufskurve einer Messung unter Verwendung einer Manschette nach dem Stand der Technik während eines externen Druckanstiegs sowie (b) Variationen innerhalb dieses Druckverlaufs nach Auswertung mittels eines digitalen Filters;

Fig. 2 (a) eine Druckverlaufskurve einer stark atmenden Person während eines externen Druckanstiegs sowie (b) Variationen innerhalb dieses Druckverlaufs nach Auswertung mittels eines digitalen Filters;

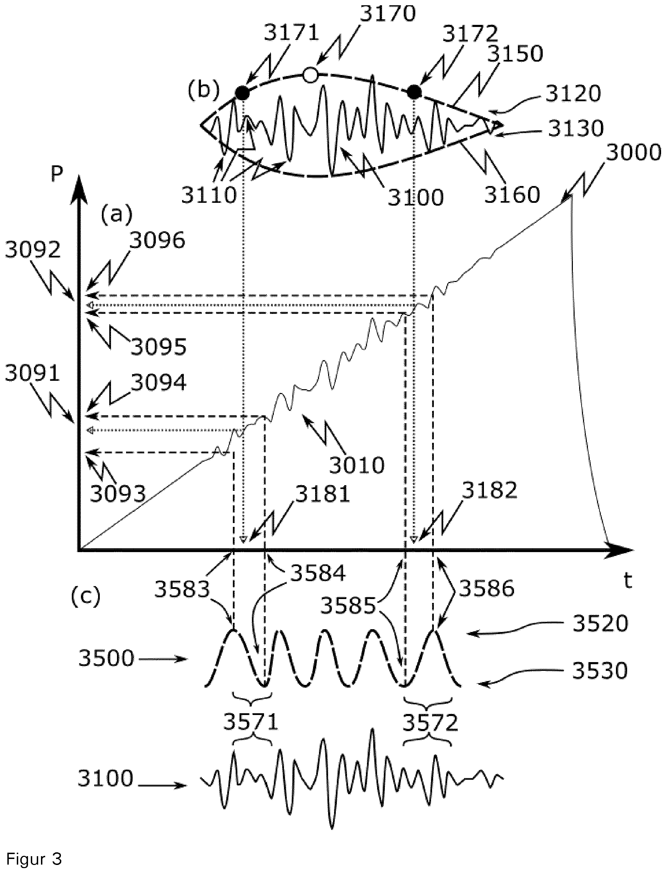

Fig. 3 (a) einen schematischen Druckverlauf des Luftdrucks (Y-Achse) über der Zeit (X-Achse) während des Druckanstiegs einer Messung mit einer Luftdruckmanschette, (b) die Auswertung des Druckverlaufs nach Riva Rocci und Korotkoff zur Bestimmung eines nicht-atmungskorrigierten diastolischen und systolischen Blutdrucks, (c) die erfindungsgemäße Auswertung mit Korrektur und Verwendung der Atmung bzw. des Atemzyklus innerhalb der Messreihe, die für die Bestimmung (in b) des diastolischen und systolischen Blutdrucks verwendet wurde um diastolische und systolische Blutdruckwerte bei unterschiedlichen Atemzuständen zu erhalten;

Fig. 4 (a) eine Messung einer Druckverlaufskurve über die Zeit und (b) ermittelte Variationen der Druckverlaufskurve aus (a) zur erfindungsgemäßen Bestimmung von Atemzuständen aus der Druckverlaufskurve;

Fig. 5. (a) eine Druckverlaufskurve mit einer gegenüber dem Stand der Technik schmaleren Manschette über die Zeit, (b) eine erfindungsgemäße Intensitätskurve als Steigungskurve der Druckverlaufskurve durch Bildung einer Ableitung der Druckverlaufskurve, (c) eine erfindungsgemäße vorrichtungsbreitenkorrigierte Intensitätskurve, die um die Effekte der schmaleren Manschette korrigiert wurde sowie in (d) die normierte und (e) die normierte und skalierte erfindungsgemäße vorrichtungsbreitenkorrigierte Intensitätskurve, um kontinuierliche Blutdruckwerte zu ermitteln;

Fig. 6. (a) ein erstes Szenario, (b) ein zweites Szenario und (c) ein drittes Szenario) der Erfassung von maximalen Einatmungen und maximalen Ausatmungen sowie Bestimmung des ersten Phasenabschnitts;

Fig. 7. Ausschnitt einer Verlaufskurve des Luftdrucks zur Veranschaulichung verschiedener Einflüsse auf die Ausprägung von Variationen innerhalb der Luftdruckkurve durch verschiedene den Blutdruck verändernde Einflüsse.

In den Figuren werden Ausführungsbeispiele des Verfahrens beispielhaft und teils vereinfacht dargestellt, so dass einzelne Merkmale der Erfindung besonders einfach und deutlich ersichtlich sind. Die Größenverhältnisse (bspw. der Peaks, Amplitude oder der Abstände zwischen den Peaks) sind somit nicht zwingend realitätsnah dargestellt. Ebenso stellen die Werte der Y-Achsen und Y-Achsen nicht notwendigerweise realitätsnahe Werte dar. Insbesondere für die Intensitätskurven sind keine Werte der Y-Achse angegeben. In den Figuren sind gleiche Elemente mit gleichen Bezugszeichen versehen.

In den nachfolgenden Beispielen wird auf eine Luftdruckmanschette, nachfolgend auch abgekürzt als "Manschette" bezeichnet, abgestellt, die die Blutdruckmessung durchführt. Dies kann beispielsweise eine gewöhnliche Handgelenk- oder Oberarmmanschette sein. Gleichwohl ist es ebenso möglich, dass andere Geräte als eine Manschette die Blutdruckmessung durchführen. Hinsichtlich der Manschette ist es möglich, dass andere Geräte als eine übliche Handgelenkmanschette hierfür eingesetzt werden. So weisen manche Smartwatches schon Manschetten auf, die den Blutdruck über die Manschette messen.

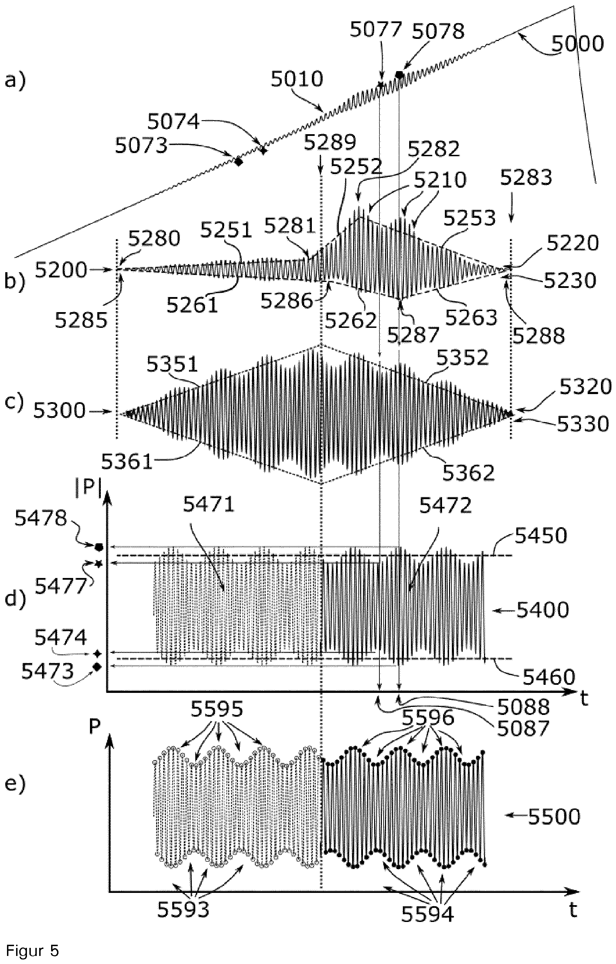

In den Figuren werden Diagramme u.a. Luftdruckverlaufskurven dargestellt, in denen gemessene Einzelwerte eines innerhalb der Manschette vorliegenden Luftdrucks abgebildet sind. Einzelwerte Luftdruckverlaufskurven lassen sich dabei Blutdruckwerten der Person zuordnen, an der die Messung mittels der Manschette durchgeführt wurde. Die Blutdruckwerte werden dabei anhand von während der Messung mittels der Druckmanschette über einen Messzeitraum (Figur 5) erfassten Einzelwertmessungen des in der Druckmanschette anliegenden Luftdrucks ermittelt. Auf der X-Achse des in den Figuren dargestellten Diagramms wird also die Zeit des Messzeitraums und auf der Y-Achse werden in der Regel die Luftdruckwerte der Einzelwertmessungen oder die aus den Einzelwertmessungen der Luftdruckwerte ermittelten Blutdruckwerte der Person dargestellt. Die sich aus den Einzelwertmessungen der Luftdruckwerte ergebene Kurve über die Zeit wird als Luftdruckverlaufskurve bezeichnet und beschreibt die Luftdruckwerte als Funktion der Zeit und die sich aus den aus den Einzelwertmessungen der Luftdruckwerte ermittelten Blutdruckwerte ergebene Kurve wird somit auch als Blutdruckverlauf bzw. Blutdruckverlaufskurve bezeichnet und beschreibt die Blutdruckwerte als Funktion der Zeit.

In der Regel wird in den nachfolgenden Figuren im Abschnitt (a) der jeweiligen Figur der Luftdruckverlauf dargestellt. Teils werden dabei auf der Y-Achse die den gemessenen Luftdruck zugeordnete absolute Blutdruckwerte angegeben oder zumindest relative Werte Dabei sind die in den Figuren abgebildeten absoluten Werte der Y-Achse wie erwähnt nicht notwendigerweise realitätsnah.

Die in der Figur 1 dargestellten Abbildung stellt einen idealen Verlauf von Luftdruckwerten dar. Der ideale Luftdruckverlauf 1000 ist dabei in dem Teilabschnitt a) der Figur 1 dargestellt. Zu erkennen ist, dass während des Messzeitraums der Luftdruckwert im Grunde kontinuierlich steigt. Dies ist dem Umstand geschuldet, dass während des Messzeitraums, also während der Einzelwertmessungen, der Luftdruck in der Manschette, bspw. mit einer konstanten Rate (Druckbeaufschlagungsrate), erhöht wurde, wie bspw. üblich zu Beginn einer Manschetten-Messung. Somit erhöhen sich die Einzelwerte des erfassten Luftdrucks und weisen somit auch diese Änderungen des Drucks während der Druckbeaufschlagung auf. Wie auch aus dem Stand der Technik kann dabei nicht aus jeder Einzelwertmessung des Luftdrucks ein sinnvoller Blutdruckwert ermittelt werden. Hierzu bedarf es eines Verfahrens um aus den so ermittelten Luftdruckwerten bspw. einen diastolischen Blutdruckwert oder einen systolischen Blutdruckwert zu ermitteln. Hierfür sind im Stand der Technik verschiedene Vorgehensweisen beschrieben. Diese bilden die Basis für die hier vorliegende Erfindung.

Zusätzlich zu dem kontinuierlichen Anstieg des ermittelten Blutdruckwerts im Druckverlauf 1000 sind mehrere Schwankungen bzw. Variationen 1010 des Luftdrucks auf der Kurve des Druckverlaufs 1000 zu erkennen. Diese Variationen basieren unter anderem auf durch den Herzschlag hervorgerufenen Schwankungen des Blutdrucks im Laufe der Zeit. Der Luftdruck in der Manschette wird von dem Puls in den Arterien, die unterhalb der Manschette vorliegen und von dieser indirekt kontaktiert werden, beeinflusst. Zu erkennen ist auch, dass anfangs (links in dem Diagramm) kaum bis keine Variationen zu erkennen sind und am Ende ebenso geringere Schwankungen zu erkennen sind.

Im Verlauf der Erhöhung des Luftdrucks in der Manschette, kann bei geringen Drücken der Manschette das Blut ungestört fließen. Im Umkehrschluss bedeutet dies, dass die Spannung der Muskeln und des Gewebes höher ist als der Druck in der Manschette, sodass keine durch den Blutfluss in den Arterien hervorgerufene Variationen in den Einzelwerten der Messung des Luftdrucks in der Manschette erkennbar sind. Daher sind der Puls, die Variationen bzw. eine Druckänderung in dem anfänglichen Verlauf der Druckkurve 1000 nicht zu erkennen. Steigt der Luftdruck in der Manschette, so kann diese Spannung, die ein Erkennen einer Variation verhindert, zu einem bestimmten Zeitpunkt und somit Luftdruck, zunächst während der diastolischen Phase im Herzzyklus nicht aufrechterhalten werden. Während der Diastole im Herzzyklus kommt es daher zu Variationen bzw. Artefakten hin zu niedrigeren Blutdruckwerten. Steigt der Luftdruck in der Manschette weiter, so kann der Blutfluss auch bei Phasen zwischen Diastole und Systole im Herzpuls nicht mehr störungsfrei aufrechterhalten werden und es sind Variationen zu erkennen. Bei noch höheren Luftdrücken ist der Blutfluss zu jeder Phase gestört, also auch während der Systole. Die Manschette wird mit dem Puls verformt bzw. der Luftdruck in der Manschette ändert sich, sodass also der Herzpuls im Luftdruck abgebildet ist. Bei diesen höheren Drücken ist der messbare Luftdruck nun genau zwischen dem Wert von Diastole und Systole. Wird der Luftdruck in der Manschette weiter erhöht, so wird die Arterie mehr und mehr abgequetscht, bis kein Blutfluss mehr möglich ist, da die Arterie kollabiert ist. Zu diesem Zeitpunkt ist dann auch keine Variation mehr erkennbar. Im Gesamtverlauf steigt also im Laufe des Druckanstiegs die Intensität der Variationen bzw. Artefakte aufgrund des Herzpulses zunächst mit dem ansteigenden Luftdruck und fällt danach bei weiter ansteigendem Luftdruck wieder ab. Wenn keine weiteren Umstände vorhanden sind, die den Druck in den Arterien beeinflussen würden, würde man einen solchen Verlauf der Variationen erwarten.

Diese Variationen sind in der Darstellung des Teilabschnitts b) der Figur 1 noch deutlicher zu erkennen.

Die Darstellung des Teilabschnitts b) der Figur 1 basiert auf den erfassten Einzelwertmessungen des Luftdrucks während des Messzeitraums, wobei der kontinuierliche Anstieg entfernt / herausgefiltert wurde. Dies kann rein mathematisch erfolgen, bspw. indem eine Regressionsgerade als Funktion der Zeit ermittelt wird und von den Blutdruckwertens des Druckverlaufs 1000 abgezogen wird. Es ist aber auch möglich, dies mit einem, insbesondere digitalen, Bandpassfilter zu realisieren. Die hierfür angewandte Technik ist aus dem Stand der Technik bekannt und insbesondere in den vorhandenen Vorrichtungen, Manschetten, basierend auf dem jeweils benutzten Sensor implementiert, insbesondere herstellerspezifisch. Diese Filterung des kontinuierlichen, generellen Anstiegs des gemessenen Luftdrucks aufgrund des externen Druckanstiegs (Druckbeaufschlagungskorrektur) im Gerät ist zwar notwendig und wird durch die Methode der Hersteller ausreichend zufriedenstellend gelöst, stellt jedoch keinen eigenständigen Aspekt der vorliegenden Erfindung dar.

In der gefilterten Verlaufskurve 1100, auch druckbeaufschlagungskorrigierte Intensitätskurve 1100 genannt, sind nun die Schwankungen bzw. Variationen 1110 deutlich erkennbar. Zu erkennen ist, dass die Intensität, also die Höhen und Tiefen der Variationen zu- und abnehmen.

Diese Variationen 1110 basieren im Grundsatz zunächst auf Änderung des Luftdrucks, die durch die systolische Phase des Herzzyklus bzw. durch die diastolische Phase des Herzzyklus hervorgerufen werden. Ferner ist zu erkennen, dass die Intensität dieser Variationen im Verlauf der Zeit zunächst ansteigen und dann wieder abfallen, jedoch nur ein einziges solches Ansteigen und Abfallen zu erkennen ist, also nur ein Bogen zu erkennen ist. In der Theorie und im Idealfall (wie oben besprochen) entsprechen die gemessenen Werte auch einem solchen einfachen Verlauf.

In der Regel und in der Praxis ist ein solcher Verlauf bei einer Messung an einer realen Person jedoch nicht zu beobachten, da noch weitere Umstände den Blutdruck verändern und somit die Einzelwertmessungen des Luftdrucks selbst beeinflussen und verändern.

Eine solche Messung an einer realen Person, die während des Messzeitraums, dem Messzeitraum, atmet, ist in der Figur 2 dargestellt.

In dieser Figur 2 sind in Teilabschnitt a) ebenso die gemessenen Einzelwerte des Luftdrucks und somit die sich daraus ergeben Luftdruckverlaufskurve 2000 dargestellt. Blutdruckwerte basieren dabei auf Einzelwertmessungen von in einer Manschette gemessenen Luftdruckwerten. Dabei wurde auch hier der Druck in der Manschette im Laufe der Zeit erhöht, weshalb sich in der Abbildung ein genereller Anstieg des ermittelten Luftdruckwertes ergibt.

Die in Figur 2 dargestellten Luftdruckwerte wurden anhand von innerhalb des Messzeitraums stattfindenden Einzelwertmessungen an einer Person durchgeführt, die während des Messzeitraums eine starke Atmung aufwies.

Sobald man den generellen aufgrund der Luftdruckerhöhung erfassten Druckanstieg herausnimmt / herausfiltert, ergibt sich der in dem Teilabschnitt b) der Figur 2 erkennbare Graph. In diesem, ist, ähnlich wie in Figur 1, die druckbeaufschlagungskorrigierte Intensitätskurve 2100 gezeigt, in der der gefilterte Druckverlauf 2100 dargestellt ist. Zur Erstellung dieser Intensitätskurve kann wieder bspw. ein Bandfilter (Druckbeaufschlagungskorrektur) eingesetzt werden.

In dem gefilterten Druckverlauf 2100 sind abermals die Variationen 2110 zu erkennen. Jedoch nimmt die Intensität der Variationen 2110 nicht stetig einmal zu, um dann wieder abzunehmen. Vielmehr sind mehrere Anstiege und Abfälle der Intensität der Variationen 2110 zu erkennen. Ein erster Anstieg mit anschließendem Abfall der Intensität der Variationen 2110 ist in einer ersten Gruppe 2111 der Variationen 2110 bei etwa 157 s, ein zweiter Anstieg mit anschließendem Abfall der Intensität der Variationen 2110 ist in einer zweiten Gruppe 2112 im Zeitabschnitt von 163 s bis 167 s und ein dritter Anstieg mit anschließendem Abfall der Intensität der Variationen 2110 ist in einer dritten Gruppe 2113 um 172 s zu erkennen. Zwar sind auch der generelle Anstieg und der generelle Abfall zu erkennen - die erste 2111 und dritte Gruppe 2113 weisen bspw. geringere Intensitäten auf als die zweite, mittlere Gruppe 2112. Gleichwohl ist zu erkennen - die einzelnen Gruppen 2111, 2112, 2113 sind klar voneinander abgrenzbar -, dass noch weitere Umstände als die Messmethode (An- oder Absteigen des Luftdrucks in der Manschette) die erfassbaren Luftdruckwerte ändern. Eine Auswertung gemäß dem Stand der Technik würde entweder nur die mittlere Gruppe 2112 auswerten (da diese klar als der bekannte Bogen erkennbar ist) oder die dem Stand der Technik zugrunde liegende Logik würde zwei oder alle drei sichtbaren Gruppen als einen Bogen, als eine Gruppe, interpretieren. Welche Interpretation gewählt wird, hängt von der genauen Implementation der dem Stand der Technik zugrunde liegenden Logik ab und kann bei leicht anderer Datenlage (z.B. bei einer direkt darauffolgenden Messung an der gleichen Person ohne Änderung der Manschette) anders erfolgen.

Es ist also gut möglich, dass die dem Stand der Technik zugrunde liegende Logik nicht nur die mittlere Gruppe 2112, sondern auch die untere Gruppe 2111 berücksichtigt. In einem solchen Fall werden kleinere diastolische Blutdruckwerte ausgegeben. Sofern nur die mittlere Gruppe 2112 berücksichtigt wird, wird ein höherer diastolische Blutdruckwert ausgegeben. Diese Unterschiede in der Ausgabe bei gleicher Datenlage sind jedoch unbefriedigend.

In der Figur 3 sind im Abschnitt (a) mit einer Luftdruckmanschette gemessene Einzelwerte des Luftdrucks schematisch dargestellt. Der in der Manschette vorherrschenden Luftdruck ist dabei auf der Y-Achse dargestellt und über die Zeit auf der X-Achse dargestellt, woraus sich eine Luftdruckverlaufskurve 3000 ergibt. Die Einzelwertmessung erfolgte über einen Messzeitraum, wobei während dieses Zeitraums der Druck in der Luftdruckmanschette erhöht wurde, also anstieg. Zu erkennen ist, dass anfangs keine Variationen 3010 in der Druckverlaufskurve 3000 zu erkennen ist. Wie im Zusammenhang mit den Figuren 1 und 2 schon beschrieben, ist dies dem angewandten Messverfahren geschuldet, da der von der Manschette ausgeübte Druck auf den Messbereich auf der Haut der Person noch nicht ausreicht, um eine messbare Änderung des Gegendrucks erfassen zu können, die durch den Druck in den Arterien im Herzpuls erzeugt wird. Im mittleren Teil des Druckverlaufs 3000 sind dann solche Variationen 3010 zu erkennen, wobei diese Variationen 3010 im späteren Verlauf mit weiterem Anstieg des Drucks in der Manschette wieder verschwinden, unter anderem deshalb, da die Arterie abgequetscht wird und kollabiert ist, also kein Blut mehr fließt und somit auch keine Druckschwankungen in der Arterie mehr auftreten können.

Wie auch im Stand der Technik werden nun zunächst ein diastolischer und ein systolischer Blutdruck bestimmt. Bei dieser Bestimmung wird der Einfluss der Atmung auf den erfassten Blutdruck jedoch ignoriert, weshalb in der Terminologie dieser Offenbarung der so bestimmte Blutdruck als nicht-atmungskorrigierter Blutdruck bezeichnet wird. Hierfür wird in einem ersten Schritt der Druckanstieg aus der Druckverlaufskurve 3000 herausgefiltert. Dies kann auf unterschiedliche Art und Weise erfolgen. Typischerweise wird hierfür ein digitaler Bandpassfilter verwendet, jedoch kann auch im einfachsten Falle eine theoretische Druckanstiegskurve, gegeben durch eine lineare Anpassung der Druckverlaufskurve, von der Druckverlaufskurve subtrahiert werden.

Im Folgenden der Beschreibung der noch verbleibenden Figurenbeschreibung wird hinsichtlich der verwendeten Terminologie bzgl. des nicht-atmungskorrigierten ersten Blutdruckwertes sowie der atmungskorrigierten ersten Blutdruckwerte auf einen systolischen Blutdruckwert, somit auf einen nicht-atmungskorrigierten systolischen Blutdruckwert sowie auf die atmungskorrigierten systolischen Blutdruckwerte, und bzgl. des nicht-atmungskorrigierten zweiten Blutdruckwertes sowie der atmungskorrigierten zweiten Blutdruckwerte auf einen diastolischen Blutdruckwert, somit auf einen nicht-atmungskorrigierten diastolischen Blutdruckwert sowie auf die atmungskorrigierten diastolischen Blutdruckwerte, abgestellt.

Die so gefilterte Druckverlaufskurve 3100 ist in dem Teilabschnitt (b) der Figur 3 dargestellt. Anhand dieser wird eine Auswertung des Blutdruckwertes nach Riva Rocci und Korotkoff durchgeführt, um so einen nicht-atmungskorrigierten diastolischen und systolischen Blutdruck zu bestimmen. In diesem gefilterten Druckverlauf 3100 wird eine obere Ausgleichskurve 3150 und wird eine untere Ausgleichskurve 3160 ermittelt, die sich über den gesamten Verlauf der Druckverlaufskurve 3100 erstreckt, in denen Variationen 3110 des Luftdruckwertes in der Druckverlaufskurve 3100 erfassbar sind. Die obere Ausgleichskurve 3150 stellt dabei eine über die X-Ache ermittelte Näherung an die größten Intensitäten 3120 der Variationen 3110 und die untere Ausgleichskurve 3160 stellt eine über die X-Ache ermittelte Näherung an die kleinsten Intensitäten 3130 der Variationen 3110 dar. Teilweise wird in dieser Offenbarung für die Kombination einer oberen Ausgleichskurve und einer unteren Ausgleichskurve auch der Begriff "Einhüllende" verwendet, auch wenn dieser mathematisch eigentlich auf Geradenscharen, ansonsten aber für das gleiche Prinzip verwendet wird.

Bei dem erfindungsgemäßen Verfahren wird dann ein größter Abstand 3170 auf der Y-Achse (also bei jeweils gleichem X-Wert, also bei jeweils gleicher Zeit) zwischen der oberen 3150 und unteren 3160 Ausgleichskurve ermittelt. Ausgehend von diesem größten Abstand 3170 wird ein weiterer Abstand 3171 zwischen den Ausgleichskurven 3150, 3160 auf der Y-Achse hin zu niedrigeren Luftdrücken, hier zu zeitlich vorangehenden Werten ermittelt, der im Vergleich zum größten Abstand 3170 um einen vorgegebenen, bspw. durch den Hersteller der Manschette vorgegebenen, Wert verhältnismäßig kleiner ist. Dieser Abstand und Punkt ist in der Figur mit dem Referenzzeichen 3171 gekennzeichnet. Diesem Abstand 3171 ist ein Zeitpunkt 3181 in der Druckverlaufskurve 3000 zugeordnet. Auf dem zu diesem Zeitpunkt erfassten Luftdruckwert 3091 basiert dann der nicht-atmungskorrigierten diastolischen Blutdruck. Hierfür wird der dem Zeitpunkt des Abstandes 3171 zugehörige Blutdruck 3091 auf der Druckverlaufskurve 3000 ausgelesen und als nicht-atmungskorrigierten diastolischen Blutdruck verwendet. Im Sinne der vorliegenden Offenbarung der Erfindung und der Ansprüche sind dieser Zeitpunkt und der Blutdruck der zweite Referenzzeitpunkt 3181 und der nicht-atmungskorrigierte zweite (diastolische) Blutdruck basierend auf dem Luftdruck 3091. In analoger Weise wird ausgehend von dem größten Abstand 3170 ein weiterer Abstand 3172 zwischen den Ausgleichskurven 3150, 3160 auf der Y-Achse hin zu höheren Luftdrücken, hier zu zeitlich späteren Werten ermittelt, der im Vergleich zum größten Abstand 3170 um einen vorgegebenen, bspw. durch den Hersteller der Manschette vorgegebenen, Wert verhältnismäßig kleiner ist. Dieser Abstand ist an der oberen Annäherungskurve 3150 in der Figur 3 mit dem Referenzzeichen 3172 als Punkt gekennzeichnet. Auf diesem Abstand 3172 und den diesem Wert zugeordneten Zeitpunkten 3182 und erfassten Luftdruckwert 3092 basiert dann der nicht-atmungskorrigierten systolische Blutdruck. Hierfür wird der zum Zeitpunkt 3182 des Abstandes 3172 zugehörige Luftdruck 3092 auf der Luftdruckverlaufskurve 3000 ausgelesen und als nicht-atmungskorrigierten systolischer Blutdruck verwendet. Im Sinne der vorliegenden Offenbarung der Erfindung und der Ansprüche ist dieser Zeitpunkt der erste Referenzzeitpunkt 3182 und ist der nicht-atmungskorrigierte erste (systolische) Blutdruck, welcher aus dem Luftdruck 3092 ermittelt wird.

Da durch die Atmung jedoch die erfassbaren Blutdruckwerte aufgrund der Atmung höher oder niedriger sind, können die zum ersten 3181 bzw. zweiten Zeitpunkt 3182 aus dem Luftdruckwerten 3091, 3092 erfassbaren Blutdruckwerte aufgrund der Atmung höher oder niedriger sein, als der eigentliche, nicht von der Atmung beeinflusste, diastolische bzw. systolische Blutdruck der Person.

Aus diesem Grund wird die Atmung in Form mehrere Phasenabschnitte und insbesondere eines ersten Phasenabschnitts 3572 und eines zweiten Phasenabschnitts 3571 der Atmung verwendet und ein entsprechender diese Atmung bzw. den jeweiligen Phasenabschnitt berücksichtigender Blutdruckwert ermittelt. Hierfür werden vorteilhafterweise mehrere maximale Einatmungen und mehrere maximale Ausatmungen, und somit mehrere auf den maximalen Einatmungen und maximalen Ausatmungen basierende Phasenabschnitte in demselben Messzeitraum erfasst, in der auch die Einzelwertmessungen des Luftdrucks 3000 der Manschette genommen wurden.

Die Atmung, die Atmungskurve, und hieraus die maximalen Einatmungen und maximalen Ausatmungen sowie die Phasenabschnitte können dabei mittels eines anderen Geräts erfasst und bestimmt werden, bspw. mittels EKG oder PPG Sensoren. Jedoch kann die Atmung und die Atmungskurve auch aus den Luftdruckwerten und der Druckverlaufskurve 3000 erfasst und bestimmt werden.

Die Atmungskurve kann mittels der erfassten Werten beschrieben werden und beschreibt die Einatmung und die Ausatmung. Die Atmungskurve weist somit einen maximal Wert auf, der bspw. die maximale Einatmung darstellt, und einen minimalen Wert, der bspw. die maximale Ausatmung darstellt. Mehrere solcher Zyklen von maximalen Einatmungen und Ausatmungen sind nun mit mehreren Minima 3530, die jeweils die maximale Ausatmung der Person zu diesem Zeitpunkt darstellen, und mit mehreren Maxima 3520, die jeweils die maximale Einatmung der Person zu diesem Zeitpunkt darstellen, in dem Teilabschnitt c) der Figur 3 zusammen mit dem gefilterten Druckverlauf 3100 ausschnittsweise dargestellt, nämlich für den Ausschnitt der Zeitspanne, in der die Ausgleichskurven 3150 und 3160 bestimmt wurden und in dem die nicht-atmungskorrigierten Blutdruckwerte basierend auf den Luftdruckwerten 3092, 3091 zu den ersten und zweiten Referenzzeitpunkten 3182, 3181 bestimmt wurden. Die Phasenabschnitte 3571, 3572 werden dabei durch die maximalen Ausatmungen und Einatmung definiert. Dabei weist ein zweiter Phasenabschnitt 3571 bspw. zunächst eine maximale Einatmung und im Verlauf noch eine maximale Ausatmung auf. Die darauffolgende maximale Einatmung ist schon dem nächsten Phasenabschnitt zugeordnet. Dabei beginnt der zweite Phasenabschnitt mit oder kurz vor dem Zeitpunkt der maximalen Einatmung und endet mit oder kurz nach dem Zeitpunkt der maximalen Ausatmung. Wie nachfolgend erläutert, wird für die Systole ein Phasenabschnitt, nämlich ein erster Phasenabschnitt 3572, verwendet, der zuerst eine maximale Ausatmung aufweist. Dementsprechend weist der erste Phasenabschnitt 3572 bspw. zunächst eine maximale Ausatmung und im Verlauf noch eine maximale Einatmung auf. Die darauffolgende maximale Ausatmung ist schon dem nächsten Phasenabschnitt zugeordnet. Dabei beginnt der erste Phasenabschnitt mit oder kurz vor dem Zeitpunkt der maximalen Ausatmung und endet mit oder kurz nach dem Zeitpunkt der maximalen Einatmung. Dabei werden die Zeitpunkte der jeweiligen Maxima 3520, also der maximalen Einatmung eines jeden Phasenabschnitts 3571, 5372, als erster Zeitpunkt 3583, 3586, sowie die Zeitpunkte der jeweiligen Minima 3530, also der maximalen Ausatmung eines jeden Phasenabschnitts 3571, 3572, als zweiter Zeitpunkt 3584. 3585 erfasst.

In dem erfindungsgemäßen Verfahren wird dann der zweite Phasenabschnitt 3571 so ermittelt, der genau eine maximale Einatmung zum ersten Zeitpunkt 3583 sowie genau eine maximale Ausatmung zum zweiten Zeitpunkt 3584 aufweist und zwischen dessen Beginn des zweiten Phasenabschnitts und dem Ende des zweiten Phasenabschnitts der zweite Referenzzeitpunkt 3181 liegt. Es wird also der beim Erfassen des nicht-atmungskorrigierten diastolischen Blutdruckwertes zum Luftdruck 3091 vorliegende Phasenabschnitt ausgewählt.

Zudem wird ein erster Phasenabschnitt 3572 so ermittelt, der genau eine maximale Ausatmung zum zweiten 3585 sowie genau eine maximale Einatmung zum ersten Zeitpunkt 3586 aufweist und zwischen dessen Beginn des ersten Phasenabschnitts und dessen Ende des ersten Phasenabschnitts, der erste Referenzzeitpunkt 3182 liegt. Es wird also der beim Erfassen des nicht-atmungskorrigierten systolischen Blutdruckwertes zum Luftdruck 3092 vorliegende Phasenabschnitts ausgewählt.

Um nun die Atmung entsprechend zu qualifizieren, kann dann ein diastolischer und ein systolischer Blutdruckwert jeweils als ein entsprechender Blutdruckwert bei maximaler Ausatmung bzw. maximaler Einatmung ermittelt und ausgegeben werden.

Für den diastolischen Blutdruckwert bei maximaler Einatmung (im Sinne der Anmeldung der erste des atmungskorrigierten zweiten Blutdruckwerts) wird aus der Druckverlaufskurve 3000, die auf den Einzelwertmessungen basieren, der zum erste Zeitpunkt 3583 des zweiten Phasenabschnitts 3571 vorliegende Luftdruck 3093 der Druckverlaufskurve 3000 ausgelesen und so der diastolische Blutdruckwert bei maximaler Einatmung ermittelt.

Für den diastolischen Blutdruckwert bei maximaler Ausatmung (im Sinne der Anmeldung der zweite des atmungskorrigierten zweiten Blutdruckwerts) wird aus der Druckverlaufskurve 3000, die auf den Einzelwertmessungen basieren, der zum zweiten Zeitpunkt 3584 des zweiten Phasenabschnitts 3571 vorliegende Luftdruck 3094 der Druckverlaufskurve 3000 ausgelesen und somit der diastolische Blutdruckwert bei maximaler Ausatmung ermittelt.

Für den systolischen Blutdruckwert bei maximaler Ausatmung (im Sinne der Anmeldung der zweite des atmungskorrigierten ersten Blutdruckwerts) wird aus den, auf den Einzelwertmessungen basierenden Blutdruckwerten der Druckverlaufskurve 3000 der zum zweiten Zeitpunkt 3585 des ersten Phasenabschnitts 3572 vorliegende Luftdruckwert 3095 der Druckverlaufskurve 3000 ausgelesen und somit der systolische Blutdruckwert bei maximaler Ausatmung ermittelt.

Für den systolische Blutdruckwert bei maximaler Einatmung (im Sinne der Anmeldung der erste des atmungskorrigierten ersten Blutdruckwerts) wird aus Luftdruckwerten der Druckverlaufskurve 3000, die auf den Einzelwertmessungen basieren, der zum ersten Zeitpunkt 3585 des ersten Phasenabschnitts 3572vorhandene, auf den Einzelwertmessungen basierende Luftdruckwert 3096 der Druckverlaufskurve 3000 ausgelesen und somit der systolische Blutdruckwert bei maximaler Einatmung ermittelt.

Die auf diese Weise erhaltenden Werte für die Diastole und Systole bei maximaler Ein- und Ausatmung können nun entweder direkt im Folgenden verwendet werden oder vorzugsweise noch um den externen Druckanstieg innerhalb der gefunden Atmungsphase korrigiert werden. Alternativ kann auch zuvor der externe Druckanstieg innerhalb der gefunden Atmungsphase korrigiert werden.

Eine Korrektur ist für höchste Genauigkeit erforderlich und z.B. für Zwecke der Kalibration dritter Geräte vorzugsweise durchzuführen. Für eine reine Wertausgabe des Blutdrucks ist dies nicht notwendig.

Die Korrektur geht davon aus, dass der externe Druckanstieg p(t) linear erfolgt mit der Zeit t über die zwei Phasenabschnitte erfolgt mit je einer Druckänderungsrate m, welche positiv bei steigendem Druck bei der Messung ist und entsprechend negativ bei einer Messung mit fallendem Druck ist und nicht notwendigerweise jedoch in der Regel für beide Phasenabschnitte gleich ist, und somit durch p(t) = m*t+b gegeben ist. Diese Druckänderungsrate wird wie vorangehend beschrieben von außen durch eine Pumpe und/oder ein Ventil vorgegeben. Ferner wird davon ausgegangen, dass die Blutdruckwerte zu den Luftdrücken 3091 und 3092 gleich diesen Luftdrücken sind und somit nicht korrigiert werden müssen. Die ermittelten Blutdrücke aus den Luftdrücken 3093, 3094, 3095 und 3096 unterliegen nun dem zu korrigierenden Fehler, da sich innerhalb der Phasenabschnitte der von außen vorgegebene Druck verändert hat. Dieser Fehldruck für den Blutdruck, der Werte, die sich aus den Luftdruckwerte 3093, 3094, 3095 und 3096 ergeben, ergibt sich aus dem Zeitunterschied zwischen den Zeitpunkten 3583, 3584 zu 3181 bzw. 3585, 3586 zu 3182 multipliziert mit der Druckänderungrate m. Anzumerken ist, dass die Zeitunterschiede eine negative Zahl sind, wenn der Zeitunterschied sich von einem Zeitlich späteren Punkt auf einen zeitlich früheren Punkt bezieht.

Dieser Fehldruck, welcher für jeden Zeitpunkt 3583, 3584, 3535, 3536 separat bestimmt wird, wird von den jeweiligen Luftdruck (3093, 3094, 3093, 3096) subtrahiert. Diese so korrigierten Luftdruckwerte werden nun für vorher beschriebenen Blutdruckwerte verwendet.

Aus dem Wertepaar aus dem diastolischen Blutdruckwert bei maximaler Einatmung und dem diastolischen Blutdruckwert bei maximaler Ausatmung kann dann bspw. durch eine Mittelung der Werte des Wertepaars ein gemittelter, atmungskorrigierter diastolischen Blutdruckwert ermittelt werden. Analog kann bei dem Wertepaar des systolischen Blutdruckwerts vorgegangen werden.

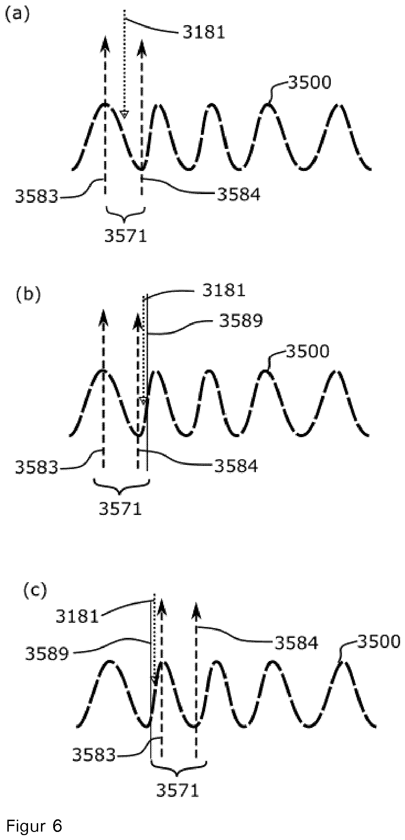

Eine Schwierigkeit, auf die die Erfinder bei der Umsetzung gestoßen sind, liegt nun darin, dass die Länge der Einatmungsphasen und die Länge der Ausatmungsphasen, sowie die verwendeten Messmethoden zum Bestimmen der Atmungskurve zu einem systematischen Fehler führen, wodurch die bestimmbaren Zeitpunkte der maximalen Einatmung und der maximalen Ausatmung relativ zu den in den Einzelwertmessungen bestimmbaren dem Blutdruckwert zugeordneten Werten und deren Zeitpunkte zeitversesetzt erfasst werden. Dies liegt darin begründet, dass die unterschiedlichen Methoden zur Atmungsbestimmung unterschiedliche Definitionen hinsichtlich des Beginns eines Atemzugs und Ende eines Atemzugs verwenden und dass unterschiedliche Methoden zur Atembestimmung die Zeiten der Atemphasen unterschiedlich erfassen. Dieser Zeitversatz kann bei einer besonders vorteilhaften Weiterbildung der Erfindung mit einfließen und korrigiert werden. Ein Beispiel einer solchen Korrektur ist in der Figur 6 dargestellt und wird später beschrieben.

In der Figur 4 ist in Teilabschnitt a) der Figur ein Druckverlauf 4000 aus mehreren Einzelwertmessungen von Luftdruckwerten, die mittels einer Messung des Luftdrucks in der Luftdruckmanschette, über die Zeit dargestellt. In diesem Druckverlauf 4000 sind - analog zu den Druckverläufen, wie in den vorangehenden Abbildungen gezeigt - zumindest in einem Zeitintervall Variationen 4010 in dem Druckverlauf zu erkennen. Sofern die Information hinsichtlich der Atmung, also der Atemzyklen, der Person während des Messzeitraums nicht von anderen Geräten oder Sensoren bereitgestellt wird, kann die Information der Atmung, der Atemzyklen, aus dem Druckverlauf 4000 während der Messung innerhalb des Messzeitraums bestimmt werden.

Hierfür kann die generelle Steigung über die Zeit aufgrund der Luftdruckerhöhung aus der Druckverlaufskurve 4000 gefiltert werden, analog zu den vorherigen Beschreibungen zum Erhalt der Kurven 1100, 2100, 3100. Vorgezogen wird es jedoch, wenn die erste mathematische Ableitung der Druckverlaufskurve 4000 über die Zeit gebildet wird, um so die Änderungen der Druckverlaufskurve abzubilden. Eine solche Ableitung der Druckverlaufskurve 4000 über die Zeit ist im Teilabschnitt b) der Figur 4 dargestellt, wobei die Werte der Ableitung zur besseren Erkennbarkeit skaliert dargestellt sind. In dieser skalierten Intensitätsverlauf 4200 sind also die Steigungen der Druckverlaufskurve 4000 skaliert dargestellt. Somit sind auch dort die in der Druckverlaufskurve 4000 gegebenen Variationen 4010 erkennbar, nämlich durch lokale maximale und minimale Werte 4210 in der Intensitätskurve 4200. Somit stellen die maximalen Werte 4210 in der Intensitätskurve 4200 lokale Maxima 4220 und die minimalen Werte 4210 in der Intensitätskurve 4200 lokale Minima 4230 dar. Dabei ist zu erkennen, dass die absoluten Werte der lokalen Maxima 4220 und lokalen Minima 4230 im Laufe der Zeit schwanken, nämlich in einer ersten, groben Annäherung zunächst anwachsen und anschließend wieder abfallen. Ferner ist zu erkennen, nämlich bei einer etwas detaillierteren Betrachtung, dass die absoluten Werte der lokalen Maxima 4220 und lokalen Minima 4230 zyklisch steigen und wieder fallen. Aus diesem zyklischen Muster können nun mittels des erfindungsgemäßen Verfahrens die Atemzyklen der Person während des Messzeitraums ermittelt werden.

Hierfür werden einerseits die maximalen Werte 4220 als auch die minimalen Werte 4230 betrachtet und wird für diese jeweils eine Ausgleichskurve erstellt. Die erste Ausgleichskurve 4250 ist also eine Ausgleichskurve zu den lokalen Maxima 4220 und die zweite Ausgleichskurve 4260 eine Ausgleichskurve zu den lokalen Minima 4230. Sowohl für die erste Ausgleichskurve 4250 als auch für die zweite Ausgleichskurve 4260 wird bevorzugt ein Best-Fit, bspw. über ein Fitting der kleinsten Quadrate (zwischen Fit und Datenpunkt), insbesondere iterativ, verwendet, sodass die jeweiligen Extremwerte in der Intensitätsverlauf 4200 optimal angenähert werden. Möglich ist es auch, die Ausgleichskurven lediglich abschnittsweise zu erstellen, so dass diese den aufgefundenen Zyklen besser entsprechen.

In den erstellten Ausgleichskurven 4250 und 4260 sind lokale Schwankungen auf und in der jeweiligen Ausgleichskurve zu erkennen. So weist beispielsweise die erste, obere Ausgleichskurve 4250 bei etwa 155 Sekunden einen lokal maximalen Intensitätswert 4253 auf. Da an derselben Stelle auch die zweite, untere Ausgleichskurve 4260 einen lokal geringsten Wert 4263 aufweist, ist dort der Abstand zwischen den beiden Ausgleichskurven 4250, 4260 an diesem Zeitpunkt im Vergleich zur Umgebung maximal. Ein solcher lokaler maximaler Abstand ergibt sich durch die Änderung des Blutdrucks innerhalb der Atmung. Im Umkehrschluss bedeutet dies, dass der Abstand ein Maß für die Atmung an sich ist Diesen Umstand haben Sie die Erfinder zu Nutze gemacht. Daher ist diesen maximalen Abständen bspw. 4253 zu 4263 und 4256 zu 4266 ein maximaler Zustand der Atmung, also eine maximale Einatmung oder eine maximale Ausatmung, innerhalb eines Atemzyklus an diesen Zeitpunkt 4283 zuzuordnen. Wie weiter unten nochmals aufgegriffen, sind derartige maximale Abstände bei Verwendung der Ableitungsunktion einer maximalen Einatmung zuzuordnen. Der nachfolgende minimale Abstand zwischen beiden Ausgleichskurven 4250, 4260, und somit einem lokal minimaler Wert 4254 in der oberen Ausgleichskurve 4250 und lokal maximaler Wert 4264 in der unteren Ausgleichskurve 4260, ist dem Zeitpunkt 4284 der entgegengesetzte maximale Zustand der Atmung zuzuordnen.

Durch die Bestimmung dieser beiden Zeitpunkte 4283 und 4284 kann somit der Zeitraum eines Phasenabschnitts der Atmung abgeleitet werden. Ein solcher Phasenabschnitt ist bei der Zuordnung des diastolischen oder systolischen Blutdruckwerten zu berücksichtigen.

Dabei kommt es nicht darauf an, ob in dem Zeitraum des Phasenabschnitts zuerst ein Maximalwert, dieser insbesondere damit beginnt, und hiernach ein Minimalwert, insbesondere damit endet, vorliegt. Möglich ist es auch, dass aus einer entgegengesetzten Kombination der minimalen und maximalen Abstände der Ausgleichskurven 4260 und 4250 der Phasenabschnitt und dessen Zeitraum gefunden. Hierzu kann ein minimaler Abstand der Ausgleichskurven z.B. zum Zeitpunkt 4285 und den Punkten 4265 auf der unteren Ausgleichskurve 4260 und 4255 auf der oberen Ausgleichskurve 4250 als Beginn gefunden werden. Das Ende dieses Phasenabschnitts bildet ein darauffolgendes lokales Maximum des Abstandes der beiden Ausgleichskurven 4250 und 4260 am Zeitpunkt 4286 zwischen den Punkten 4266 und 4256.

Die Zuordnung eines lokalen maximalen (z.B. 4253 zu 4263 oder 4256 zu 4266) bzw. minimalen (z.B. 4254 zu 4264 oder 4255 zu 4265) Abstands der Ausgleichskurven 4250 und 4260 zu einer maximalen Position innerhalb der Atmung, also maximale Einatmung oder maximale Ausatmung, hängt von der Wahl der Ermittlung der Intensitätskurve ab. Wird beispielsweise die Intensitätskurve mittels eines Bandpassfilters ermittelt, so spiegelt sich in der Intensitätskurve der Blutdruck wider. Folglich ist ein lokal maximaler Abstand der Ausgleichskurven 4250 und 4260 einer maximalen Ausatmung zuzuordnen und umgekehrt ein lokal minimaler Abstand einer maximalen Einatmung. Genau umgekehrt gilt für den Fall, wenn die Intensitätskurve aus einer mathematischen Ableitung hervorgeht.

Folglich ist die Zuordnung dieser beiden Typen der Phasenabschnitte auf den für die Analyse notwendigen ersten 3571 und zweiten Phasenabschnitte 3572 durch die genaue Methode der Ermittlung der Intensitätskurve bestimmt.

Zudem oder alternativ kann auch der zeitliche Abstand zwischen den lokalen Maxima und den lokalen Minima betrachtet werden. Es wird also anstelle des absoluten Wertes der lokalen Maxima und Minima der zeitliche Abstand zwischen zeitlich aufeinanderfolgenden lokalen Maxima oder zeitlich aufeinander folgenden Minima oder der zeitliche Abstand von aufeinanderfolgenden lokalen Minima und Maxima oder Maxima und Minima betrachtet. Hierfür ist es vorteilhaft, wenn die dieser Betrachtung zugrunde liegenden Daten mit einer Datenrate von mindestens 200 Hz erfasst wurden.

Dabei ist ein geringster zeitlicher Abstand, auch Intervall genannt, zwischen lokalen Maxima bzw. lokalen Minima einer maximalen Einatmung und ein größter zeitlicher Abstand zwischen den lokalen Maxima bzw. Minima einer maximalen Ausatmung zuzuordnen.

Diese beiden Arten der Zuordnung einer maximalen Ausatmung bzw. Einatmung mittels absoluter Werte der lokalen Maxima und Minima bzw. mittels der Intervalllänge zwischen den lokalen Maxima bzw. Minima stellen zwei unterschiedliche Phänomene dar, die mittels des erfindungsgemäßen Verfahrens technisch ausgewertet werden können.

Im folgenden Abschnitt wird ein Ausführungsbeispiel näher erläutert:

Bei der Verarbeitung der Daten werden zwei Datensätze benötigt: Intensitätsverlauf der Variationen in der Druckkurve der Manschette und die Informationen zur Atmung. Soll die Atmung direkt aus der Luftdruckkurve bestimmt werden, ist eine schmale Manschette vorteilhaft. Dies benötigt jedoch eine weitere Berechnung, um den Übergang der Ursachen für die Variationen unterscheiden zu können. Im Stand der Technik wird der Intensitätsverlauf der Variationen direkt aus der Druckverlaufskurve mit Hilfe eines geeigneten Bandpassfilters ermittelt. Dies ist auch für die hier vorgestellte Methode eine mögliche Vorgehensweise. Da der absolute Wert der Variationen nicht von Bedeutung ist kann auch die erste mathematische Ableitung der Druckverlaufskurve verwendet werden. Dies hat den Vorteil, dass Informationen nicht durch Filterung verloren gehen. Die Ableitung zeigt für jede Variation ein lokales Maximum und ein lokales Minimum; Die Ableitung an sich (über einen längeren Zeitraum gemittelt betrachtet) zeigt die Druckveränderungsrate, die durch die Pumpe (bei Messung bei steigendem Druck) oder die durch das Ventil (bei Messung bei reduzierendem Druck) vorgegeben wird und sollte durch Pumpe bzw. Ventil einen möglichst gleichbleibenden Wert einnehmen. Der Intensitätsverlauf der Variationen wird hinsichtlich lokaler Minima und Maxima untersucht, es ergibt sich je eine Liste bestehend aus Tupeln der Form Zeit zu Wert der Intensität. Die Information zur Atmung ist eine Liste von Tupeln der Form Zeitpunkt der maximalen Einatmung und Zeitpunkt der maximalen Ausatmung. Aus diesen Werten können die Werte für Zeit der Ein- und Ausatmung, sowie der Atemfrequenz bestimmt werden. Die Information zur Atmung kann entweder durch ein anderes Verfahren bestimmt werden, z.B. Verfahren zur Bestimmung aus EKG, Lichtwellen und Beschleunigungssensordaten sind dem Fachmann bekannt, Verfahren beruhend auf Mikrophon, Ultraschall oder Temperatur sind nicht geläufig, jedoch sind diese in ihrer Auswertung sehr ähnlich zur Auswertung von Verfahren unter Verwendung von Lichtwellen. Vorteilhaft ist jedoch die Bestimmung der Atmung direkt aus der Luftdruckkurve. In Abbildung 4 weißt die Einhüllende (bestehend aus der oberen Ausgleichskurve 4250 und der unteren Ausgleichskurve 4260) des Intensitätsverlauf der Variationen der Luftdruckkurve, welcher durch mathematische Ableitung erhoben wurde, Veränderungen mit der Atmung auf. Mit dem Referenzzeichen 4284 ist ein beispielhafter Zeitpunkt markiert, an dem die Einhüllende einen lokalen minimalen Höhendurchmesser aufweist. Ein solcher Zeitpunkt ist ein Zeitpunkt der maximalen Ausatmung, die Zeitpunkte der lokalen maximalen Höhendurchmesser der Einhüllenden am mit dem Referenzzeichen 4283 beispielhaft markierten Zeitpunkt ist der Zeitpunkt einer maximalen Einatmung. Eine andere Methode ist die Erkennung des Intervalls zwischen zwei aufeinander folgende Variationen. Dieses Intervall nimmt mit Einatmung ab und steigt wieder mit Ausatmung. Beide Methoden der Bestimmung der Atmung sind von Mensch zu Mensch unterschiedlich tauglich, da diese Phänomene nicht immer sichtbar sind. Grundsätzlich gilt für beide Varianten, dass der Zeitpunkt der maximalen Einatmung bzw. der Zeitpunkt der maximalen Ausatmung methodisch bedingt mit einer zeitlichen Fehlerbreite von der Hälfte der Herzpulsintervalls, welches an dem Zeitpunkt der jeweiligen Messpunkte vorliegt, bestimmt werden können.

Idealerweise sind in einem Datensatz beide Phänomene erkennbar und können zur Erhöhung der Genauigkeit miteinander vereint ausgewertet werden. Es gibt jedoch auch Datensätze, in denen eines der beiden Merkmale nicht zur Erkennung der Atmung ausreichend aufgelöst werden kann. Dies ist insbesondere bei einer Datenrate von unter 200 Hz der Fall, da in diesem Fall die Intervalle oft nicht ausreichend aufgelöst werden können. Zunächst werden deshalb beide Merkmale der Phänomene zur Erkennung der Atmung ausgewertet. Danach wird anhand von Gütefaktoren der Ergebnisdaten eingeschätzt, welches Verfahren tauglich ist und welches nicht. Diese Gütefaktoren sind die Periodizität und die Amplitude der Atmungskurve. Die Periodizität ergibt sich aus den mittels des Verfahrens bestimmten Zeitpunkten der Ein- und Ausatmung, bzw. aus den Intervallzeiten der Atmung. Dazu wird ein Variationskoeffizient der Intervallzeiten bestimmt. Der Variationskoeffizient ist geben durch die Standardabweichung geteilt durch den Mittelwert der Intervallzeiten und wird in Prozent angegeben. Bei der Bestimmung aus dem Merkmal des Abstandes zwischen den Ausgleichskurven 4260, 4250 ist die Amplitude durch den Unterschied im Abstand bei Einatmung zum Abstand bei Ausatmung gegeben. Bei der Bestimmung aus dem Merkmal des Intervalls zwischen den Variationen ist die Amplitude durch die Differenz der Intervallzeiten bei Ein- und Ausatmung gegeben.

Hinsichtlich der Schwierigkeit der Bestimmung des Beginns und des Endes des ersten bzw. zweiten Phasenabschnitts wird auf die Figur 6 verwiesen.

In der Figur 5 sind in Abschnitt a) der Figur 5 mittels einer Manschette gemessene Einzelwerte eines Luftdrucks innerhalb eines Messzeitraums als Luftdruckverlaufskurve 5000 über die Zeit dargestellt. Dem Luftdruck kann im Grunde ein Blutdruckwert zugeordnet werden. In der Luftdruckverlaufskurve 5000 ist ein genereller Anstieg des Drucks zu erkennen. Dieser Druckanstieg ist ein Resultat der Manschettenmessung. Denn während der Manschettenmessung wird während des Messzeitraums der von der Manschette auf den Messbereich ausgeübte Druck variiert, in diesem Fall erhöht, und somit eine Druckbeaufschlagung auf den Messbereich an einer Person erfolgt. Dies erfolgt dadurch, dass mehr Luft in die Manschette gepumpt wird. Dabei wird Luft mit einer vorbestimmten Druckbeaufschlagungsänderungsrate in die Manschette gepumpt. In der Abbildung ist ein einfacher Fall einer Druckbeaufschlagung gezeigt, nämlich mit einer konstanten und positiven Druckbeaufschlagungsänderungsrate. Möglich ist jedoch auch eine nicht konstante oder teils positiv und teils negative Druckbeaufschlagungsänderungsrate über den Messzeitraum hinweg. Dabei weist die dann messbaren Druckverlaufskurve 5000 diese variierende (konstant oder nicht-konstant, positive, negativ oder beides) Druckbeaufschlagung auf.

Ungeachtet des Druckanstiegs sind in der Druckverlaufskurve 5000 zusätzlich Variationen 5010 in den gemessenen Luftdrücken messbar.

Aus dieser Druckverlaufskurve 5000 und den Variationen 5010 können neben den mittels, der bevor beschriebenen erfindungsgemäßen Methode mindestens zwei atmungskorrigierten ersten, systolischen und zweiten, diastolischen Einzelwerten des Blutdrucks, hier angedeutet durch die systolischen Blutdruckwerte 5077 und 5078 und die diastolischen Blutdruckwerte 5073 und 5074 jedoch eine erste Vielzahl von atmungskorrigierten systolischen Blutdruckwerten ermittelt werden. Auch ist es möglich eine zweite Vielzahl von atmungskorrigierten diastolischen Blutdruckwerten zu ermitteln. In dem in der Figur 5 dargestellten Beispiel einer erfindungsgemäßen Auswertung von schematischen Luftdruckwerten, soll nun diese beiden letztgenannten Aspekte der weiteren vorteilhaften Auswertemöglichkeit dargestellt werden.

Diese Auswertemöglichkeit beinhaltet zwei Aspekte. Zu meinem wird aufgezeigt, wie kontinuierliche Blutdruckwerte bestimmt werden und zum anderen soll, dies ist jedoch nicht immer notwendig, aufgezeigt werden, wie eine schmale Manschette verwendet werden kann. Hierbei bezieht sich der Ausdruck kontinuierlich auf die Erfassung von Blutdruckwerten über einen längeren Zeitraum in vorzugsweise in kurzen Zeitabständen, bevorzugt für jeden Herzschlag. Des Weiteren bedeutet hierbei schmaler, dass die Datenerfassung mit einer Manschette, die eine geringere Breite als die beim Stand der Technik verwendeten Manschette aufweist, verwendet wird. Eine Manschette zum Gebrauch am Oberarm hat nach dem Stand der Technik eine optimale Breite, wenn diese Breite einem dritte der Länge zwischen Schultergelenk und Ellenbogengelenk aufweist. Wie bereits weiter oben konkretisiert kann eine schmale Manschette insbesondere nur 3 cm breit sein.

Wie bereits weiter oben beschrieben führt eine schmale Manschette bei der Verwendung der Verfahren nach dem Stand der Technik zu Messfehlern. Typischerweise wird in diesem Fall eine zu hohe Bewertung des Blutdrucks, insbesondere der Systole ausgegeben. Dies liegt daran, dass die Druckvariationen, hier gezeigt durch 5010, nicht nur von dem Gewebe und Arterien unterhalb der Manschette ausgelöst werden, sondern auch von direkt angrenzendem Gewebe und Arterien herrühren können. Bei einer breiten und damit optimalen Manschette für den Stand der Technik ist dieser Effekt gering im Verhältnis zu den Druckvariationen, welche durch die Arterien unterhalb der Manschette ausgelöst werden, und wird durch das große Volumen der Manschette mechanisch weiter unterdrückt. Bei schmalen Manschetten ist dieser Effekt jedoch nicht mehr vernachlässigbar und führt dazu, dass auch bei hohen Manschettendrücken Druckvariation zu erkennen sind.

Selbst wenn eine übliche Manschette mit üblichen Manschettenbreiten verwendet wird, kann zwar bspw. mittels eines digitalen Bandpassfilters aus der Luftdruckverlaufskurve 5000 ein oder zweit Einzelwert(e) eins Blutdruckwertes bestimmt werden, jedoch ist es wünschenswert, insbesondere bei geringer Druckbeaufschlagungsänderungsrate, einen kontinuierlichen Blutdruckwert zu ermitteln. Aufgrund der der Riva Rocci und Korotkoff-Methode innewohnende Methode (es werden mehrere Herzpulse zur Bestimmung benötigt), ist eine Bestimmung von kontinuierlichen Blutdruckwerten jedoch mit dieser Methode nicht möglich, da zur Gewinnung der Messwerte gerade ein ausreichend großes Zeitintervall benötigt wird, somit bspw. nicht für jeden Herzpuls ein Blutdruckwert bestimmbar ist.

In der Figur 5 ist nun eine erfindungsgemäße Weiterbildung der erfindungsgemäßen Methode dargestellt, um kontinuierliche Blutdruckwerte aus Einzelwertmessungen einer Manschette des Luftdruckwertes zu erhalten, wobei eine schmale Manschette verwendet wird. Aus diesem Grund werden wie in den Teilabschnitte b) und c) der Figur 5 gezeigt einige weitere Verfahrensschritte benötigt, um aus den Luftdruckwerten kontinuierliche Blutdruckwerte zu erhalten. Sofern eine Manschette mit ausreichender Breite verwendet wird, kann wie erwähnt, bspw. mittels eines Bandpassfilters direkt eine Intensitätskurve ermittelt werden, die dann die Form wie in dem Abschnitt c) der Intensitätskurve 5300 hat.

Die Verwendung einer breiten Manschette gegenüber einer schmalen Manschette hat den Vorteil, dass das Verfahren einfacher ist. Es hat jedoch mehrere Nachteile. Zum einem wird die Intensitätskurve durch das große Volumen zum einem mechanisch gefiltert und zum anderen verwaschen, da die Messfläche groß ist und somit viele Arterien einen Einfluss haben.

Es kann bei einer breiten Manschette auch nicht festgestellt werden, in welchem Zeitraum die Blutdruckkurve in der Luftdruckkurve vollständig abgebildet wird. Aus anderer Sicht ist eine schmale Manschette vorteilhaft, da diese eine geringere Belastung des Nutzers erlaubt und somit eine geringere Druckänderungsrate und somit eine längere Messung und somit eine längere kontinuierliche Ermittlung des Blutdrucks erlaubt.

Im Folgenden wird jedoch auf den Fall wie in Figur 5 dargestellt eingegangen, also bei einer Messung mit einer schmalen Manschette.

Nach der Messung der Einzelwerte und dem Erstellen der Druckverlaufskurve 5000 wird bspw. mittels der mathematischen Ableitungsfunktion die Änderungskurve 5200 bzw. Intensitätskurve 5200 erstellt. Diese Intensitätskurve 5200 stellt die erfassten Werte des Luftdrucks unabhängig von der variierenden Druckbeaufschlagung dar. In dieser Intensitätskurve 5200 sind entlang des Messzeitraums die Variationen 5010 der Druckverlaufskurve 5000 als maximale Werte 5220 und als minimale Werte 5230 der Intensitätskurve 5200 vorhanden und zu erkennen. Die minimalen Werte 5230 sind dabei solche Einzelwerte der Intensitätskurve 5200, die im Vergleich zu dem zeitlich unmittelbar vorangehenden Einzelwert und zu dem zeitlich unmittelbar darauffolgenden Einzelwert die geringsten Einzelwerte aufweisen. Die maximalen Werte 5220 sind dabei solche Einzelwerte der Intensitätskurve 5200, die im Vergleich zu dem zeitlich unmittelbar vorangehenden Einzelwert und zu dem zeitlich unmittelbar darauffolgenden Einzelwert die höchsten Einzelwerte aufweisen.

Diese lokalen Extrema 5220, 5230 sind nun jedoch nicht homogen verteilt. Vielmehr ist zu beobachten, dass ab einem bestimmten Zeitpunkt bzw. bestimmten höherem Druck der Druckbeaufschlagung die lokalen Maxima und ab einem bestimmten Zeitpunkt bzw. bestimmten höherem Druck der Druckbeaufschlagung die lokalen Minima sich deutlich steigern, also die Maxima größer werden und die Minima kleinere Werte aufweisen. Dies ist zum einem der grundlegende Effekt der Druckbeaufschlagung, wie weitere oben beschrieben und zum anderen ein Effekt der schmalen Manschette, da ab diesen Drücken der Druckbeaufschlagung der Effekt der zu schmalen Manschette aufzutreten beginnt, also überhöhte Drücke und Rückschwankungen erfasst werden.

Mit den folgenden Verfahrensschritten können diese Effekte jedoch ausreichend bei der Auswertung berücksichtigt und somit herausgenommen werden. Es wird also eine Korrektur der aufgrund der Manschettenbreite hervorgerufenen Effekte vorgenommen und so eine manschettenbreitekorrigierte Kurve erstellt, die dann bspw. für die Bestimmung von Einzelblutdruckwerten oder wie in der Figur 5 gezeigt für die Bestimmung von kontinuierlichen Blutdruckwerten verwendet werden kann.

In einem ersten Verfahrensschritt werden mehrere, insbesondere drei, untere Annäherungsgeraden 5261, 5262, 5263 und eine drei obere Annäherungsgeraden 5251 5252, 5253 gebildet. Dies wird beispielsweise durch jeweils eine lineare Näherung einer Geraden an die lokalen Extrema umgesetzt.

Dabei wird als Anfangspunkt der ersten oberen Annäherungsgeraden 5251 und der ersten unteren Annäherungsgeraden 5261 vorzugsweise der erste Zeitpunkt innerhalb der Druckverlaufskurve und somit innerhalb des Messzeitraums gewählt, an denen die Variationen 5010 außerhalb des Rauschniveaus liegen, und als Endpunkt der dritten oberen Annäherungsgeraden 5253 und der dritten unteren Annäherungsgeraden 5263 vorzugsweise der letzte Zeitpunkt innerhalb der Druckverlaufskurve gewählt, an denen die Variationen 5010 außerhalb des Rauschniveaus liegen.

Die erste obere Annäherungsgerade 5251 wird derart gebildet und bestimmt, dass für die erste obere Annäherungsgerade 5251 eine Annäherungsgerade zu den in einer ersten Zeitspanne vorliegenden lokalen Maxima 5220 gebildet wird, womit der Beginn 5280 der ersten Zeitspanne durch den Beginn der ersten oberen Annäherungsgeraden 5251 definiert ist. Als ein temporäres Ende der ersten oberen Annäherungsgeraden 5251 und der ersten Zeitspanne wird der Zeitpunkt genommen, ab welchem die Annäherung der ersten oberen Annäherungsgeraden 5251 an die den lokalen Maxima 5220 ein vorbestimmtes Bestimmtheitsmaß unterschreitet.

In einem nächsten Schritt wird die dritte obere Annäherungsgerade 5253 gebildet, welche als eine Annäherungsgerade zu den innerhalb einer dritten Zeitspanne vorliegenden lokalen Maxima 5220 gebildet wird, womit das Ende 5283 der dritten Zeitspanne durch das Ende der dritten oberen Annäherungsgeraden 5253 definiert ist. Als ein temporärer Beginn der dritten oberen Annäherungsgeraden 5253 und der dritten Zeitspanne wird der Zeitpunkt genommen, ab welchem die Annäherung der dritten oberen Annäherungsgeraden 5253 an die den lokalen Maxima 5220 das vorbestimmte Bestimmtheitsmaß unterschreitet.

Ausgehend von dem temporären Ende der ersten Zeitspanne und ersten oberen Annäherungsgeraden 5251 und dem temporären Beginn der dritten Zeitspanne und dritten oberen Annäherungsgeraden 5253 wird eine zweite obere Annäherungsgerade 5252 innerhalb einer zweiten Zeitspanne bestimmt, sodass der temporäre Beginn der zweiten oberen Annäherungsgeraden 5253 und der zweiten Zeitspanne mit dem temporären Ende der ersten oberen Annäherungsgeraden 5251 und das temporäre Ende der zweiten oberen Annäherungsgeraden 5253 und der zweiten Zeitspanne mit dem temporären Beginn der dritten oberen Annäherungsgeraden 5253 übereinstimmt. Iterativ wird daraufhin das temporäre Ende der zweiten oberen Annäherungsgeraden 5252, und somit der temporäre Beginn der dritten oberen Annäherungsgeraden 5253, und der temporäre Beginn der zweiten oberen Annäherungsgeraden 5252, und somit das temporäre Ende der ersten oberen Annäherungsgeraden 5251, verändert, bis die zweite obere Annäherungsgerade 5252 eine Annäherungsgerade ist, die zu den in der zweiten Zeitspanne vorliegenden lokalen Maxima 5220 eine geringste Abweichung aufweist, insbesondere ein vorbestimmtes Bestimmtheitsmaß überschreitet. Sobald diese finale zweite obere Annäherungsgerade 5252 iterativ bestimmt ist, wird das sich hieraus ergebende Ende der ersten oberen Annäherungsgeraden 5251 als finales Ende 5281 der ersten Zeitspanne, das sich hieraus ergebende Ende der zweiten oberen Annäherungsgeraden 5252 als finales Ende 5282 der zweiten Zeitspanne, der sich hieraus ergebende Anfang der zweiten oberen Annäherungsgeraden 5252 als finaler Beginn 5281 der zweiten Zeitspanne und der sich hieraus ergebende Beginn der dritten oberen Annäherungsgeraden 5253 als finaler Beginn 5282 der dritten Zeitspanne bestimmt.

In analoger Weise wird hinsichtlich der unteren Annäherungsgeraden verfahren.

Die erste untere Annäherungsgerade 5261 wird derart bestimmt und gebildet, dass für die erste untere Annäherungsgerade 5261 eine Annäherungsgerade zu den innerhalb einer vierten Zeitspanne vorliegenden lokalen Minima 5230 gebildet wird, womit der Beginn 5285 der vierten Zeitspanne durch den Beginn der ersten unteren Annäherungsgeraden 5261 definiert ist. Als ein temporäres Ende der ersten unteren Annäherungsgeraden 5261 und der ersten Zeitspanne wird der Zeitpunkt genommen, ab welchem die Annäherung der ersten unteren Annäherungsgeraden 5261 an die den lokalen Minima 5230 das vorbestimmtes Bestimmtheitsmaß unterschreitet.

In einem nächsten Schritt wird die dritte untere Annäherungsgerade 5263 gebildet, welche als eine Annäherungsgerade zu den innerhalb einer sechsten Zeitspanne vorliegenden lokalen Minima 5230 gebildet wird, womit das Ende 5288 der sechsten Zeitspanne durch das Ende der dritten unteren Annäherungsgeraden 5263 definiert ist. Als ein temporärer Beginn der dritten unteren Annäherungsgeraden 5263 und der dritten Zeitspanne wird der Zeitpunkt genommen, ab welchem die Annäherung der dritten unteren Annäherungsgeraden 5263 an die den lokalen Minima 5230 das vorbestimmte Bestimmtheitsmaß unterschreitet.

Ausgehend von dem temporären Ende der vierten Zeitspanne und ersten unteren Annäherungsgeraden 5261 und dem temporären Beginn der sechsten Zeitspanne und dritten unteren Annäherungsgeraden 5263 wird eine zweite untere Annäherungsgerade 5262 innerhalb einer fünften Zeitspanne bestimmt, sodass der temporäre Beginn der zweiten unteren Annäherungsgeraden 5263 und der fünften Zeitspanne mit dem temporären Ende der ersten unteren Annäherungsgeraden 5261 und das temporäre Ende der zweiten unteren Annäherungsgeraden 5263 und der fünften Zeitspanne mit dem temporären Beginn der dritten unteren Annäherungsgeraden 5263 übereinstimmt. Iterativ wird daraufhin das temporäre Ende der zweiten unteren Annäherungsgeraden 5262, und somit der temporäre Beginn der dritten unteren Annäherungsgeraden 5263, und der temporäre Beginn der zweiten unteren Annäherungsgeraden 5262, und somit das temporäre Ende der ersten unteren Annäherungsgeraden 5261, verändert, bis die zweite untere Annäherungsgerade 5262 eine Annäherungsgerade ist, die zu den in der zweiten Zeitspanne vorliegenden lokalen Minima 5230 eine geringste Abweichung aufweist, insbesondere ein vorbestimmtes Bestimmtheitsmaß überschreitet. Sobald diese finale zweite untere Annäherungsgerade 5262 iterativ bestimmt ist, wird das sich hieraus ergebende Ende der ersten unteren Annäherungsgeraden 5261 als finales Ende 5286 der vierten Zeitspanne, das sich hieraus ergebende Ende der zweiten unteren Annäherungsgeraden 5262 als finales Ende 5287 der fünften Zeitspanne, der sich hieraus ergebende Anfang der zweiten unteren Annäherungsgeraden 5262 als finaler Beginn 5286 der fünften Zeitspanne und der sich hieraus ergebende Beginn der dritten unteren Annäherungsgeraden 5263 als finaler Beginn 5287 der sechsten Zeitspanne bestimmt.

Hierdurch ergeben sich zu unterschiedlichen Zeitpunkten Differenzen zwischen den unterschiedlichen oberen und den unteren Annäherungsgeraden und somit unterschiedliche Abstände, auch Höhenunterschiede genannt, zwischen den oberen und den unteren Annäherungsgeraden. Als erster Zeitpunkt, an dem der Überhöhungseffekt der schmalen Manschette die Luftdruckwerte in relevanter Weise beeinflusst, ist der Beginn 5281 der zweiten Zeitspanne bzw. der Beginn 5286 der vierten Zeitspanne. Der Grund dafür, dass der Beginn 5281 der zweiten Zeitspanne und der Beginn 5286 der vierten Zeitspanne nicht identisch sind, liegt wohl darin, dass der Effekt vom konkreten Blutdruck innerhalb des Herzzyklus abhängt. Die unterschiedlichen Zeitpunkte 5281 und 5286 kommen daher zustande, dass bei dem hohen Druck des systolischen Herzzyklus der Effekt der (zu) schmalen Manschette schon bereits ab 5281 zum Tragen kommt, während der Effekt bei dem niedrigeren Druck während der diastolischen Phase des Herzzyklus gerade noch nicht auftaucht und erst ab 5286 zum Tragen kommt. Als mögliche Lösung wird daher vorgeschlagen, dass aus dem Beginn 5281 der zweiten Zeitspanne und dem Beginn 5286 der vierten Zeitspanne ein Mittelwert 5289 gebildet wird und der zu diesem Zeitpunkt 5289 des Mittelwerts vorliegende Abstand zwischen oberen und unteren Annäherungsgeraden als eine Normierungsparameter, die Manschettenbreitenkorrekturkonstante verwendet wird.

In einem weiteren Verfahrensschritt werden, um von dem Effekt der Manschettenbreite unabhängige Werte und somit die manschettenbreitenkorrigierte Intensitätskurve 5300 zu erhalten, die zum jeweiligen Zeitpunkt vorliegenden Einzelwerte der Änderungskurve 5200 mit einem Breitenkorrekturfaktor, insbesondere einem Manschettenbreitenkorrekturfaktor multipliziert. Dabei basiert der Manschettenbreitenkorrekturfaktor auf einer normierten Differenz zwischen oberen und unteren Annäherungsgeraden. Die Differenz basiert dabei auf dem Abstand, dem Höhenunterschied, zwischen oberen und unteren Annäherungsgeraden zu dem jeweiligen Zeitpunkt und die Normierung der Differenz auf der Normierungsparameter, der Manschettenbreitenkorrekturkonstante. Diese normierte Differenz, auch Manschettenbreitenkorrekturfaktor genannt, ist insbesondere der Quotient aus dem Höhenunterschied zum jeweiligen Zeitpunkt und Manschettenbreitenkorrekturkonstante, mit der Manschettenbreitenkorrekturkonstante als Divident und dem Höhenunterschied als Divisor des Quotienten. Der Breitenkorrekturfaktor besteht neben dem Manschettenbreitenkorrekturfaktor auch aus einem Manschettenzielfaktor dieser beschreibt die Zielform der Intensitätsschwankung 5300. Die Zielform 0 an den Zeitpunkten 5285 und 5283 und am Zeitpunkt 5289 1. Zwischen diesen drei Zeitpunkten ist die Zielform je abschnittsweise linear. Zusammenfassend ergibt sich ein Wert der Kurve 5300 zu einem Zeitpunkt aus dem Wert zu diesem Zeitpunkt der Kurve 5200 multipliziert mit dem Manschettenbreitenkorrekturfaktor zu diesem Zeitpunkt multipliziert mit dem Manschettenzielfaktor zu diesem Zeitpunkt.

Aus den so erhaltenen Einzelwerten wird die vorrichtungsbreitekorrigierte Intensitätskurve 5300 gebildet, die mehre lokale Maxima 5320 und mehrere lokale Minima 5330 aufweist, also Einzelwerte, die im Vergleich zu den benachbarten Einzelwerten geringere bzw. größere Einzelwerte aufweisen.

Aus dieser vorrichtungsbreitekorrigierte Intensitätskurve 5300 könnten zumindest relative kontinuierliche Blutdruckwerte bestimmt werden. So könnten Änderungen im Laufe der Zeit bestimmt werden und diese relativen Änderungen verwendet werden, bspw. um Zustandsänderungen nachzuverfolgen. Auch ist es möglich aus dieser vorrichtungsbreitekorrigierte Intensitätskurve 5300 nicht-atmungskorrigierte Absolutwerte des Blutdrucks zu erhalten. Hierfür müsste die dieser vorrichtungsbreitekorrigierte Intensitätskurve 5300 noch skaliert werden. Da es jedoch Aufgabe der Erfindung ist insbesondere atmungskorrigierte Blutdruckwerte anzugeben können aus dieser vorrichtungsbreitekorrigierte Intensitätskurve 5300 atmungskorrigierte Blutdruckeinzelwerte bestimmt werden. Dies kann in analoger Weise wie zu Figur 3 und gegebenenfalls Figur 4 beschrieben erfolgen.

Da eine vorteilhafte Weiterbildung die Bereitstellung von kontinuierlichen Blutdruckwerten darstellt, werden wie nachfolgend beschrieben aus dieser vorrichtungsbreitekorrigierte Intensitätskurve 5300 nun jedoch kontinuierliche Blutdruckwerte ermittelt. Hierfür wird vor allem eine zweite obere Näherungsgerade 5352 und eine zweite unteren Näherungsgerade 5362 ermittelt.

Dabei stellt die zweite obere Näherungsgerade 5352 eine, insbesondere lineare, Näherung an die lokalen Maxima 5320 der vorrichtungsbreitekorrigierte Intensitätskurve 5300 dar, nämlich ausgehend vom Ende der vorrichtungsbreitekorrigierte Intensitätskurve 5300, welches den Luftdruckwerten bei hohen Druckbeaufschlagungen entspricht. Die zweite obere Näherungsgerade 5352 stellt dabei vorzugsweise eine Näherung bis zum Zeitpunkt des Mittelwerts 5289 dar. Die zweite untere Näherungsgerade 5362 stellt eine, insbesondere lineare, Näherung an die lokalen Minima 5330 der vorrichtungsbreitekorrigierte Intensitätskurve 5300 dar, nämlich ausgehend vom Ende der vorrichtungsbreitekorrigierte Intensitätskurve 5300, welches den Luftdruckwerten bei hohen Druckbeaufschlagungen entspricht. Die zweite untere Näherungsgerade 5362 stellt dabei vorzugsweise eine Näherung bis zum Zeitpunkt des Mittelwerts 5289 dar.

Dieses Vorgehen (lediglich den Bereich 5472 bei hohen Druckbeaufschlagungen zu betrachten) liefert in der Regel und insbesondere bei guten Sensoren für 90% dieses Zeitbereichs der zweiten Näherungsgeraden (Einhüllenden in diesem Zeitbereich bei hohen Drücken) ein verwendbares Ergebnis.

Anzumerken ist, dass auch direkt von 5200 auf 5400 umgeformt werden kann, indem der Manschettenzielfaktor bzw. Manschettenkorrekturfaktor nicht verwendet wird. Das hier beschriebene Verfahren ist daher notwendig, wenn von einer nach dem Stand der Technik optimal breiten Manschette auf die Kurven 5472 und 5471 gefolgert werden soll. Das Signal der Luftdruckverlaufskurve 5000 und der daraus ermittelten vorrichtungsbreitenkorrigierten Intensitätskurve 5300 im Bereich 5471 zu niedrigeren Drücken bzw. Druckbeaufschlagungen, also bis zum Zeitpunkt 5289, kann nur eingeschränkt medizinisch betrachtet werden, da in diesem Bereich die Blutdruckkurve gegebenenfalls und unter Umständen nicht vollständig in der Luftdruckkurve abgebildet wird. Dies ist in der Regel bei höheren Drücken nicht der Fall, vielmehr sind in der Regel in diesem Bereich die Blutdruckwerte vollständig in der Luftdruckkurve abgebildet.

Gleichwohl ist es auch möglich die Werte bei niedrigeren Druckbeaufschlagungen zu verwenden. Hierfür ist es möglich eine erste obere Näherungsgerade 5351 und eine erste untere Näherungsgerade 5361 zu ermitteln. Dabei stellt die erste obere Näherungsgerade 5351 eine, insbesondere lineare, Näherung an die lokalen Maxima 5320 der vorrichtungsbreitekorrigierte Intensitätskurve 5300 dar, nämlich ausgehend vom Anfang der vorrichtungsbreitekorrigierte Intensitätskurve, welches den Luftdruckwerten bei niedrigen Druckbeaufschlagungen entspricht. Die erste obere Näherungsgerade 5351 stellt dabei vorzugsweise eine Näherung bis zum Zeitpunkt des Mittelwerts 5289 dar. Die erste untere Näherungsgerade 5361 stellt eine, insbesondere lineare, Näherung an die lokalen Minima 5330 der vorrichtungsbreitekorrigierte Intensitätskurve 5300 dar, nämlich ausgehend vom Anfang der vorrichtungsbreitekorrigierte Intensitätskurve, welches den Luftdruckwerten bei niedrigen Druckbeaufschlagungen entspricht. Die erste untere Näherungsgerade 5361 stellt dabei vorzugsweise eine Näherung bis zum Zeitpunkt des Mittelwerts 5289 dar.

Hierdurch ergeben sich zu unterschiedlichen Zeitpunkten Differenzen zwischen den unterschiedliche den oberen und den unteren Annäherungsgeraden und somit unterschiedliche Abstände, auch Höhenunterschiede genannt, zwischen den oberen und den unteren Näherungsgeraden 5351, 5352, 5361, 5362.

In einem weiteren Verfahrensschritt werden, um kontinuierliche Blutdruckwerte ermitteln zu können und somit zunächst eine kontinuierliche, relative Blutdruckverlaufskurve 5400 zu erhalten, die zum jeweiligen Zeitpunkt vorliegenden Einzelwerte der vorrichtungsbreitekorrigierte Intensitätskurve 5300 normiert. Die Normierung basiert dabei auf den zum jeweiligen Zeitpunkt vorliegenden Höhenunterschiede zwischen den oberen und den unteren Näherungsgeraden 5351, 5352, 5361, 5362. Im einfachsten Fall wird der zum jeweiligen Zeitpunkt vorliegenden Einzelwert der vorrichtungsbreitekorrigierte Intensitätskurve 5300 durch den zu diesem Zeitpunkt vorliegenden Höhenunterschied geteilt. Hierdurch schwanken die lokalen Maxima in der so normierten vorrichtungsbreitekorrigierte Intensitätskurve um eine auf der ersten und/oder zweiten oberen Näherungsgerade basierende obere Näherungsgerade 5450, die durch die Normierung nun flach verläuft. Auch schwanken dann die lokalen Minima in der so normierten vorrichtungsbreitenkorrigierten Intensitätskurve um eine auf der ersten und/oder zweiten unteren Näherungsgerade basierende untere Näherungsgerade 5460, die durch die Normierung nun flach verläuft.

Diese normierten Einzelwerte für die jeweiligen Zeitpunkte, die die relative Blutdruckverlaufskurve 5400 darstellen, werden daraufhin mit einer Skalierungskonstante skaliert.

Die Skalierung basiert dabei auf den Unterschieden zwischen den normierten Einzelwerten in der relativen Blutdruckverlaufskurve und den absoluten atmungskorrigierten Blutdruckwerten. Sofern also die atmungskorrigierten ersten, also systolischen Blutdruckwerte als Basis verwendet werden soll, wie auch in der Figur 5 dargestellt, werden die Werte für die Systole 5077 und 5078, die nach dem vorbeschriebenen Verfahren bestimmt wurden, verwendet. Diese Werte der Systole beschreiben den höchsten und niedrigsten Wert innerhalb der Atmung, dabei sind die Werte zu einem Zeitpunkt, insbesondere zu einem Herzschlag, bestimmt worden. Zudem werden die normierten Einzelwerte zu diesen beiden Zeitpunkten verwendet, also die normierten Einzelwerte 5477, 5478, die zu dem ersten 5087 und zweiten Zeitpunkt 5088 aus der relative Blutdruckverlaufskurve 5400 bestimmt werden können. Neben dieser Skalierung bedarf es noch einer Korrektur im absoluten Wertbereich, da aufgrund der Verwendung der mathematischen Ableitung jeglicher Zusammenhang zu einem absoluten Wert abhandengekommen ist, dieser "Offset"-Fehler jedoch hingenommen wird und wie folgt wieder herausgerechnet wird. Hierfür wird vorgeschlagen und in der Figur 5 so durchgeführt, dass ein Mittelwert aus den atmungskorrigierten systolischen und diastolischen Blutdruckwerten verwendet wird, also ein Mittelwert aus den vier Werten genommen wird. Hintergrund ist dabei der Folgende:

Der Offsetfehler bezieht sich auf die Werte der Diastole und ist die Hälfte der Veränderung des Pulsdrucks während der Messung. Sofern beispielsweise als Werte für atmungskorrigierte Diastole die Werte 78 mmHg und 82 mmHg bestimmt wurden und zudem zu diesem Zeitpunkt eine Systole vorliegt (die man mit der Einzelwertbestimmung aber zu diesem Zeitpunkt nicht messen kann) von 118/122 mmHg vorläge, so ist der Pulsdruck 40 mmHg. Zu einem anderen Zeitpunkt des Messzeitraums, an dem die Systole messbar ist in einer Einzelwertbestimmung (vgl. Figur 3) hat sich jedoch der Blutdruck verändert und es sind erfindungsgemäß lediglich die Werte 128 mmHg und 132 mmHg bestimmbar. Es ist anzunehmen, dass die Diastole, die nun nicht messbar ist, an diesem Zeitpunkt auch gestiegen ist, sodass der Pulsdruck unverändert bleibt. Ausgehend von der erfindungsgemäßen Bestimmung ergäbe sich nun jedoch ein falscher Pulsdruck von 50 mmHg. Dieser um 10 mmHg zu hohe Pulsdruck führt zu einem um 5 mmHg zu hohe Wert für die kontinuierlichen Werte der Diastole.

Grundlage sind die dem atmungskorrigierten Blutdruckwert zugrunde liegenden Blutdruckwerte bei Ein- und Ausatmung hier 5077 und 5078 und der Blutdruckmittelwert der vier atmungskorrigierten ersten und zweiten Blutdrücke 5073, 5074, 5077, 5078.

Die Blutdruckwerte 5077, 5078 beziehen sich auf den systolischen Blutdruck zu den Zeitpunkten 5087 bzw. 5088. Es wäre jedoch auch möglich anstelle dessen die atmungskorrigierten diastolischen Blutdruckwerte zu verwenden. In der relativen Blutdruckverlaufskurve 5400 können zu diesen Zeitpunkten die normierten Einzelwerte 5477 und 5478 bestimmt werden. Um nun die absoluten, kontinuierlichen atmungskorrigierten Blutdruckwerte zu erhalten, wird ein jeder Einzelwert in der relativen Blutdruckverlaufskurve 5400 linear verschoben, um die Werte für die Blutdruckverlaufskurve 5500 zu erhalten, wobei EW für Einzelwert, *BDVK* für Blutdruckverlaufskurve, BDW für Blutdruckwert, *atmk.* für atmungskorrigiert, *syst.* für Systole bzw. systolisch und *diast.* für Diastole bzw. diastolisch stehen:

$$\text{Werte der BDVK 5500 (als Funktion der Zeit)}$$

$$= \frac{\text{EW (für alle Zeiten)der relativen BDVK 5400}}{\text{normierte EW 5478} - \text{normierter EW 5477}}$$

$$* \,(\text{atmk. syst. BDW 5078} - \text{atmk. syst. BDW 5077})$$

$$+ \;0{,}25\,(\text{atmk. syst. BDW 5078} + \text{atmk. syst. BDW 5077}$$

$$+ \text{atmk. diast. BDW 5073} + \text{atmk. diast. BDW 5074})$$

Diese Blutdruckverlaufskurve 5500 weist lokale Maxima und lokale Minima auf, wobei kontinuierliche systolische Blutdruckwerte 5495, 5496 durch die lokalen Maxima der normierten und skalierten vorrichtungsbreitenkorrigierten Intensitätskurve, also der Blutdruckverlaufskurve 5500 gebildet und hierdurch bestimmt werden können.

Ferner sind auch kontinuierliche diastolische Blutdruckwerte 5493, 5494 ableitbar. Diese werden durch die lokalen Minima der normierten und skalierten vorrichtungsbreitenkorrigierten Intensitätskurve, also der Blutdruckverlaufskurve 5500 gebildet.

In der Figur 6 sind in den drei Teilbereiche (a), (b) und (c) drei verschieden Szenarien hinsichtlich einer Atmungskurve 3500 dargestellt, die sich nach der Ermittlung eines diastolischen nicht-atmungskorrigierten Blutdruckwertes zu dem zweiten Referenzzeitpunkt 3181 ergeben kann. Im Teilbereich (a) ist ein Idealfall dargestellt. In diesem Fall ist kein Zeitversatz zwischen den erfassten Zeitpunkten der maximalen Einatmung 3583 und der maximalen Ausatmung 3584 innerhalb des zweiten Phasenabschnitts 3571 vorhanden, bspw. da die Atmungskurve aus den Einzelwertmessungen ermittelt wurde, die auch Grundlage für die Bildung der Blutdruckwerte bilden.

In Teilbereich (b) ist ein zweites Szenario dargestellt. In diesem Fall ist der zweite Referenzzeitpunkt 3181, an dem der Blutdruckwert des diastolischen nicht atmungskorrigierten Blutdruckwertes ermittelt wurde, im Vergleich zum Idealfall, ein späterer Zeitpunkt. Dies ist nicht ungewöhnlich, da die Bestimmung des nicht atmungskorrigierten Blutdruckwertes nach dem Stand der Technik derartige Ungenauigkeiten beinhaltet. Problematisch ist nun jedoch, sofern die verwendete Atmungskurve 3500 und die damit verbundenen Zeitpunkte der maximalen Einatmung 3583 und der maximalen Ausatmung 3584 einen Zeitversatz zu den Zeitpunkten, die der Bestimmung der Blutdruckwerte zugrunde gelegt werden, aufweisen. In einem solchen Fall wäre der Zeitpunkt 3181 außerhalb eines Phasenabschnitts, der allein auf Basis der Zeitpunkte der maximalen Einatmung 3583 und der maximalen Ausatmung 3584 bestimmt wird. Aus diesem Grund basiert das Ende des zu verwenden Phasenabschnitts 3571 zwar auf den Zeitpunkt der erfassten maximalen Ausatmung 3584, jedoch wird ein gewichteter Zeitmittelpunkt 3589 zwischen dem Zeitpunkt der erfassten maximalen Ausatmung 3584 und dem Zeitpunkt der nachfolgenden erfassten maximalen Einatmung verwendet. Wenn der Zeitpunkt 3181 nicht auf einen passenden Phasenabschnitt fällt, wird also bspw. ein gewichteter Zeitmittelpunkt 3589 zwischen der maximalen Einatmung und Ausatmung (zu dem nicht passenden gefundenen Phasenabschnitt) ermittelt. Je nach Position des Zeitpunkts 3181 zum Zeitmittelpunkt 3589 wird der passende Phasenabschnitt davor oder danach zur weiteren Verarbeitung verwendet.

In dem Szenario, das dem Teilbereich (b) zugrunde gelegt wurde, befindet sich der Zeitpunkt 3181 noch vor dem gemittelten Zeitmittelpunkt 3589. In dem Szenario, das dem Teilbereich (c) zugrunde gelegt wurde, befindet sich der Zeitpunkt 3181 nach dem gemittelten Zeitmittelpunkt 3589, sodass der nachfolgende Phasenabschnitt verwendet wird.

In der Figur 7 ist schematisch ein Druckverlauf 7000 des Blutes in einer Arterie bei sich verändernden Belastungszustand, hier als Beispiel die Erholung dargestellt. Je nachdem in welcher zeitlichen Betrachtung der Druck analysiert wird, können die einzelnen Einflüsse auf den Druck erkannt werden. Wird mit einer zeitlich hohen Auflösung die Kurve analysiert, so ist die Blutdruckveränderung 7100 aufgrund des Herzpulses zu erkennen und es sind Variationen 7010, nämlich Maxima 7020 und Minima 7030 erkennbar. Bei mittlerer zeitlicher Auflösung (angedeutet mit der gestrichelten Linie 7200) ist der Einfluss der Atmung auf den Druck zu erkennen und bei geringer zeitlicher Auflösung (angedeutet mit der gestrichelten Linie 7300) ist der Einfluss der Belastung zu erkennen. Ein Einflussfaktor wirkt in zweierlei Formen auf den Blutdruck. Zunächst verändert ein Einflussfaktor den Blutdruck direkt. Der Einfluss Puls führt zu einer Änderung mit dem Herzpuls, der Einfluss Atmung führt zu einer Änderung mit dem Atemzyklus und der Einfluss Belastung führt zu einer Änderung mit dem Belastungszustand. Ein Einflussfaktor wirkt auch auf die anderen Einflussfaktoren und wirkt somit indirekt auf den Blutdruck. Bei hoher Belastung steigt die Herz- und Atemfrequenz (nach links steigend). Bei steigender Belastung wird auch der Einfluss der Atmung auf den Blutdruck geringer. Dies kann mehrere Gründe haben. Ein typischer Grund ist, dass unter Belastung flacher geatmet wird. Andererseits führt ein hoher Grunddruck dazu, dass die Arterienwände steifer werden und die Druckerhöhung aufgrund der Atmung nur noch geringer ausfällt. Auch die Atmung hat einen Einfluss auf den Puls bei Einatmung steigt die Pulsfrequenz und bei Ausatmung fällt diese.

Referenzzeichen:

1000, 2000, 3000, 4000, 5000, 7000: Verlaufskurve der Einzelwerte der Einzelwertmessungen, nämlich Luftdruckverlaufskurve;

1010, 2010, 3010, 4010, 5010, 7010: Variationen in der Luftdruckverlaufskurve;

1100, 2100, 3100 druckbeaufschlagungskorrigierte Intensitätskurve der Luftdruckverlaufskurve, erhalten durch entweder einen geeigneten Filter, bspw. Bandpassfilter oder durch Abzug einer Ausgleichsgeraden;

4200, 5200: Intensitätskurve der Luftdruckverlaufskurve, erhalten durch die erste mathematische Ableitung über die Zeit;

1110, 2110, 3110, 4210, 5210: Variationen in der Intensitätskurve;

2111, 2112, 2113: erste, zweite bzw. dritte Gruppe in den Variationen;

3120, 3130: lokale Maxima bzw. Minima in der druckbeaufschlagungskorrigierten Intensitätskurve;

3150, 3160: obere Ausgleichskurve an die lokalen Maxima in der druckbeaufschlagungskorrigierten Intensitätskurve bzw. untere Ausgleichskurve an die lokalen Minima in der druckbeaufschlagungskorrigierten Intensitätskurve;

3170: Punkt des maximalen Abstands zwischen der oberen Ausgleichskurve und unteren Ausgleichskurve;

3171 : Punkt des Abstands zwischen der oberen Ausgleichskurve und unteren Ausgleichskurve, der um ein vorbestimmtes Verhältnis kleiner ist, als der Punkt 3170 hin zu geringeren Drücken zur Bestimmung des diastolischen Blutdruckwertes nach dem Stand der Technik;

3172: Punkt des Abstands zwischen der oberen Ausgleichskurve und unteren Ausgleichskurve, der um ein vorbestimmtes Verhältnis kleiner ist, als der Punkt 3170 hin zu höheren Drücken zur Bestimmung des systolischen Blutdruckwertes nach dem Stand der Technik;

3181 : Zeitpunkt des Punkt 3171, auch Referenzzeitpunkt, teils zweiter Referenzzeitpunkt;

3182: Zeitpunkt des Punkt 3172, auch Referenzzeitpunkt, teils erster Referenzzeitpunkt;

3091: Blutdruckwert nach dem Stand der Technik bestimmt anhand der Luftdruckverlaufskurve zum Zeitpunkt 3181 ;

3092: Blutdruckwert nach dem Stand der Technik bestimmt anhand der Luftdruckverlaufskurve zum Zeitpunkt 3182;

3500: Atmungskurve;

3520: lokale Maxima in der Atmungskurve;

3530: lokale Minima in der Atmungskurve;

3571: zweiter Phasenabschnitt;

3572: erster Phasenabschnitt;

3583: erster Zeitpunkt des zweiten Phasenabschnitts als Zeitpunkt der maximalen Einatmung im zweiten Phasenabschnitt;

3584: zweiter Zeitpunkt des zweiten Phasenabschnitts als Zeitpunkt der maximalen Ausatmung im zweiten Phasenabschnitt;

3585: zweiter Zeitpunkt des ersten Phasenabschnitts als Zeitpunkt der maximalen Ausatmung im ersten Phasenabschnitt;

3586: erster Zeitpunkt des ersten Phasenabschnitts als Zeitpunkt der maximalen Einatmung im ersten Phasenabschnitt;

3589: Ende eines Phasenabschnitts;

3093: Blutdruckwert nach dem hier erfindungsgemäßen Verfahren bestimmt anhand der Luftdruckverlaufskurve zum Zeitpunkt 3583;

3094: Blutdruckwert nach dem hier erfindungsgemäßen Verfahren bestimmt anhand der Luftdruckverlaufskurve zum Zeitpunkt 3584;

3095: Blutdruckwert nach dem hier erfindungsgemäßen Verfahren bestimmt anhand der Luftdruckverlaufskurve zum Zeitpunkt 3585;

3096: Blutdruckwert nach dem hier erfindungsgemäßen Verfahren bestimmt anhand der Luftdruckverlaufskurve zum Zeitpunkt 3586;

4220: lokale Maxima der Variationen in der Intensitätskurve der Luftdruckverlaufskurve;

4230: lokale Minima der Variationen in der Intensitätskurve der Luftdruckverlaufskurve;

4250: obere Ausgleichskurven an die lokalen Maxima in der Intensitätskurve;

4253, 4256: lokal maximaler Wert in der ersten, oberen Ausgleichskurve;

4254, 4255: lokal minimale Werte in der ersten, oberen Ausgleichskurve;

4260: zweite, untere Ausgleichskurven an die lokalen Minima in der Intensitätskurve;

4263,4266: lokal minimale Wert in der zweiten, unteren Ausgleichskurve;

4264, 4265: lokal maximale Werte in der zweiten, unteren Ausgleichskurve;

4283, 4286: Zeitpunkte der lokalen maximalen Werte in der ersten, oberen Ausgleichskurve und des lokal minimalen Werts in der zweiten, unteren Ausgleichskurve;

4284, 4285: Zeitpunkte der lokal minimalen Werte in der ersten, oberen Ausgleichskurve und der lokal maximalen Werte in der zweiten, unteren Ausgleichskurve;

5073,5074: Ergebnisse für die zwei erfindungsgemäßen atmungskorrigierten zweiten, diastolischen Blutdrucke, nämlich bei maximaler Ein- und Ausatmung;

5077, 5078: Ergebnisse für die zwei erfindungsgemäßen atmungskorrigierten ersten, systolischen Blutdrucke, nämlich bei maximaler Ein- und Ausatmung;

5087, 5088: Zeitpunkte der zwei erfindungsgemäß atmungskorrigierten ersten, systolischen Blutdruckwerte in der Luftdruckverlaufskurve;

5220: lokale Maxima in der Intensitätskurve;

5230: lokale Minima in der Intensitätskurve;

5251: erste obere Annäherungsgerade;

5252: zweite obere Annäherungsgerade;

5253: dritte obere Annäherungsgerade;

5261: erste untere Annäherungsgerade;

5262: zweite untere Annäherungsgerade;

5263: dritte untere Annäherungsgerade;

5280, 5281: Beginn und Ende der ersten Zeitspanne;

5281, 5282: Beginn und Ende der zweiten Zeitspanne;

5282, 5283: Beginn und Ende der dritten Zeitspanne;

5285, 5286: Beginn und Ende der vierten Zeitspanne;

5286, 5287: Beginn und Ende der fünften Zeitspanne;

5287, 5288: Beginn und Ende der sechsten Zeitspanne;

5289: Mittelwert der Zeiten zwischen Beginn der zweiten Zeitspanne und Beginn der vierten Zeitspanne;

5300: Vorrichtungsbreitenkorrigierte Intensitätskurve;

5320: lokale Maxima in der vorrichtungsbreitenkorrigierten Intensitätskurve;

5330: lokale Minima in der vorrichtungsbreitenkorrigierten Intensitätskurve;

5351, 5352: erste bzw. zweite obere Näherungsgerade;

5361, 5362: erste bzw. zweite untere Näherungsgerade;

5400: normierte vorrichtungsbreitenkorrigierte Intensitätskurve;

5477, 5478: normierte Einzelwerte an den Maxima der vorrichtungsbreitenkorrigierten Intensitätskurve zu den Zeitpunkten der zwei erfindungsgemäß atmungskorrigierten ersten, systolischen Blutdruckwerte in der Luftd ru ckverlaufsku rve;

5473, 5474: normierte Einzelwerte an den Minima der vorrichtungsbreitenkorrigierten Intensitätskurve zu den Zeitpunkten der zwei erfindungsgemäß atmungskorrigierten ersten, systolischen Blutdruckwerte in der Luftdruckverlaufskurve;

5450: obere Näherungsgerade;

5460: untere Näherungsgerade;

5471: Bereich in dem die Blutdruckkurve aufgrund der Druckverhältnisse in der Manschette keine vollständige Abbildung des Blutdruckverlaufs in den Arterien unterhalb der Manschette ermöglichen;

5472: Bereich in dem die Blutdruckkurve in der Regel fehlerfrei abgebildet werden kann;

5495, 5496: erste Vielzahl der kontinuierlichen, atmungskorrigierten ersten, systolische Blutdruckwerte;

5493, 5494: zweite Vielzahl der kontinuierliche, atmungskorrigierten zweiten, diastolische Blutdruckwerte;

7020, 7030: lokale Maxima und Minima in der Druckverlaufskurve;

7100: Kurve der Luftdruckverlaufskurve;

7200: Kurve über die lokalen Maxima, als Anzeiger der der Auswirkung der Atmung auf die Luftdruckverlaufskurve;

7300: Kurve über den gesamten Verlauf der Luftdruckverlaufskurve als Anzeiger der Auswirkung der Belastungsänderung auf die Luftdruckverlaufskurve;

## Patentansprüche

1. Verfahren zum Ermitteln von mindestens zwei atmungskorrigierten ersten Blutdruckwerten einer Person,

wobei mehrere Einzelwertmessungen (1000, 2000, 3000, 4000, 5000) eines dem Blutdruckwert der Person zugeordneten Wertes innerhalb eines Messzeitraums zeitaufgelöst mittels eines ersten Sensors einer ersten Vorrichtung erfasst werden, wobei innerhalb des Messzeitraums die erste Vorrichtung eine auf einen als Messbereich verwendeter Teil der Person variierende Druckbeaufschlagung mit einer über die Zeit des Messzeitraums vorbestimmte Druckbeaufschlagungsänderungsrate durchführt und die Werte der Einzelwertmessungen die variierende Druckbeaufschlagung zumindest teilweise aufweisen,

wobei aus den Einzelwertmessungen ein nicht-atmungskorrigierter erster Blutdruckwert (3092, 3093, 5092) der Person bestimmt wird, wobei als ein erster Referenzzeitpunkt (3181, 3182) der Zeitpunkt des nicht-atmungskorrigierten ersten Blutdruckwerts innerhalb des Messzeitraums bestimmt wird,

wobei ein erster Phasenabschnitt (3571, 5372) einer Atmung der Person aufweisend einen Maximalwert (3520), insbesondere eine maximale Einatmung, in einer Atmungskurve (3500) zu einem ersten Zeitpunkt (3583, 3586) des ersten Phasenabschnitts und einen Minimalwert (3530), insbesondere eine maximale Ausatmung, in der Atmungskurve (3500) zu einem zweiten Zeitpunkt des ersten Phasenabschnitts innerhalb des Messzeitraums zeitaufgelöst erfasst wird, wobei der erste Phasenabschnitt so gewählt wird, dass der erste Referenzzeitpunkt innerhalb des den ersten Phasenabschnitt umfassenden Zeitraums fällt, und

wobei zur Bestimmung des atmungskorrigierten Blutdruckwerts (3093, 3094, 3095, 3096; 5073, 5074, 5077, 5078, 5593, 5594, 5595, 5596) der in den Einzelwertmessungen dem ersten Zeitpunkt des ersten Phasenabschnitts zugeordnete Blutdruckwert (3093, 3096, 5074, 5078) oder einem auf Basis des zum ersten Zeitpunkt des ersten Phasenabschnitts zugeordneten Blutdruckwerts ermittelten Blutdruckwert (3093, 3096, 5074, 5078) als der erste der mindestens zwei atmungskorrigiertem ersten Blutdruckwerte (3093, 3096, 5074, 5078), insbesondere der maximalen Einatmung, und der in den Einzelwertmessungen dem zweiten Zeitpunkt des ersten Phasenabschnitts zugeordnete Blutdruckwert (3094, 3095, 5073, 5077) oder einem auf Basis des zum zweiten Zeitpunkt des ersten Phasenabschnitts zugeordnete Blutdruckwerts ermittelten Blutdruckwert (3094, 3095, 5073, 5077) als der zweite der mindestens zwei atmungskorrigierten ersten Blutdruckwert, insbesondere der maximalen Ausatmung, bestimmt werden.

2. Verfahren nach Anspruch 1 **dadurch gekennzeichnet, dass** der Wert der Einzelmessungen ein Druckwert, insbesondere ein Luftdruckwert oder ein auf Basis einer Messung einer, insbesondere durch den Piezoeffekt verursachten, elektrischen Spannung oder des elektrischen Widerstandes ermittelter Druckwert, ist und der erste Sensor einen Drucksensor, insbesondere einen Luftdrucksensor, aufweist, wobei die erste Vorrichtung insbesondere eine, insbesondere automatisierte, Druckmanschette ist, und dass der mindestens eine Phasenabschnitt mittels eines zweiten Sensors, insbesondere einer zweiten Vorrichtung, bestimmt wird, wobei der zweite Sensor oder die zweite Vorrichtung Teil eines EKG ist oder ein Impedanzsensor, ein Lichtwellen-basierender Sensor, insbesondere als Teil eines PPG- oder SpO2-Messgeräts, ein Mikrophon, ein Beschleunigungssensor, ein Ultraschallsensors oder ein Temperatursensors ist.

3. Verfahren nach Anspruch 1 **dadurch gekennzeichnet, dass** der Wert der Einzelmessungen ein Druckwert, insbesondere ein Luftdruckwert oder ein auf Basis einer Messung einer, insbesondere durch den Piezoeffekt verursachten, elektrischen Spannung oder des elektrischen Widerstandes ermittelter Druckwert, ist und der erste Sensor einen Drucksensor, insbesondere einen Luftdrucksensor, aufweist, wobei die erste Vorrichtung insbesondere eine, insbesondere automatisierten, Druckmanschette ist, und dass der mindestens eine Phasenabschnitt (3571, 3572), insbesondere der erste und zweite Zeitpunkt, aus den Werten der Einzelwertmessungen bestimmt werden.

4. Verfahren nach einem der voranstehenden Ansprüche **dadurch gekennzeichnet, dass** aus den Werten der Einzelwertmessungen mittels einer Regressionsgeraden oder mittels eines Bandpassfilter zur Korrektur der variierenden Druckbeaufschlagung eine druckbeaufschlagungskorrigierte Intensitätskurve (3100, 5300) bestimmt wird.

5. Verfahren nach einem der voranstehenden Ansprüche **dadurch gekennzeichnet, dass** innerhalb des Messzeitraums im zeitlichen Verlauf vorliegende Änderungen der Werte des ersten Sensors aus den Werten der Einzelwertmessungen als Änderungsverlaufskurve (4200), insbesondere ermittelt aus der mathematischen Ableitung der Werte der Einzelwertmessungen im zeitlichen Verlauf, einer, insbesondere Bandpass-, Filterung oder einem Abzug einer Ausgleichsgeraden, bestimmt werden, wobei aus der Änderungsverlaufskurve (4200) der erste und zweite Zeitpunkt (3583, 3584, 3585, 3586, 4283, 4284, 4285, 4286), der Phasenabschnitt (3571, 3572) und/oder die Zeitspanne des Phasenabschnitts, insbesondere der Beginn und das Ende des Phasenabschnitts (3571, 3572), bestimmt werden.

6. Verfahren nach dem voranstehenden Anspruch und dem Anspruch 3
**dadurch gekennzeichnet, dass** für die Bestimmung des, insbesondere des ersten, Phasenabschnitts (3571, 3572) aus der zeitlichen Änderungsverlaufskurve (4200,) mehrere lokale Extrema (4210), nämlich lokale Maxima (4220) und lokale Minima (4230), ermittelt werden,

wobei für die Bestimmung des Phasenabschnitts, insbesondere der Phasenabschnitte, (3571, 3572), insbesondere des ersten und zweiten Phasenabschnitts je, eine erste und eine zweite Ausgleichskurve (4250, 4260) ermittelt werden, wobei die erste Ausgleichskurve (4250) eine, basierend auf den Punkten der lokalen Maxima (4220) gebildete, Ausgleichskurve ist und wobei die zweite Ausgleichskurve (4260) eine, basierend auf den Punkten der lokalen Minima (4230) gebildete, Ausgleichskurve ist, wobei mehrere lokale maximale Abstände (4253, 4263; 4256, 4266) und mehrere lokale minimale Abstände (4254, 4264; 4255, 4265) zwischen der ersten Ausgleichskurve (4250) und zweiten Ausgleichskurve (4260) bestimmt werden,

wobei der Phasenabschnitt (3571, 3572), insbesondere dessen Zeitraum, insbesondere der jeweilige Beginn und das jeweilige Ende des Zeitraums, durch den Zeitpunkt zweier im zeitlichen Verlauf aufeinanderfolgender lokaler minimaler oder maximaler Abstände (4253; 4263; 4254, 4264; 4255; 4265; 4256, 4266) bestimmt wird.

7. Verfahren nach einem der voranstehenden Ansprüche **dadurch gekennzeichnet, dass** eine erste Vielzahl atmungskorrigierter erster Blutdruckwerte (5493, 5494, 5495, 5496) der Person als kontinuierliche Blutdruckwerte, und insbesondere eine dritte Vielzahl von Phasenabschnitten, ermittelt werden, wobei vorzugsweise jeder Blutdruckwert der ersten Vielzahl je einem Herzzyklus zugeordnet ist, und insbesondere jeder Phasenabschnitt der dritten Vielzahl von Phasenabschnitten mindestens zwei der mindestens zwei atmungskorrigierten ersten Blutdruckwert der ersten Vielzahl aufweist,

wobei von der variierenden Druckbeaufschlagung unabhängige Änderungen (5200, 5300), insbesondere Steigungen, der Werte der Einzelwertmessungen, insbesondere ermittelt mittels einer mathematischen Ableitung nach der Zeit, aus den, insbesondere die variierende Druckbeaufschlagung aufweisenden, Werten (5000) der Einzelwertmessungen ermittelt werden und hieraus eine Intensitätskurve (5200, 5300), aufweisend mehrere lokale Extrema (5220, 5320, 5230, 5330), ermittelt wird,

wobei gegebenenfalls basierend auf der Intensitätskurve (5200) eine vorrichtungsbreitenkorrigierte, insbesondere manschettenbreitekorrigierte, Intensitätskurve (5300), aufweisend mehrere lokale Extrema (5320, 5330), nämlich mehrere lokale Maxima (5320) und mehrere lokale Minima (5330), ermittelt wird,

wobei zu den in dem Messzeitraum, insbesondere der dritten und/oder der zweiten Zeitspanne oder ersten Zeitspanne, vorliegenden lokalen Maxima (5320) der, insbesondere vorrichtungsbreitenkorrigierten, Intensitätskurve mindestens eine, insbesondere mindestens zwei, obere Näherungsgerade (5351, 5352) gebildet wird und zu den in dem Messzeitraum, insbesondere der sechsten und/oder der fünften Zeitspanne oder der vierten Zeitspanne, vorliegenden lokalen Minima (5330) der, insbesondere vorrichtungsbreitenkorrigierten, Intensitätskurve mindestens eine, insbesondere mindestens zwei, untere Näherungsgerade (5361, 5362) gebildet wird,

wobei zur Ermittlung der ersten Vielzahl atmungskorrigierter erster Blutdruckwerte die Einzelwerte der, insbesondere vorrichtungsbreitenkorrigierten, Intensitätskurve (5300) auf Basis der zu den jeweiligen Zeitpunkten der zweiten Vielzahl vorliegenden Differenz zwischen der oberen Näherungsgerade (5351, 5352) und unteren Näherungsgerade (5361, 5362) normiert und um eine Skalierungskonstante skaliert werden, wobei die Skalierungskonstante auf dem Unterschied zwischen dem zum ersten und zweiten Zeitpunkt des ersten Phasenabschnitts (5087, 5088) vorliegenden normierten Wert (5477, 5478) der, insbesondere vorrichtungsbreitenkorrigierten, Intensitätskurve sowie dem ersten und zweiten der mindestens zwei atmungskorrigierten ersten Blutdruckwerte (5077, 5078) basiert, wobei aus den normierten und skalierten Einzelwerten eine Blutdruckverlaufskurve (5500) bestimmt wird, die lokale Maxima (5495, 5496) und lokale Minima (5493, 5494) aufweist,

wobei aus den den lokalen Maxima (5595, 5596) oder Minima (5593, 5594) der Blutdruckverlaufskurve (5400) zugeordneten Blutdruckwerten die erste Vielzahl der atmungskorrigierten, kontinuierlichen ersten Blutdruckwerte bestimmt werden.

8. Verfahren nach einem der voranstehenden Ansprüche **dadurch gekennzeichnet, dass** mindestens zwei atmungskorrigierte zweite Blutdruckwerte der Person dadurch bestimmt werden, dass

aus den Einzelwertmessungen ein nicht-atmungskorrigierter zweiter Blutdruckwert (3092, 3093, 5092) der Person bestimmt wird, wobei als ein zweiter Referenzzeitpunkt der Zeitpunkt des nicht-atmungskorrigierten zweiten Blutdruckwerts innerhalb des Messzeitraums bestimmt wird,

ein zweiter Phasenabschnitt der Atmung aufweisend einen Minimalwert (3520), insbesondere eine maximale Einatmung, in der Atmungskurve (3500) zu einem ersten Zeitpunkt des zweiten Phasenabschnitts und einen Maximalwert (3530), insbesondere eine maximale Ausatmung, in der Atmungskurve (3500) zu einem zweiten Zeitpunkt des zweiten Phasenabschnitts innerhalb des Messzeitraums zeitaufgelöst erfasst wird, wobei der zweite Phasenabschnitt so gewählt wird, dass der zweite Referenzzeitpunkt innerhalb des den zweiten Phasenabschnitt umfassenden Zeitraums fällt, und

dass der in den Einzelwertmessungen dem ersten Zeitpunkt des zweiten Phasenabschnitts zugeordnete Blutdruckwert (3096) oder einem zum ersten Zeitpunkt des zweiten Phasenabschnitts ermittelten Blutdruckwert (5078, 5074) als der erste der mindestens zwei atmungskorrigiertem zweiten Blutdruckwerte, insbesondere der maximalen Einatmung, und der in den Einzelwertmessungen dem zweiten Zeitpunkt des zweiten Phasenabschnitts zugeordnete Blutdruckwert (3095) oder einem zum zweiten Zeitpunkt des zweiten Phasenabschnitts ermittelten Blutdruckwert (5078, 5074) als der zweite der mindestens zwei atmungskorrigierten zweiten Blutdruckwert, insbesondere der maximalen Ausatmung, bestimmt werden,

wobei der atmungskorrigierte erste Blutdruckwert und der nicht-atmungskorrigierte erste Blutdruckwert ein systolischer Blutdruckwert und der atmungskorrigierte zweite Blutdruckwert und der nicht-atmungskorrigierte zweite Blutdruckwert ein diastolischer Blutdruckwert sind.

9. Verfahren nach den beiden voranstehenden Ansprüchen **dadurch gekennzeichnet, dass** eine zweite Vielzahl der mindestens zwei atmungskorrigierten zweiter diastolischer Blutdruckwerte als kontinuierliche diastolische Blutdruckwerte ermittelt werden, wobei aus den den lokalen Maxima (5595, 5596) der Blutdruckverlaufskurve (5400) zugeordneten Blutdruckwerten atmungskorrigierte, kontinuierliche, erste systolische Blutdruckwerte der ersten Vielzahl der mindestens zwei atmungskorrigierten ersten systolischen Blutdruckwerte und aus den den lokalen Minima (5593, 5594) der Blutdruckverlaufskurve (5400) zugeordneten Blutdruckwerten atmungskorrigierte, kontinuierliche, zweite diastolischen Blutdruckwerte der zweiten Vielzahl der mindestens zwei atmungskorrigierten zweiten diastolischen Blutdruckwerte bestimmt werden.

10. Verfahren nach einem der voranstehenden Ansprüche, insbesondere der Ansprüche 7 bis 9, **dadurch gekennzeichnet, dass** basierend auf den Werten der Einzelwertmessungen, insbesondere basierend auf der Intensitätskurve (5200), eine vorrichtungsbreitenkorrigierte, insbesondere manschettenbreitekorrigierte, Intensitätskurve (5300) bestimmt wird, aufweisend mehrere lokale Extrema (5320, 5330), nämlich mehrere lokale Maxima (5320) und mehrere lokale Minima (5330), wobei zur Ermittlung der vorrichtungsbreitenkorrigierten, insbesondere manschettenbreitekorrigierten, Intensitätskurve (5300) eine erste, eine zweite und eine dritte untere Annäherungsgerade (5261, 5262, 5263) und eine erste, eine zweite und eine dritte obere Annäherungsgerade (5251 5252, 5253) gebildet werden,

wobei zur Bildung der vorrichtungsbreitenkorrigierten Intensitätskurve (5300) Einzelwerte der vorrichtungsbreitenkorrigierten Intensitätskurve (5300) durch Multiplikation der Werte der Einzelwertmessungen, insbesondere der Einzelwerte der Intensitätskurve (5200), mit einem zeitabhängigen Breitenkorrekturfaktor gebildet werden, wobei der Breitenkorrekturfaktor auf einer normierten Differenz basiert, wobei die Differenz eine Differenz zwischen den zu den jeweiligen Zeitpunkten vorhandenen Werten, insbesondere Einzelwerten, der ersten, der zweiten und der dritten unteren Annäherungsgerade (5261, 5262, 5263) und den zu den jeweiligen Zeitpunkten vorliegenden Werten, insbesondere Einzelwerten, der ersten, der zweiten und der dritten oberen Annäherungsgerade (5251, 5252, 5253) ist und um einen Normierungsparameter normiert wird.

11. Verfahren nach dem vorstehenden Anspruch,

wobei die erste obere Annäherungsgerade (5251) zu den in einer ersten Zeitspanne vorliegenden lokalen Maxima (5220), die zweite obere Annäherungsgerade (5252) zu den in einer zweiten Zeitspanne vorliegenden lokalen Maxima (5220), die dritte obere Annäherungsgerade (5253) zu den in einer dritten Zeitspanne vorliegenden lokalen Maxima (5220), die erste untere Annäherungsgerade (5261) zu den in einer vierten Zeitspanne vorliegenden lokalen Minima (5230), die zweite untere Annäherungsgerade (5262) zu den in einer fünften Zeitspanne vorliegenden lokalen Minima (5230) und die dritte untere Annäherungsgerade (5263) zu den in einer sechsten Zeitspanne vorliegenden lokalen Minima (5230) gebildet werden,

wobei der Beginn und das Ende der ersten, zweiten, dritten, vierten, fünften und sechsten Zeitspanne innerhalb des Messzeitraums liegt, und

wobei der Beginn (5280) der ersten Zeitspanne vor dem Beginn (5281, 5282) und Ende (5282, 5283) der zweiten und der dritten Zeitspanne und der Beginn (5285) der vierten Zeitspanne vor dem Beginn (5286, 5287) und Ende (5287, 5288) der fünften und sechsten Zeitspanne liegt und

wobei das Ende (5283) der dritten Zeitspanne nach dem Beginn (5280, 5281) und Ende (5281, 5282) der ersten und zweiten Zeitspanne und das Ende (5288) der sechsten Zeitspanne nach dem Beginn (5285, 5286) und Ende (5286, 5287) der vierten und fünften Zeitspanne liegt,

wobei der Beginn (5280) der ersten Zeitspanne insbesondere mit dem Beginn (5285) der vierten Zeitspanne und das Ende (5283) der dritten Zeitspanne insbesondere mit dem Ende (5288) der sechsten Zeitspanne übereinstimmt,

und wobei der Beginn (5281) der zweiten Zeitspanne dem Ende (5281) der ersten Zeitspanne, das Ende (5282) der zweiten Zeitspanne dem Beginn (5282) der dritten Zeitspanne, der Beginn (5286) der fünften Zeitspanne dem Ende (5286) der vierten Zeitspanne und das Ende (5287) der fünften Zeitspanne dem Beginn (5287) der sechsten Zeitspanne entspricht,

wobei das Ende (5281) der ersten Zeitspanne und der Beginn (5282) der dritten Zeitspanne so gewählt wird, dass die zweite obere Annäherungsgerade (5252) die minimalste Abweichung von den innerhalb der zweiten Zeitspanne vorliegenden lokalen Maxima (5220) aufweist, und

wobei das Ende der vierten Zeitspanne (5286) und der Beginn der sechsten Zeitspanne (5287) so gewählt wird, dass die zweite untere Annäherungsgerade (5262) die minimalste Abweichung von den innerhalb der fünften Zeitspanne vorliegenden lokalen Minima (5230) aufweist.

12. Verfahren nach den beiden voranstehenden Ansprüchen, wobei die Normierungsparameter auf der Differenz zwischen den unteren und oberen Annäherungsgeraden (5251, 5252, 5253, 5261, 5262, 5263) zum Beginn (5281) der zweiten Zeitspanne und der Differenz zwischen den unteren und oberen Annäherungsgeraden (5251, 5252, 5253, 5261, 5262, 5263) zum Beginn (5286) der fünften Zeitspanne basiert.

13. Manschette, insbesondere Luftdruckmanschette mit einem Armband, einem aufblasbaren Volumen und einer Steuereinheit, wobei die Steuereinheit eingerichtet und konfiguriert ist, ein Verfahren nach einem der vorangehenden Ansprüche, insbesondere soweit abhängig nach Anspruch 3, auszuführen.

14. Manschette nach dem voranstehenden Anspruch **dadurch gekennzeichnet, dass** das Armband eine Längserstreckung aufweist, wobei das Armband eingerichtet ist, entlang deren Längserstreckung einen Teil des Körpers zu umschließen, wobei das Armband eine Breitenerstreckung mit einer Breite von weniger als 5 cm, insbesondere 4 cm oder weniger, aufweist, wobei die Manschette insbesondere ein Teil einer Armbanduhr, insbesondere einer Smart-Watch, ist.

Figur 1

Figur 2

Figur 3

Figur 4

Figur 5

(a)

3181

3500

3583 3584

3571

(b)

3181

3589

3500

3583 3584

3571

(c)

3181

3589

3584 3500

3583

3571

Figur 6

Figur 7

Europäisches Patentamt
European Patent Office
Office européen des brevets

**EUROPÄISCHER RECHERCHENBERICHT**

Nummer der Anmeldung

**EP 23 19 6265**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2022/031176 A1 (REDTEL HOLGER [DE]) 3. Februar 2022 (2022-02-03) | 1-9, 12-14 | INV. A61B5/022 |
| Y | * Absätze [0028], [0048] – [0053], [0148], [0184], [0257], [0349], [0350], [0477] * <br> * Abbildungen 4, 5, 9, 14 * <br> ----- | 10,11 | A61B5/08 A61B5/113 A61B5/00 A61B5/33 |
| Y | WO 2016/148956 A1 (LIFECUFF TECH INC [US]) 22. September 2016 (2016-09-22) * Absatz [0074] * <br> ----- | 10,11 | |
| A | EP 3 456 254 A1 (KONINKLIJKE PHILIPS NV [NL]) 20. März 2019 (2019-03-20) * Absatz [0027] * <br> ----- | 1-14 | |

**RECHERCHIERTE SACHGEBIETE (IPC)**

**A61B**

Der vorliegende Recherchenbericht wurde für alle Patentansprüche erstellt

| Recherchenort | Abschlußdatum der Recherche | Prüfer |
|---|---|---|
| **Den Haag** | **14. Februar 2024** | **Worms, Georg** |

EPO FORM 1503 03.82 (P04C03)

**EP 4 520 260 A1**

## ANHANG ZUM EUROPÄISCHEN RECHERCHENBERICHT
## ÜBER DIE EUROPÄISCHE PATENTANMELDUNG NR.

EP 23 19 6265

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-02-2024

| Im Recherchenbericht angeführtes Patentdokument | Datum der Veröffentlichung | Mitglied(er) der Patentfamilie | Datum der Veröffentlichung |
|---|---|---|---|
| US 2022031176 A1 | 03-02-2022 | CN 113507884 A | 15-10-2021 |
| | | EP 3890600 A2 | 13-10-2021 |
| | | IL 283689 A | 29-07-2021 |
| | | US 2022031176 A1 | 03-02-2022 |
| | | WO 2020115216 A2 | 11-06-2020 |
| WO 2016148956 A1 | 22-09-2016 | CN 107530223 A | 02-01-2018 |
| | | DK 3270863 T3 | 25-05-2021 |
| | | EP 3270863 A1 | 24-01-2018 |
| | | JP 6777647 B2 | 28-10-2020 |
| | | JP 2018508314 A | 29-03-2018 |
| | | KR 20170129767 A | 27-11-2017 |
| | | WO 2016148956 A1 | 22-09-2016 |
| EP 3456254 A1 | 20-03-2019 | KEINE | |

EPO FORM P0461

Für nähere Einzelheiten zu diesem Anhang : siehe Amtsblatt des Europäischen Patentamts, Nr.12/82

49

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **H. HINGHOFER-SZALKAY**. Praktische Physiologie.
  Blackwell, 1994